# EUROPÄISCHE PATENTSCHRIFT

(11) **EP 1 945 767 B1**
(45) Veröffentlichungstag und Bekanntmachung des Hinweises auf die Patenterteilung: **07.04.2010**
(21) Anmeldenummer: 05811334.1
(22) Anmeldetag: 09.11.2005
(51) Int. Cl.: C07K 16/18, C07K 16/30, A61K 39/395, A61P 35/00, G01N 33/574, G01N 33/563, C07K 14/78

(54) **IDENTIFIZIERUNG UND CHARAKTERISIERUNG VON FUNKTIONSBLOCKIERENDEN ANTI-ED-B-FIBRONEKTIN ANTIKÖRPERN**
IDENTIFICATION AND CHARACTERIZATION OF FUNCTION-BLOCKING ANTI-ED-B-FIBRONECTIN ANTIBODIES
IDENTIFICATION ET CARACTÉRISATION D'ANTICORPS BLOQUANTS ANTI-ED-B DE FIBRONECTINE

(43) Veröffentlichungstag der Anmeldung: 23.07.2008
(73) Patentinhaber: MorphoSys AG, 82152 Martinsried/München (DE)
(72) Erfinder: MENRAD, Andreas, Ely Cambs CB6 2 HP (GB); PRASSLER, Josef, 82110 Germering (DE); WEIDMANN, Armin, 86911 Diesser am Ammersee (DE)
(74) Vertreter: Hutter, Bernd
(86) Internationale Anmeldenummer: PCT/EP2005/012185
(87) Internationale Veröffentlichungsnummer: WO 2007/054120

(56) Entgegenhaltungen:
- WO-A-02/20563
- WO-A-99/58570
- US-A1- 2002 049 247
- MENRAD A ET AL: "Molecular and functional characterization of ED-B fibronectin selective function blocking antibodies and the evaluation of phage angiomics for target identification and validation" HUMAN ANTIBODIES, Bd. 13, Nr. 1-2, 2004, Seite 33, XP008063389 & 11TH INTERNATIONAL CONFERENCE ON HUMAN ANTIBODIES AND HYBRIDOMAS; DUBLIN, IRELAND; OCTOBER 06-08, 2004 ISSN: 1093-2607
- EBBINGHAUS C ET AL: "Diagnostic and therapeutic applications of recombinant antibodies: Targeting the extra-domain B of fibronectin, a marker of tumor angiogenesis" CURRENT PHARMACEUTICAL DESIGN 2004 NETHERLANDS, Bd. 10, Nr. 13, 2004, Seiten 1537-1549, XP008063359 ISSN: 1381-6128
- MENRAD A ET AL: "ED-B FIBRONECTIN AS A TARGET FOR ANTIBODY-BASED CANCER TREATMENTS" EXPERT OPINION ON THERAPEUTIC TARGETS, ASHLEY PUBLICATIONS, LONDON, GB, Bd. 9, Nr. 3, Juni 2005 (2005-06), Seiten 491-500, XP009052148 ISSN: 1472-8222
- FATTORUSSO ROBERTO ET AL: "NMR structure of the human oncofoetal fibronectin ED-B domain, a specific marker for angiogenesis" STRUCTURE (LONDON), Bd. 7, Nr. 4, 15. April 1999 (1999-04-15), Seiten 381-390, XP002378221 ISSN: 0969-2126

## Beschreibung

Die Erfindung betrifft rekombinante Antikörper oder Antikörperfragmente, die an die ED-B-Isoform von Fibronektin binden und deren Funktion blockieren können. Weiterhin wird die diagnostische und pharmazeutische Anwendung der erfindungsgemäßen Polypeptide offenbart.

Während der Tumorentstehung und Progression wird die extrazelluläre Matrix (ECM), in welcher der Tumor wächst, durch proteolytischen Abbau von bereits existierender ECM umgebaut. Dieser Prozess generiert eine vom Tumor induzierte Matrix, die sich von der im normalen Gewebe vorkommenden ECM unterscheidet. Die tumorinduzierte ECM scheint die optimale Umgebung für das Tumorwachstum- und die Tumorangiogenese zu sein (1-4).

Bei der Tumorangiogenese bilden sich neue Blutgefäße aus bereits existierenden Gefäßen. Dieser Prozess setzt die Proteolyse von ECM, das zielgerichtete Wachstum und Differenzierung von Endothelzellen in neue Gefäßstrukturen voraus, die essentiell für das weitere Tumorwachstum sind (5).

Fibronektine sind eine wichtige Klasse von Matrix-Glykoproteinen. Ihre Hauptrolle besteht darin, das Anhaften von Zellen an eine Vielzahl verschiedener extrazellulärer Matrizes zu ermöglichen. Das Vorhandensein von Fibronektinen auf der Oberfläche von nicht-transformierten Zellen in Kultur sowie deren Abwesenheit bei transformierten Zellen führte zur Identifizierung von Fibronektinen als wichtigen Adhäsionsproteinen. Sie wechselwirken mit zahlreichen verschiedenen anderen Molekülen, z.B. Collagen, Heparansulfat-Proteoglykanen und Fibrin, und regulieren damit die Zellform und den Aufbau des Zytoskeletts. Weiterhin sind sie verantwortlich für Zellmigration und Zelldifferenzierung während der Embryogenese. Sie sind außerdem wichtig für die Wundheilung, bei der sie die Wanderung von Makrophagen und anderen Immunzellen in das betroffene Gebiet ermöglichen, und bei der Bildung von Blutgerinnseln, indem sie das Anheften von Blutplättchen an beschädigte Regionen der Blutgefäße ermöglichen.

Fibronektine sind Dimere zweier ähnlicher Peptide, wobei jede Kette ungefähr 60-70 nm lang ist. Wenigstens 20 verschiedene Fibronektin-Ketten sind identifiziert worden, von denen alle durch alternatives Spleißen des RNA-Transkripts eines einzigen Fibronektin-Gens erzeugt werden.

Fibronektine sind hochmolekulare, adhäsionsvermittelnde Glykoproteine, die eine wichtige Rolle bei der Entwicklung des Blutgefäßsystems spielen. Weiterhin wirken Fibronektine chemotaktisch auf Endothelzellen, modulieren die Wirkung von Wachstumsfaktoren und unterstützen das Längenwachstum von Endothelzellen während der Angiogenese (6-9). Eine Analyse vpm proteolytischen Fibronektin-Spaltprodukten zeigt, dass die Polypeptide aus sechs stark gefalteten Domänen bestehen, von denen jede Domäne wiederung so genannte Wiederholungssequenzen ("repeats") enthält, deren Ähnlichkeiten hinsichtlich ihrer Aminosäuresequenz eine Klassifikation in drei Typen erlauben (Typ I, II, III). Die zentrale Region beider Ketten aus dem Dimer besteht aus einem Abschnitt von so genannten Typ-III-Repeats, die durchschnittlich 90 Aminosäuren lang sind (10). Strukturelle Studien haben ergeben, dass jeder Typ-III-Repeat aus sieben beta-Strängen besteht, die in zwei antiparallele Faltblätter gefaltet sind, wobei kurze Loop-Regionen als potentielle Protein-Protein-Wechselwirkungsstellen exponiert sind (11).

Die Typ-III-Repeats ermöglichen es, dass Fibronektine als Adhäsionsmoleküle fungieren, die mit Zelloberflächenmolekülen, den so genannten "Integrinen" interagieren. Der Begriff des Integrins wurde 1987 erstmals in (12) verwendet, um eine verwandte Gruppe von heterodimeren Zelloberflächenmolekülen zu beschreiben, die als Vermittler zwischen der extrazellulären Matrix und dem intrazellulären Zytoskelett fungieren und so die Zelladhäsion und Migration induzieren. Diese heterodimeren Rezeptoren "integrieren" oder vermitteln also Signale vom extrazellulären Milieu mit spezifischen zellulären Funktionen. Bis heute sind 17 beta-Untereinheiten bekannt, die mit mehr als 20 alpha-Untereinheiten spezifisch und nicht kovalent interagieren können, um so mehr als 20 verschiedene Familien zu bilden (13). Insbesondere vermittelt die Sequenz RGDS, die sich im zehnten Repeat vom Typ III des Fibronektins befindet (III-10), die Wechselwirkung von Fibronektin mit wenigstens 8 verschiedenen Integrinen. Darüber hinaus wurde gezeigt, dass mindestens vier Integrine in einer RGDS-unabhängigen Weise spezifisch mit Fibronektin interagieren können (13). Die Gruppe der Wiederholungssequenzen vom Typ III umfasst neben den III7-, III8-, III9- und III10-Sequenzen auch die Repeats EIIIB und EIIIA (ED-B und ED-A).

Das ED-B-Fibronektin ist in normalem Gewebe von Erwachsenen (einzige Ausnahme: proliferierendes Endometrium) nicht nachweisbar, während es im fötalen Gewebe und beim Tumorwachstum stark exprimiert wird, neben einer stromalen lokalen Expression von ED-B. Darüber hinaus lokalisiert ED-B-Fibronektin perivaskulär um Blutgefäße, die sich während der Angiogenese neu gebildet haben. Aus diesem Grund ist ED-B-Fibronektin ein spezifisches Markerprotein für den Prozess der (Tumor-)Angiogenese (14).

Die ED-B-Domäne ist ein hochkonservierter, kompletter Typ-III-Homologiebaustein, bestehend aus 91 Aminosäuren. Der Homologiegrad zwischen Mensch und Ratte beträgt 100 %, zwischen Mensch und Huhn 96 %. Über die Funktion der ED-B-Domäne ist in der Literatur sehr wenig bekannt. Einige wenige Publikationen (15-17) spekulieren über eine generelle adhäsionsvermittelnde Wirkung für verschiedene Zellen. Eine spezifische Wirkung auf Endothelzellen wurde bisher noch nicht gezeigt.

In WO 02/20563 wurde gezeigt, dass rekombinantes ED-B eine spezifische pro-angiogene Wirkung *in vitro* zeigt: (i) die Proliferation von bFGFstimulierten humanen dermalen microvaskulären Endothelzellen (HDMVEc) wird von rekombinantem ED-B verstärkt, (ii) das Protein vermittelt die Adhäsion von HDMVEC und (iii) rekombinantes ED-B stimuliert die Invasion und Differenzierung (tube formation) von HDMVEc in Kollagengelen.

Weiterhin konnten diese ED-B-vermittelten Effekte durch synthetische Peptide, die aus der ED-B-Domäne abgeleitet wurden, spezifisch blockiert werden. Die Peptidsequenzen stellen somit die Bindungsregion für einen ED-B-spezifischen Rezeptor auf der Endothelzelloberfläche dar, der mittels Affinitätschromatographie als das α₂β₁-Integrin identifiziert wurde. Die spezifische Interaktion zwischen dem α₂β₁-Integrin und der ED-B-Domäne war bisher in der Literatur nicht beschrieben.

Ferner werden in WO 02/20563 Proteine offenbart, die spezifisch durch die ED-B-Fibronektindomäne reguliert werden und welche das α₂β₁-Integrin, die fokale Adhäsionskinase, den CD6-Ligand (ALCAM), die alpha-Kette des Vitronektinrezeptors, die integrierte alpha 8-Untereinheit oder den Precursor des Follistatin-verwandten Proteins umfasst.

Eine Vielzahl von Integrinrezeptoren mit teilweise überlappenden Eigenschaften werden von Endothelzellen exprimiert (Lit.: DG Stupack und DA Cheresh, SciSTKE, 2002 Feb 12; 2002). Diese Expressionsmuster zeigen, dass verschiedene Integrine (z.B. alphavbeta3 und alpha5beta1) ähnliche biologische Phänomene (Adhäsion, Migration und Überleben) vermitteln und daher redundante Systeme für die Endothelzelle darstellen, die ihr Verhalten und Überleben absichern. Bisher war bekannt, dass alpha2beta1 mit seinen natürlichen Liganden, den Kollagenen, interagiert. Die Blockade dieser Interaktion kann zu einer anti-angiogenen Wirkung führen (Y Funahashi et al. Cancer Res. 62:6116-6123, 2002).

Menrad et al. (Molecular and functional characterization of ED-B fibronectin selective function blocking antibodies and the evaluation of phage angiomics for target identification and validation" HUMAN ANTIBODIES, Bd. 13, Nr. 1-2, 2004, Seite 33, 11TH INTERNATIONAL CONFERENCE ON HUMAN ANTIBODIES AND HYBRIDOMAS; DUBLIN; IRELAND; OCTOBER 06-08, 2004 ISSN: 1093-2607) beschreibt sehr allgemein, dass hochaffine ED-B Fibronektin funktionsblockierende Antikörperfragmente erfolgreich identifiziert wurden. Es wird jedoch nicht beschrieben, wie derartige Antikörperfragmente erhalten und selektiert werden können. Auch werden keine Antigenbindungsstellen angegeben. Menrad et al. offenbart auch nicht, ob die Antikörper an die ED-B Domände von ED-B Fibronketin binden. Ebenfalls sind keine Antikörperfragment-Konzentrationen angegeben. Auch wurden hier nur erste Wirkanzeichen festgestellt. Eine Antikörperherstellung kann aus diesem Artikel nicht hergeleitet werden.

Eine Aufgabe der vorliegenden Erfindung war daher die Bereitstellung von funktionsblockierenden Bindemolekülen, wie z.B. Antikörpern, die Rezeptorbindungsstellen der ED-B-Domäne spezifisch blockieren. Diese Bindemoleküle besitzen eine anti-angiogene Wirkung auf (Tumor-) Endothelzellen. Im Gegensatz zum relativ breit exprimierten α₂β₁-Integrin stellt die ED-B-Domäne ein ideales und spezifisches Zielmolekül für solche Bindemoleküle dar.

Die Struktur von ED-B macht die Entwicklung von monoklonalen und polyklonalen Antikörpern schwierig, da damit zu rechnen ist, dass ED-B eine geringe Immunogenität *in vivo* hat. So reagiert der Antikörper BC-1 (J. Cell Biol. 108 (1989), 1139-1148) mit einem kryptischen Epitop von Fibronektin, das nur in Anwesenheit von ED-B vorliegt und daher nicht direkt mit ED-B. Der Antikörper L19 (Tarli et al. Blood 94 (1999), 192-198 und WO 01/62800), der durch Verwendung von rekombinantem ED-B als Immunogen erzeugt wurde, ist wiederum biologisch inaktiv, d.h. er kann die Zelladhäsion an ED-B nicht signifikant kennen.

Überraschenderweise wurde nun gefunden, dass funktionsblockierende ED-B-Bindemoleküle hergestellt werden können, wie etwa der ED-B-funktionsblockierende Antikörper MOR03255, die die Adhäsion von Zellen an ED-B stark hemmen und *in vivo* eine signifikante Verringerung des Tumorwachstums herbeiführen. Die durch die erfindungsgemäßen Bindemoleküle bewirkte Blockade von ED-B kann offensichtlich nicht durch kompensatorische Mechanismen der Kollagen-Integrin Interaktion ausgeglichen werden.

Mittels der HuCAL^{®}-GOLD-Antikörperbibliothek, einer Bibliothek mit etwa 1,6 x 10E10 unterschiedlichen Antikörpern im Fab-Fragmentformat, die ausgehend von den HuCAL-Konsensussequenzen (WO 97/08320; Knappik A, Ge L, Honegger A, Pack P, Fischer M, Wellnhofer G, Hoess A, Wolle J, Plunckthun A, and Vimekas B (2000) J. Mol. Biol. 296:57-86) durch Diversifikation entsprechend der Diversitat humaner Antikörper in allen sechs CDR-Bereichen generiert wurde und mittels CysDisplay (WO 01/05950), einer Variante des Phagendisplay-Verfahrens, durchmustert wird, wurden in den zur vorliegenden Erfindung führenden Versuchen funktionsblockierende und selektiv an die ED-B-Domäne bindende Fab-Antikörperfragmente identifiziert. Die Wirksamkeit dieser Antikörperfragmente konnte in einem *in* vitro Adhäsionstest gezeigt werden, der die spezifische Interaktion zwischen rekombinantem ED-B und isolierten HDMVEc widerspiegelt. Durch gezielte Veränderung der Bindemoleküle konnte die Bindeaffinität an die ED-B-Domäne erheblich verbessert werden. Ferner konnte *in vivo* gezeigt werden, dass die Bindemoleküle in einem Tiermodell das Wachstum von Tumoren hemmen.

Ein erster Aspekt der Erfindung ist somit ein Antikörper oder Antikörperfragment, das
(i)spezifisch an die ED-B-Domäne von Fibronektin bindet und
(ii)die Interaktion zwischen der ED-B-Domäne und ihrem Rezeptor inhibiert.

Der Begriff "Antikörper" im Sinne der vorliegenden Erfindung umfasst polyklonale, monoklonale, chimäre, humanisierte oder humane Antikörper sowie rekombinante Antikörper, z.B. einzelkettige Antikörper, oder Antigenbindende Antikörperfragmente, z.B monovalente Antikörperfragmente wie etwa Fab-Fragmente oder scFv-Fragmente, oder divalente Antikörperfragmente wie etwa F(ab')₂-Fragmente.

Erfindungswesentlich ist in diesem Zusammenhang, dass die Antikörper eine oder mehrere Antigenbindungsstellen enthalten, welche die o.g. Voraussetzungen, d.h. spezifische Bindung an die ED-B-Domäne und Inhibition der Interaktion zwischen der ED-B-Domäne und ihrem Rezeptor, insbesondere ihrem Rezeptor auf Endothelzellen, erfüllen. Diese Antigenbindungseigenschaften werden vorzugsweise durch Kombination einer VH- und VL-Region erreicht, wobei diese Regionen aus so genannten Gerüstregionen (FR1, FR2, FR3 und FR4) sowie den die Antigenbindung vermittelnden CDR-Regionen (H-CDR1, H-CDR2, H-CDR3 für die VH-Region und L-CDR1, L-CDR2, L-CDR3 für die VL-Region) aufgebaut sind.

Die erfindungsgemäßen Antikörper oder Antikörperfragmente besitzen vorzugsweise eine Affinität für die ED-B-Domäne entsprechend einem Ko-Wert ≤ 1 µM, vorzugsweise ≤ 100 nM, besonders bevorzugt ≤ 10 nM, noch mehr bevorzugt ≤ 1 nM und am meisten bevorzugt ≤ 0,1 nM, wobei die Affinität wie in den Beispielen angegeben durch einen Test am BIAcore^{®} System ermittelt werden kann.

Das erfindungsgemäßen Antikörper oder Antikörperfragmente zeichnen sich aus, dass sie spezifisch an die ED-B-Domäne von Fibronektin bindet, d.h. es bindet mit einer signifikant geringeren Affinität an andere Fibronektindomänen, insbesondere an die Fibronektindomäne 6 (FN6) oder/und die Fibronektindomänen 7-8-9 (7-8-9). In einer bevorzugten Ausführungsform binden die erfindungsgemäßen Antikörper oder Antikörperfragmente und die Fibronektin ED-B-Domäne mit um mindestens den Faktor 2, insbesondere um mindestens den Faktor 5 und besonders bevorzugt um mindestens den Faktor 10 höheren Affinität als an FN6 oder/und 7-8-9, wie etwa durch den in den Beispielen angegebenen Bindetest ermittelt werden kann.

In einer weiteren bevorzugten Ausführungsform zeigen die erfindungsgemäßen Antikörper oder Antikörperfragmente *in vitro* eine Hemmung der Adhäsion von rekombinantem ED-B an HDMVEc-Zellen zeigt, vorzugsweise eine Hemmung von mindestens 50 % und besonders bevorzugt von mindestens 75 % in einer Konzentration von jeweils 10 µg/ml.

Darüber hinaus ist es bevorzugt, dass die erfindungsgemäße Antikörper oder Antikörperfragmente *in vivo* eine Hemmung des Wachstums eines durch Implantation von F9-Teratokarzinomzellen (ATCC CRL-1720) in Versuchstieren, beispielsweise Nacktmäusen, erzeugten Tumors zeigt.

Bevorzugt sind in der VL-Region Veränderungen der L-CDR3-Region oder/und in der VH-Region Veränderungen der H-CDR2-Region.

Die erfindungsgemäßen Antikörper oder Antikörperfragmente, die spezifisch an die ED-B Domäne von Fibronektin binden und ihre Interaktion mit ihrem Rezeptor inhibieren, sind dadurch gekennzeichnet, dass sie die VH-Region kodiert von SEQ ID NO. 75 (MOR 03251) und die VL-Region kodiert von SEQ ID NO. 77 (MOR 03251) oder die H-CDR1-, H-CDR2- und H-CDR3- Region aus dieser VH Region und die L-CDR1-, L-CDR2- und L-CDR3-Region aus dieser VL Region aufweisen, wobei der Antikörper oder das Antikörperfragment die ED-B-Domäne mit einem KD-Wert von 0,1 nM oder niedriger binder und der KD-Wert durch Oberflächenplasmonenresonanz ermittelt wird,
oder dadurch gekennzeichnet sind, dass sie
die VH-Region kodiert von SEQ ID NO. 79 (MOR 03252) und die VL-Region kodiert von SEQ ID NO. 77 (MOR 03252) oder die H-CDR1-, H-CDR2- und H-CDR3- Region aus dieser VH Region und die L-CDR1-, L-CDR2- und L-CDR3- Region aus dieser VL Region aufweisen, wobei der Antikörper oder das Antikörperfragment die ED-B-Domäne mit einem KD-Wert von 0,1 nM oder niedriger bindet und der KD-Wert durch Oberflächenplasmonenresonanz ermittelt wird,
oder dadurch gekennzeichnet sind, dass sie die VH-Region kodiert von SEQ ID NO. 83 (MOR 03255) und die VL-Region kodiert von SEQ ID NO. 77 (MOR 03255) oder die H-CDR1-, H-CDR2- und H-CDR3- Region aus dieser VH Region und die L-CDR1-, L-CDR2- und L-CDR3- Region aus dieser VL Region aufweisen, wobei der Antikörper oder das Antikörperfragment die ED-B-Domäne mit einem KD-Wert von 0,1 nM oder niedriger bindet und der KD-Wert durch Oberflächenplasmonenresonanz ermittelt wird,
oder dadurch gekennzeichnet sind, dass sie die VH-Region kodiert von SEQ ID NO. 85 (MOR 03257) und die VL-Region kodiert von SEQ ID NO. 77 (MOR 03257) oder die H-CDR1-, H-CDR2- und H-CDR3- Region aus dieser VH Region und die L-CDR1-, L-CDR2- und L-CDR3- Region aus dieser VL Region aufweisen, wobei der Antikörper oder das Antiktirperfragment die ED-B-Domäne mit einem KD-Wert von 0,1 nM oder niedriger bindet und der KD-Wert durch Oberflächenplasmonenresonanz ermittelt wird,
oder dadurch gekennzeichnet sind, dass sie die VH-Region kodiert von SEQ ID NO. 87 (MOR 03258) und die VL- Region kodiert von SEQ ID NO. 59 (MOR 03258) oder die H-CDR1-, H-CDR2- und H-CDR3- Region aus dieser VH Region und die L-COR1-, L-CDR2 und L-CDR3- Region aus dieser VL Region aufweisen, wobei der Antikörper oder das Antikörperfragment die ED-B-Domäne mit einem KD-Wert von 0,1 nM oder niedriger bindet und der KD-Wert durch Oberflächenplasmonenresonanz ermittelt wird.

Der Aufbau der VH- und VL-Ketten der erfindungsgemäßen Polypeptide ist wie folgt:
VH-Kette:
   - Die Framework 1-Region reicht von nt 1-75 (also 25aa), das letzte Codon (aa) von FR1 ist immer: TCC (Ser).
   - Die "COR1-Region" kann zwei verschiedene Längen aufweisen, entweder 30 nt (10 aa) (möglich in Familien VH1A, VH1B, 3, 4 und 5); oder 36 nt (12 aa) (möglich in Familien VH2, VH4 und VH6).
   - Die Framework 2-Region weist immer 33 nt (also 11 aa) auf, das erste Codon (aa) von FR2 ist immer: TGG (Trp), die letzten beiden Codons sind immer CTC.GAG (LeuGlu).
   - Die "CDR2-Region" kann drei verschiedene Längen aufweisen, entweder 57 nt (19 aa) (möglich in Familien VH2 und VH4); 60 nt (20 aa) (möglich in Familien VH1A, VH1B, 3 und 5) oder 63 nt (21 aa) (möglich in Familie VH6).
   - Die Framework 3-Region weist immer 96 nt (also 32 aa) auf, das dritte Codon (aa) von FR3 ist immer: ACC (Thr), die letzten fünf Codons sind immer TAT.TAT.TGC.GCG.CGT (Tyr Tyr Cys Ala Arg).
   - Die "CDR3-Region" kann mehrere verschiedene Längen aufweisen, von 12-69 nt (4-23 aa) (in allen Familien VH2 und VH4); 60 nt (20 aa) (möglich in Familien VH1A, VH1B, 3 und 5) oder 63 nt (21 aa) (möglich in Familie VH6).
   - Die Framework 4-Region weist immer 33 nt (also 11 aa) und ist in allen 7 Familien identisch: TGG.GGC.CAA.GGC.ACC.CTG.GTG.ACG.GTT. AGC.TCA
VL kappa-Kette:
   - Die Framework 1-Region reicht von nt 1-69 (also 23 aa), das letzte Codon (aa) von FR1 ist immer: TGC (Cys).
   - Die "CDR1-Region" kann vier verschiedene Längen aufweisen, entweder 24 nt (8 aa) (möglich in Familien Vk1 und Vk3); 27 nt (9 aa) (möglich in Familie Vk3); 39 nt (13 aa) (möglich in Familie Vk2); oder 42 nt (14 aa) (möglich in Familie Vk4); erstes Codon (aa) immer AGA (Arg), vorletztes Codon (aa) von CDR1-Region immer: CTG (Leu).
   - Die Framework 2-Region weist immer 33 nt (also 11 aa) auf, die ersten beiden Codons (aa) von FR2 sind immer: TGG.TAC (Trp Tyr), das vorletzte Codon ist immer CCG (Pro).
   - Die "CDR2-Region" weist immer 33 nt (also 11 aa) auf, wobei die ersten drei Codons (aa) immer CTA.TTA.ATT (Leu Leu Ile) sind.
   - Die Framework 3-Region weist immer 96 nt (also 32 aa) auf, die ersten drei Codons (aa) von FR3 sind immer: GGG.GTC.CCG (Gly Val Pro), die letzten drei Codons sind immer TAT.TAT.TGC (Tyr Tyr Cys).
   - Die "CDR3-Region" weist immer 24 nt (also 8 aa) auf, wobei das zweite Codon (aa) immer CAG (Gln) ist.
   - Die Framework 4-Region weist immer 39 nt (also 13 aa) und ist in allen vier Familien identisch: ACC.TTT.GGC.CAG.GGT.ACG.AAA.GTT.GAA. ATT.AAA.CGT.ACG.
VL lambda-Kette:
   - Die Framework 1-Region reicht von nt 1-66 (also 22 aa), das letzte Codon (aa) von FR1 ist immer: TGT (Cys).
   - Die "CDR1-Region" kann drei verschiedene Längen aufweisen, entweder 33 nt (11 aa) (möglich in Familie VI3); 39 nt (13 aa) (möglich in Familie VI1); oder 42 nt (14 aa) (möglich in Familien VI1 und VI2).
   - Die Framework 2-Region weist immer 33 nt (also 11 aa) auf, die ersten beiden Codons (aa) von FR2 sind immer: TGG.TAC (Trp Tyr), das drittletzte Codon ist immer GCG (Ala).
   - Die "CDR2-Region" weist immer 33 nt (also 11 aa) auf, wobei das dritte Codon (aa) immer ATT (Ile) ist, und die letzten drei Codons immer CGT.CCC.TCA (Arg Pro Ser) sind.
   - Die Framework 3-Region weist immer 96 nt (also 32 aa) auf, wobei das erste Codon (aa) von FR3 immer: GGC (Gly) ist und die letzten vier Codons immer GAT.TAT.TAT.TGC (Asp Tyr-Tyr Cys) sind.
   - Die "CDR3-Region" kann drei verschiedene Langen aufweisen, entweder 24 nt (8 aa); 27 nt (9 aa); oder 30 nt (10 aa).
   - Die Framework 4-Region weist immer 39 nt (also 13 aa) und ist in allen 3 Familie identisch: GTG.TTT.GGC.GGC.GGC.ACG.AAG.TTA.ACC. GTT.CTT. GGC.CAG.

Das erfindungsgemäße Polypeptid kann für therapeutische oder diagnostische Zwecke, z.B für eine *in vitro* oder *in vivo* Diagnose verwendet werden.

Für therapeutische Anwendungen kann das erfindungsgemäße Polypeptid in Form eines Konjugats mit einem therapeutischen Wirkstoff, beispielsweise ausgewählt aus Radio- oder Chemotherapeutika, z.B. niedermolekularen oder biologischen zytostatischen oder zytotoxischen Wirkstoffen, vorliegen. Die Konjugation des therapeutischen Wirkstoffs an das Polypeptid kann nach bekannten Methoden, vorzugsweise über eine kovalente Kopplung an reaktive Amino-, Carboxy-, Hydroxy- oder/und Thiolgruppen des Proteins, gegebenenfalls unter Verwendung von homo- oder heterobifunktionellen Linke, nach bekannten Methoden erfolgen.

Darüber hinaus kann das Polypeptid auch in Form eines Fusionsproteins vorliegen, welches neben dem Antikörper, z.B. in Form eines IgG-Moleküls oder eines Fragments davon, ein daran fusioniertes Cytokin, z.B. IL2, II12 oder TNFa-Polypeptid, enthält. Weiterhin kann das Fusionsprotein auf in Form eines bispezifischen Antikörpers vorliegen, wobei neben der Bindung an die ED-B-Domäne eine Bindung an ein weiteres Antigen, bevorzugt ist. Weitere Antikörperspezifitäten bispezifischer Antikörper sind Bindedomänen gegen Chelatoren für diagnostisch oder/und therapeutisch relevante Radionuklide, z.B. α-, β- oder γ-Strahler wie etwa ⁹⁰Y, diagnostische NIR (Nahinfrarot)-Farbstoffe, therapeutisch wirksame Farbstoffe, Oberflächenmoleküle auf immunologischen Effektorzellen (z.B. NK-Zellen, cytotoxische T-Zellen oder NKT-Zellen), angiogenese-relevante Integrine, insbesondere Bindedomänen, die deren Funktion blockieren (z.B. α₁, β₃, α₁ß₃, α₂β₁, α₂β₂), inaktivierende anti VEGF-Bindedomänen und inaktivierende Bindedomänen gegen VEGF-Rezeptor 1, 2 und 3.

Für diagnostische Anwendungen kann das Polypeptid in Form eines Konjugats einer diagnostisch nachweisbaren Markierungsgruppe, z.B. einer Markierungsgruppe für eine *in vitro* oder eine *in vivo* Diagnostik, vorliegen. Beispiele für Markierungsgruppen sind radioaktive Markierungsgruppen, NMR-, Farbstoff-, Enzym- und Fluoreszenz (z.B. Fluoreszenz im Nahinfrarotbereich)-Markierungsgruppen.

Für therapeutische Anwendungen wird das Polypeptid vorzugsweise als pharmazeutische Zusammensetzung formuliert, die als Wirkstoff das erfindungsgemäße Polypeptid, gegebenenfalls weitere Wirkstoffe sowie pharmakologisch übliche Träger, Hilfsstoffe oder/und Verdünnungsmittel enthält. Die pharmazeutische Zusammensetzung enthält den Wirkstoff in einer therapeutisch wirksamen Dosis, die von einem Fachmann auf einfache Weise durch *in vitro* Versuche, beispielsweise an geeigneten Zellkulturen, oder in Tiermodellen bestimmt werden kann. Die Verabreichung der Zusammensetzung erfolgt vorzugsweise durch Injektion oder Infusion, aber auch andere Arten der Verabreichung sind denkbar. Bevorzugt erfolgt eine intravenöse oder/und subkutane Verabreichung. Die Dosis des verabreichten Wirkstoffs hängt von der Art und Schwere der Erkrankung sowie dem Zustand des zu behandelnden Patienten ab. Bevorzugt wird die therapeutische Zusammensetzung mehrmals über einen längeren Zeitraum von beispielsweise mindestens 2-4 Wochen verabreicht. Es wird in diesem Zusammenhang auf bekannte Verfahren zur Verabreichung von Antikörpern oder Antikörperkonjugaten, wie etwa in Ferarra, N. et al., Nature Reviews Drug Discovery, Volume 3, Mai 2004, 391-400 und Salgaller, M.L., Current Opinion in Molecular Therapeutics 2003 5(6):657-667, beschrieben, oder auf existierende Verabreichungsprotokolle für pharmazeutische Antikörper, wie Rituximab, CAMPATH, Remicade etc., verwiesen.

Ein weiterer Gegenstand der Erfindung eine diagnostische Zusammensetzung, umfassend als diagnostisches Reagenz ein erfindungsgemäßes Polypeptid. Daneben kann die diagnostische Zusammensetzung noch weitere diagnostisch übliche Reagenzien, Träger, Hilfsstoffe oder/und Verdünnungsmittel enthalten. Die diagnostische Zusammensetzung enthält das Polypeptid in einer ausreichenden Menge, um einen diagnostischen Nachweis in dem jeweiligen Format, z.B. in einem *in vivo* oder *in vitro* Diagnostikformat zu ermöglichen. Es wird in diesem Zusammenhang auf bekannte Verfahren zur *in vivo* und *in vitro* Diagnostik unter Verwendung von markierten Antikörpern verwiesen.

Die pharmazeutische und diagnostische Zusammensetzung können zur Therapie und Diagnose aller Erkrankungen, die mit einer ED-B-Expression, beispielsweise einer stromalen oder/und perivaskulären ED-B-Expression einhergehen. Beispiele für solche Erkrankungen sind hyperproliferative Erkrankungen, z.B. Erkrankungen, die mit Angiogenese assoziiert sind, insbesondere Krebs, Augenhintergrunderkrankungen, hypertrophe Narben etc., Erkrankungen, die mit einer Fehlfunktion von Myofibroblasten assoziiert sind, z.B. Endometriose, arteroisklerotische Plaques etc. oder entzündliche Erkrankungen, z.B. Psoriasis, Morbus Crohn, rheumatoide Arthritis oder Multiple Sklerose.

Die erfindungsgemäße pharmazeutische oder diagnostische Zusammensetzung kann ein oder mehrere wirksame Polypeptide enthalten, z.B. eine Kombination von Polypeptiden, die an unterschiedliche Bereiche der ED-B-Domäne binden. Die Zusammensetzungen sind zur Anwendung in der Human- und in der Veterinärmedizin geeignet.

Ein weiterer Gegenstand der Erfindung ist eine Nukleinsäure, die für ein erfindungsgemäßes Polypeptid bzw. Fusionspolypeptid kodiert. Diese Nukleinsäure kann eine einzelsträngige oder doppelsträngige DNA oder eine RNA sein. Die Nukleinsäure ist vorzugsweise in operativer Verknüpfung mit einer Expressionskontrollsequenz, die eine Expression in einer geeigneten Wirtszelle bzw. einem geeigneten Wirtsorganismus ermöglicht. Die Nukleinsäure kann auf einem Vektor vorliegen, der zur Einführung in eine Wirtszelle oder einen Wirtsorganismus geeignet ist. Der Vektor kann beispielsweise ein prokaryontischer Vektor, geeignet zur Einführung prokaryontische Zellen, z.B. ein Plasmid oder Bakteriophage, sein. Andererseits kann der Vektor auch eine eukaryontischer Vektor, geeignet zur Einführung in eukaryontische Wirtzellen bzw. Wirtsorganismen, z.B. ein Plasmid, ein artifizielles Chromosom oder ein viraler Vektor, sein. Geeignete Vektoren sind dem Fachmann beispielsweise aus Sambrook et al. (1989), Molecular Cloning, A Laboratory Manual, Cold Spring Harbor Laboratory Press, und Ausubel et al. (1989), Current Protocols in Molecular Biology, John Wiley and Sons, bekannt.

Noch ein weiterer Gegenstand der Erfindung ist eine Zelle, z.B. eine prokaryontische Zelle oder eine eukaryontische Zellen, wie etwa eine humane Zelle, die mit einer erfindungsgemäßen Nukleinsäure oder einem erfindungsgemäßen Vektor transformiert ist. Ein weiterer Gegenstand der Erfindung ist ein nicht-humaner Organismus, z.B. ein transgenes Tier, das mit einer erfindungsgemäßen Nukleinsäure oder einem erfindungsgemäßen Vektor transformiert ist. Der Begriff "Transformation" im Rahmen der vorliegenden Erfindung beinhaltet dabei sämtliche Möglichkeiten zum Einbringen von fremden Nukleinsäuren in eine Zelle oder einen Organismus einschließlich Transfektion oder Infektion.

Das erfindungsgemäße Polypeptid kann durch Kultivierung einer erfindungsgemäßen Zelle oder eines erfindungsgemäßen nicht-humanen Organismus unter Bedingungen erfolgen, bei denen eine Expression des Polypeptids stattfindet, und anschließend das exprimierte Polypeptid, z.B. aus der Zelle, dem Kulturmedium, dem Organismus oder Ausscheidungsprodukten des Organismus, gewonnen wird.

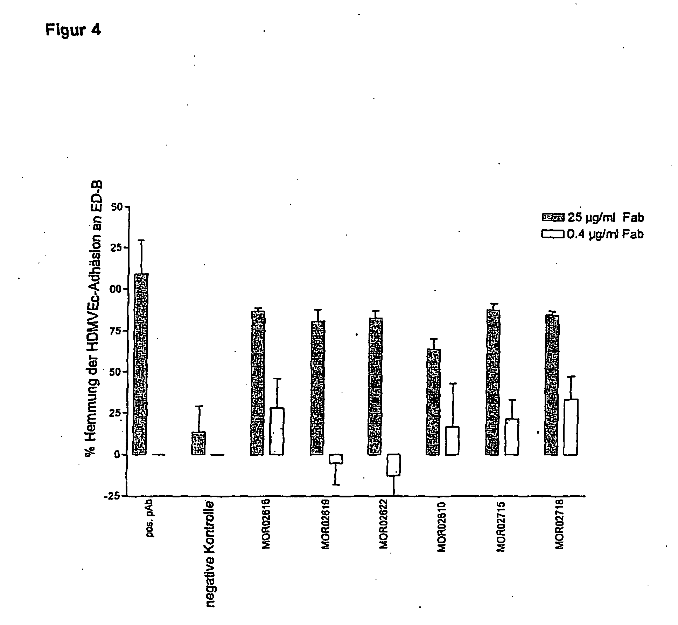

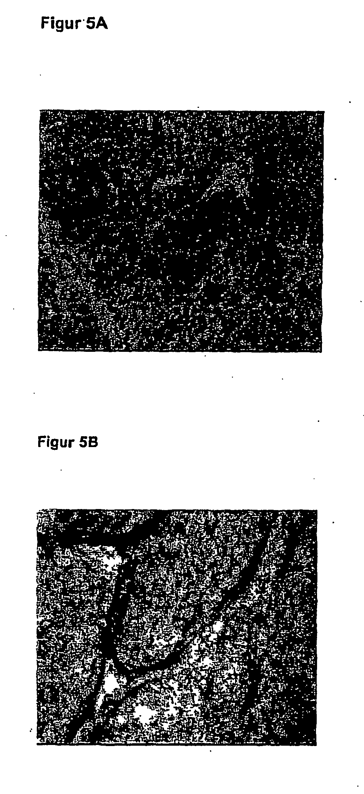

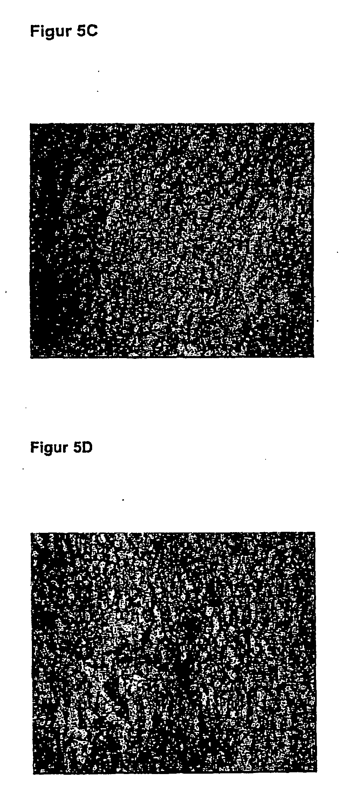

Weiterhin soll die Erfindung durch die nachfolgenden Figuren und Beispiele erläutert werden:

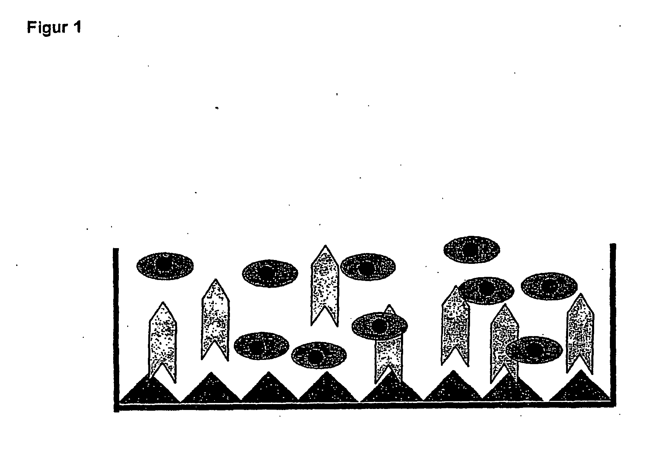

**Figur 1** zeigt die schematische Durchführung eines Inhibitionstests zur Identifizierung funktionell aktiver Antikörper.
   Menschliche Endothelzellen, die aus Mikrogefäßen der Haut gewonnen wurden (human dermal microvessel endothelial cells, HDMVEc) wurden zusammen mit ED-B-spezifischen Fab-Antikörperfragmenten inkubiert. Die Anzahl der gebundenen Zellen wird mittels Kristallviolett-Färbung sichtbar gemacht und im Photometer gemessen. Hohe Farbintensität bedeutet viele adhärierte Zellen und kein bindungsblockierender Antikörper. Geringe Farbintensität bedeutet wenig Adhäsion und ein bindungsblockierender Antikörper.

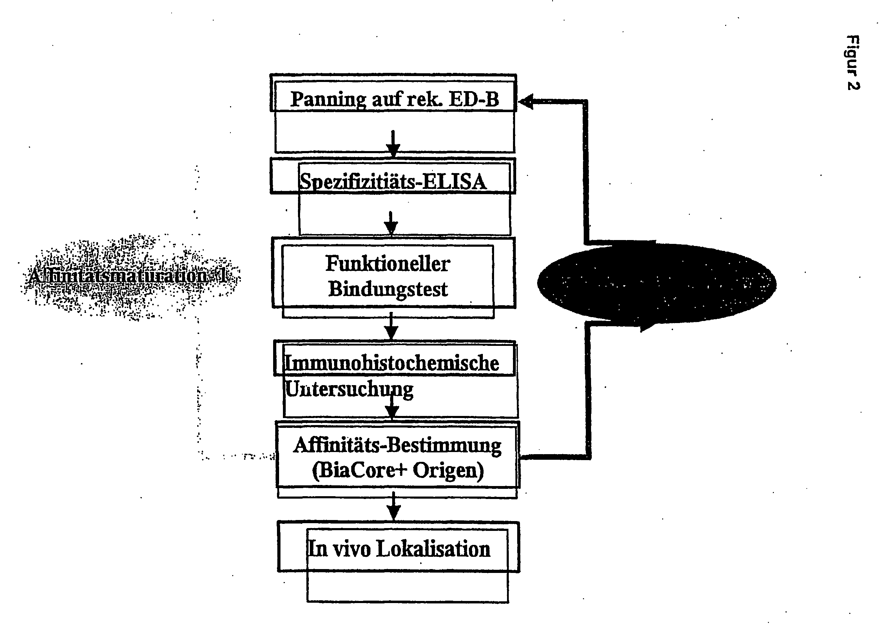

**Figur 2** zeigt ein Screening-Schema zur Identifizierung ED-B-Fibronektinfunktionsblockierender Antikörper mittel Fab-Fragment Display.
   In einem ersten Schritt wird eine Panning-Prozedur mit rekombinantem ED-B durchgeführt. Die auf diese Weise erhaltenen Antikörperfragmente werden einem Spezifitätstest (ELISA), einem funktionellen Adhäsionstest sowie einer immunhistochemischen Untersuchung unterzogen. Danach wird die Affinität der diese Tests erfüllenden Antikörper bestimmt. Anschließend werden zur Verbesserung der Affinität Veränderungen in den CDR-Domänen durchgeführt, wobei Affinitätsmaturation 1 eine Veränderung der L-CDR3-Domäne und Affinitätsmaturation 2 eine Veränderung der H-CDR2-Domäne bedeutet.
   Nachdem die durch Affinitätsmaturation erhaltenen Antikörper zuvor genannten Tests durchlaufen haben, wird anschließend die *in vivo* Wirksamkeit getestet.

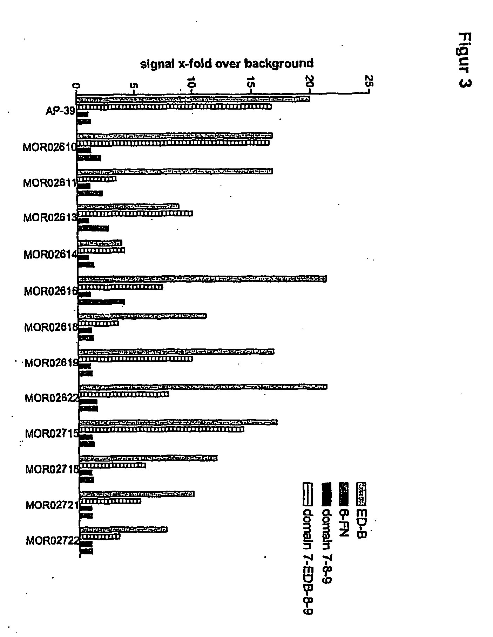

**Figur 3** zeigt die Ergebnisse eines Spezifitätstests im ELISA-Format mit den durch Panning mit rekombinantem ED-B erzeugten Antikörper-Fab-Fragmenten.
   ED-B bedeutet die Bindung an rekombinantes ED-B, 6-FN die Bindung an die rekombinante Fibronektin-Domäne 6, Domäne 7-8-9 bedeutet die Bindung an die rekombinanten Fibronektin-Domänen 7-8-9 (ohne ED-B) und Domäne 7-EDB-8-9 bedeutet die Bindung an die rekombinanten Fibronektin-Domänen 7-8-9 mit ED-B. Das Verhältnis von ED-B zu 6-FN bzw. 7-8-9 ergibt die Spezifität des untersuchten Antikörpers. AP-39 bedeutet ein kovalentes Dimer des biologisch inaktiven anti-ED-B scFv Antikörpers L19.
**Figur 4** zeigt das Ergebnis eines Adhäsionstests mit den untersuchten Antikörpern.
   Es wurde die Hemmung der Adhäsion von HDMVEc-Zellen an ED-B beschichtete Platten durch die angegebenen Fab-Fragmente bei Konzentrationen 25 µg/ml bzw. 0,4 µg/ml Fab untersucht. Die angegebenen Werte sind das Ergebnis einer Dreifach-Bestimmung.
**Figur 5** zeigt das immunhistochemische Reaktionsmuster funktionsblockierender Anti-ED-B-Fab-Fragmente am Beispiel von MOR 02616. In den Figuren 5A und 5C sind die Ergebnisse mit einer Negativkontrolle (negative HuCAL-Fab) und in den Figuren 5B und 5D die Ergebnisse mit dem Antikörper MOR 02616 an humanen Neuroblastomzellen-IRM Xenotransplantaten (Figuren 5A und 5B) sowie an murinen F9-Teratokarzinomzellen (Figuren 5C und 5D) gezeigt.
   Cryoschnitte der Zellen (Dicke: 10 µm) wurden luftgetrocknet und anschließend für 10 min in eiskaltem Aceton fixiert. Danach wurden die Schnitte in PBS gewaschen und für 60 min mit 2 µg/ml MOR 02616 bzw. Negativkontrolle bei Raumtemperatur inkubiert. Als Sekundärantikörper wurde ein Peroxidase-markiertes polyklonales Ziege anti Human F(ab)₂ Antiserum (1:100 in PBS verdünnt, Dianova) in Kombination mit Diaminobenzidin (Sigma Chemicals) als chromogenes Substrat verwendet.

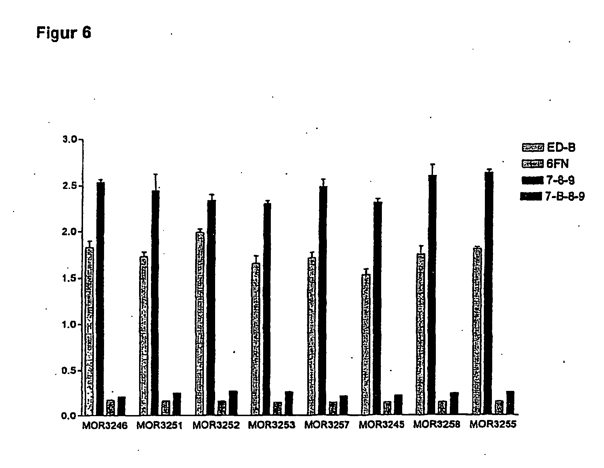

**Figur 6** zeigt die Ergebnisse eines Spezifitätstests im ELISA-Format mit den durch Maturierung optimierten Antikörper Fab-Fragmenten.
   Die Versuchsdurchführung war wie in Figur 3 beschrieben.

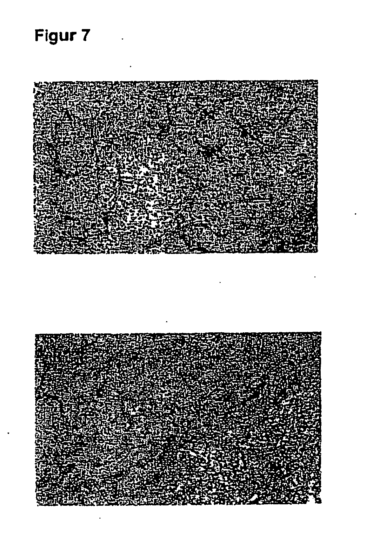

**Figur 7** zeigt das immunhistochemische Reaktionsmuster des durch Maturierung optimierten Antikörper Fab-Fragments MOR 03255 an Cryostatschnitten von F9-Teratokarzinomzellen (Figur 7A) und an SKMEL-28 Human-Melanomzell-Xenotransplantaten in der Nacktmaus (Figur 7B).
   Die Versuchsdurchführung war wie in Figur 5 beschrieben.

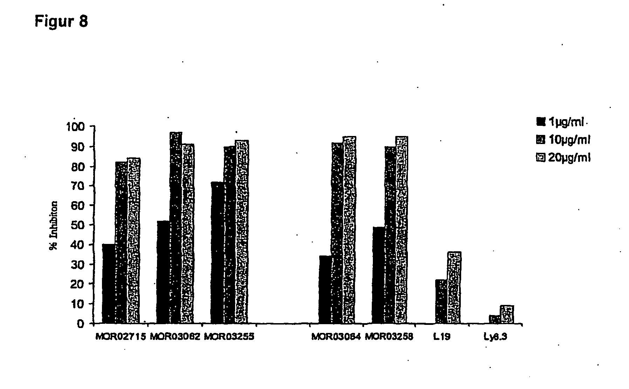

**Figur 8** zeigt das Ergebnis eines Adhäsionstests mit den erfindungsgemäßen Antikörper Fab-Fragmenten gegenüber den Vergleichsantikörpern L19 und Ly6 0.3.
   Die Versuchsdurchführung war ähnlich wie in Figur 4 beschrieben. Die Antikörperkonzentrationen waren 1 µl/ml, 10 µl/ml bzw. 20 µl/ml.

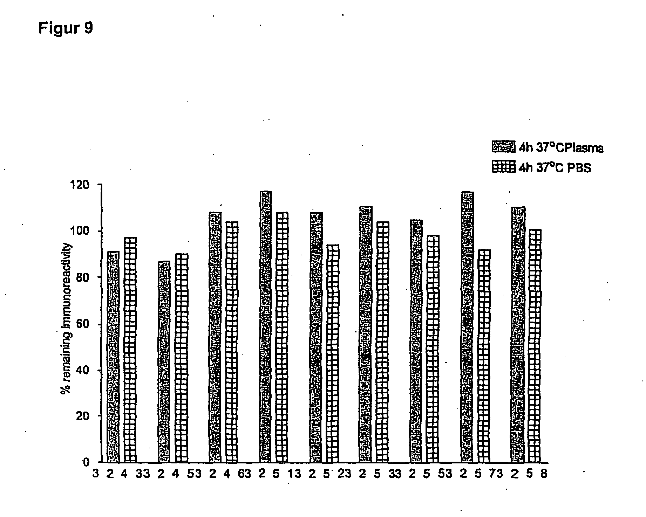

**Figur 9** zeigt die Stabilität der erfindungsgemäßen Antikörper-Fab-Fragmente nach Inkubation für 4 h in Humanplasma bzw. PBS bei 37 °C.
   Die Immunreaktivität wurde im ELISA-Test mit Konzentrationen im Bereich von 0,039 bis 5 µg/ml bestimmt. Als 100 %-Wert wurde das Signal mit 0,62 µg/ml in Humanplasma ohne Inkubation definiert.

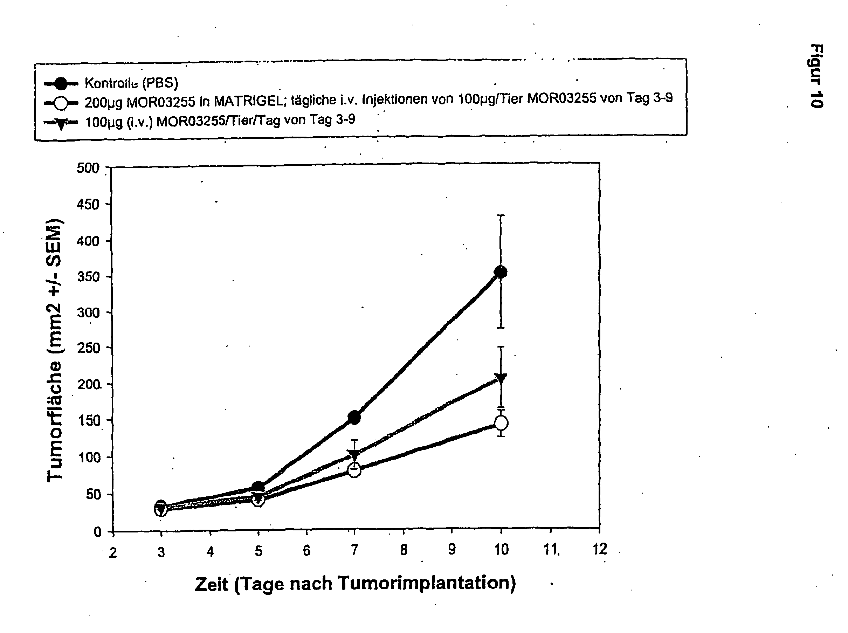

**Figur 10** zeigt die therapeutische Wirksamkeit von Anti-ED-B-funktionsblockierenden Fab-Antikörperfragmenten am Beispiel von MOR 03255.

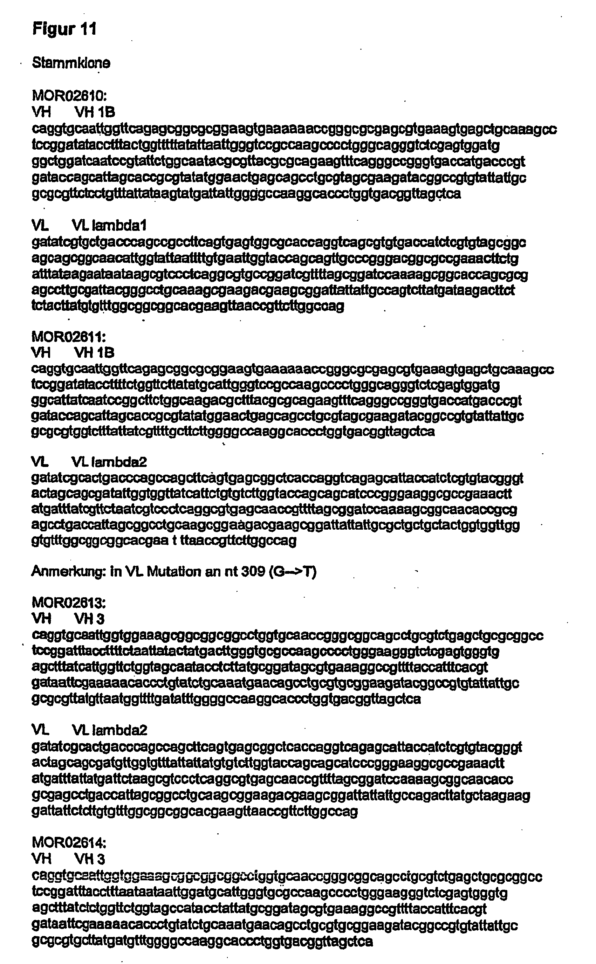

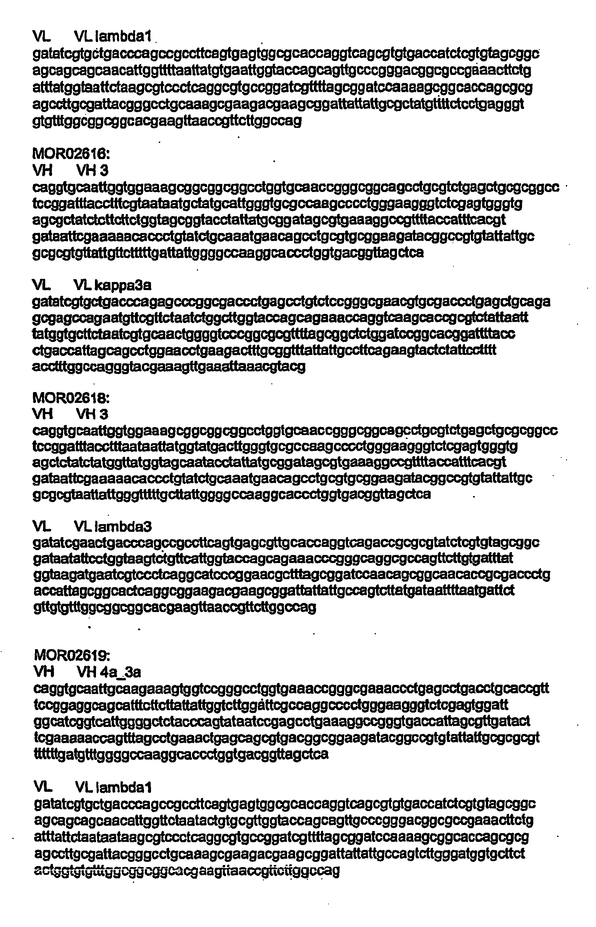

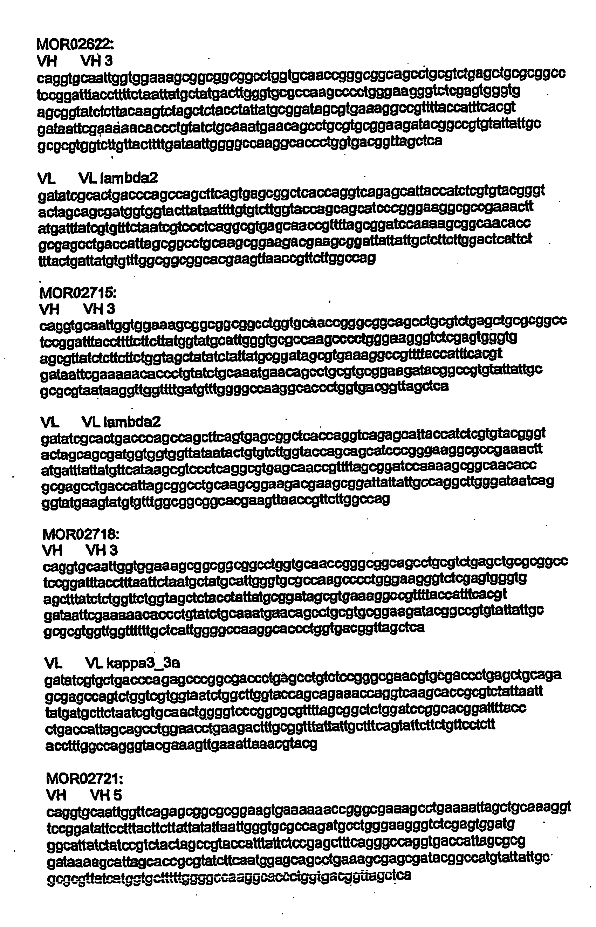

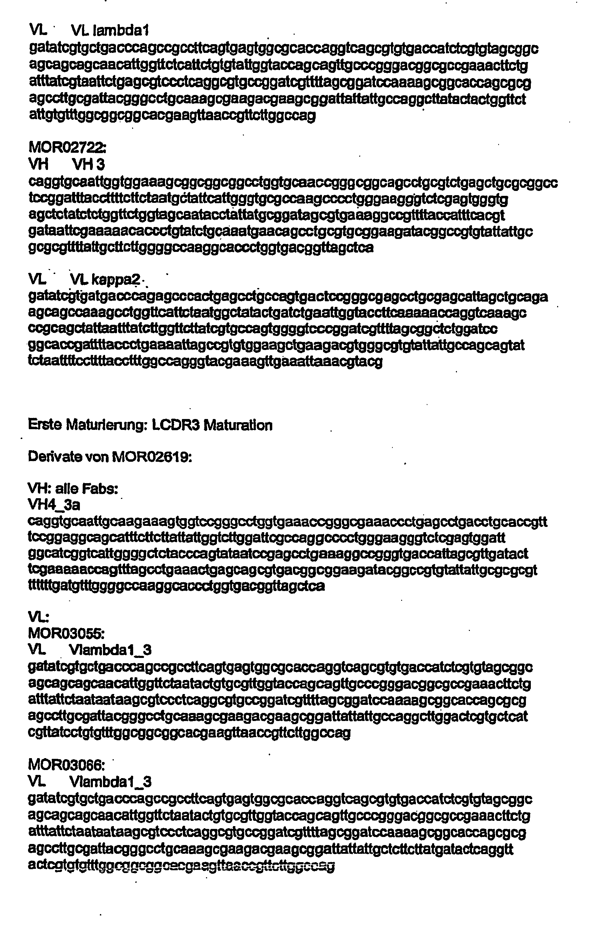

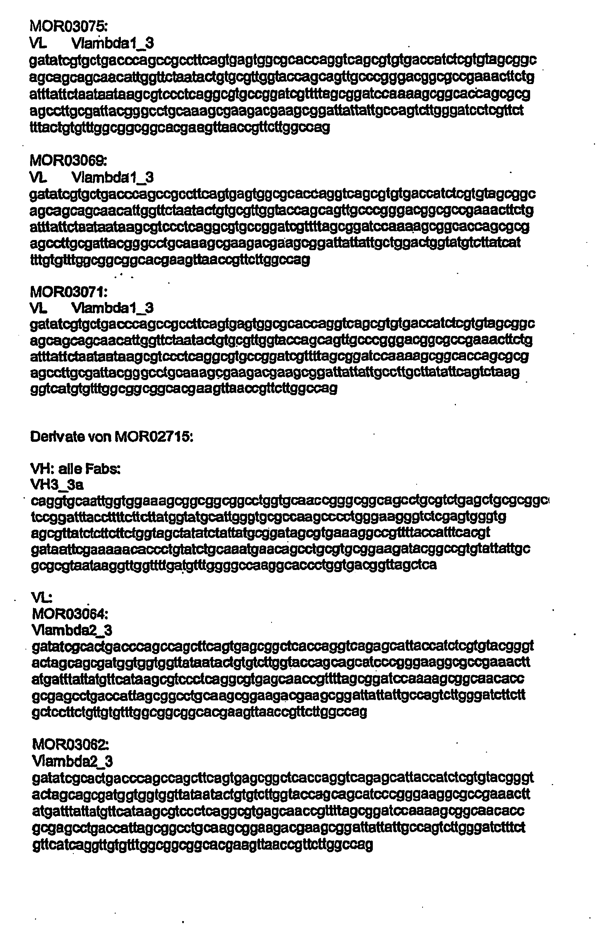

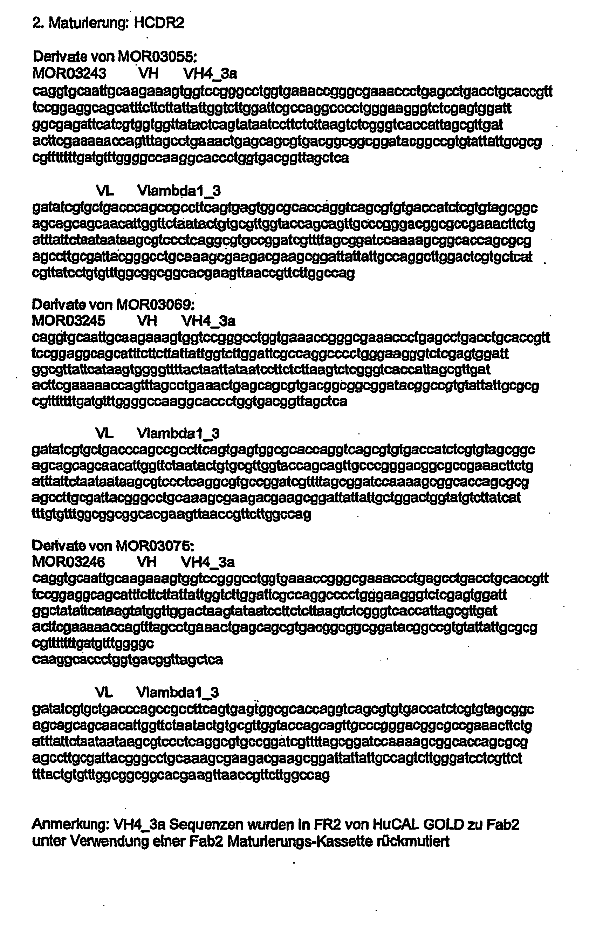

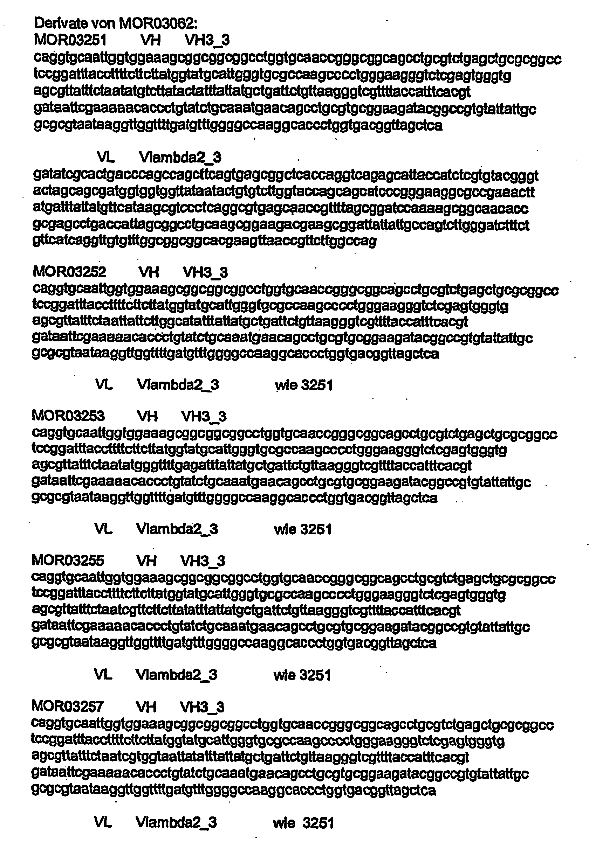

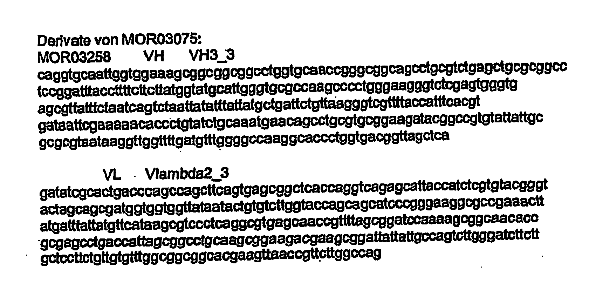

**Figur 11** zeigt die für die VH- und VL-Regionen der erfindungsgemäßen Antikörper kodierenden Nukleotidseqenzen.

### Beispiel

### 1. Material und Methoden

### 1.1 Proteine und Antikörper

Aufgereinigte rekombinante Fibronektin-Domäne ED-B (His)₆, Fibronektin-Domäne 6 (6FN), Fibronektin-Domäne 7-8-9 und Domäne 7-ED-B-8-9 wurden nach Standardprozeduren (z.B. Sambrook et al., Molecular Cloning. A Laboratory Manual, Cold Spring Harbour Press) hergestellt.

Mausantikörper gegen gereinigten ED-B-Rezeptor wurden ebenfalls nach Standardverfahren hergestellt, indem einer Maus 50 µg gereinigter Rezeptor in Freund's Komplett-Adjuvanz verabreicht wurden, 3 Wochen später 25 µg gereinigter Rezeptor in Freund's Inkomplett-Adjuvanz verabreicht wurden und weitere 3 Wochen später alle Applikationen in Inkomplett-Adjuvanz intraperitoneal verabreicht wurden. 14 Tage nach der 3. Immunisierung wurde dann Blut über den Retroorbitalsinus entnommen.
Plasmafibronektin wurde von Sigma bezogen.

### 1.2 Selektion von ED-B spezifischen Phagen

Die Selektion wurde nach dem HuCAL^{®} Gold Phage Selektionsprotokoll durchgeführt. Die HuCAL^{®} Gold-Bibliothek wurde in sechs Frameworkspezifische Pools unterteilt. Die ED-B spezifischen Phagen wurden in drei aufeinander folgenden Selektionsrunden an rekombinantem Protein immobilisiert in 96-Loch-Platten (Maxisorb, Nunc, Rochester , NY, USA) angereichert. Für das Panning A wurde die Platte mit 1 µg/Loch ED-B (His₆) (10 µg/ml in Ampuwa^{®}) für 2 h bei 37 °C beschichtet. Für das Panning B wurde die Platte mit ED-B in derselben Konzentration in PBS (pH 7,2 mit 1 mM MgCl₂/1 mM CaCl₂) über Nacht bei 4 °C beschichtet. Die Platten wurden mit 5 % Magermilchpulver in PBS, 0,05 % Tween20 (Sigma, St. Louis, MO, USA) (Blockierungspuffer) blockiert und mit 1 x 10¹³ HuCAL^{®} Gold Phagen inkubiert, die mit einem Volumen Blockierungspuffer vorinkubiert worden waren. Für das Panning B wurden die Phagen zusätzlich mit 0,5 µg/ml Fibronektin-Domäne 6 vorinkubiert, um die Selektion von Bindemolekülen zu verringern, die spezifisch für die konservierte Struktur eines Typ3 Domänen-Repeats von Fibronektin sind. Nach einer Inkubation mit den Phagen für 2 h bei Raumtemperatur wurden die Löcher 5x mit PBS, 0,05 % Tween20 und 5x mit PBS gewaschen. Die verbleibenden Phagen wurden 20 mM Dithiothreitol (DTT) in 10mM Tris/HCl (pH 8,0) für 10 min bei Raumtemperatur eluiert. Anschließend erfolgte eine Inkubation mit E.coli TG-1 (Stratagene, OD₆₀₀ = 0,5) zur Infektion. Danach wurden die Löcher mit E.coli TG-1 als zusätzlichem Elutionsschritt inkubiert.

### 1.3 Phagemidgewinnung, Phagenamplifikation und Aufreinigung

Die HuCAL^{®} Gold Phagen wurden in 2x TY-Medium mit 34 µg/ml Chloramphenicol und 1 % Glucose (2x TY-CG) amplifiziert. Nach Infektion mit einem Helferphagen (VCSM13) bei 37 °C und einer OD₆₀₀ von etwa 0,5, Zentrifugation und Resuspendierung in 2x TY-CG/50 µg/ml Kanamycin wurden die Zellen mit 0,25 mM IPTG induziert und über Nacht bei 22 °C kultiviert. Die Phagen wurden mit Polyethylenglycol aus dem Überstand präzipitiert (Ausubel et al. (1998), Current Protocols of Microbiology), in PBS resuspendiert und für nachfolgende Selektionsrunden verwendet.

### 1.4 Subklonierung von selektierten Fab-Fragmenten und Expression von löslichen Fab-Fragmenten

Die Fab-kodierenden Insertionen der selektierten HuCAL^{®} Gold Phagen wurden in den Expressionsvektor pMORPHx9-FS subkloniert. Die Plasmid-DNA-Präparation der selektierten HuCAL^{®} Gold Klone wurde mit den Restriktionsenzymen Xball/EcoRI gespalten, wobei die Fab-kodierende Insertion herausgeschnitten wurde (ompA-VL und phoA-Fd). In diesem Vektor exprimierte Fab-Moleküle enthalten zwei C-terminale Markierungen (FLAG^{™} und Strep-tagII) zum Nachweis und zur Aufreinigung.

### 1.5 Expression und Aufreinigung von HuCAL^{®} Gold Antikörpern in E.coli

Die Expression der auf pMORPHx9-FS-kodierten Fab-Fragmente in E.coli TG-1 F-Zellen wurde in Schüttelflaschenkulturen mit 0,75 I 2xTY-Medium und 34 µg/ml Chloramphenicol durchgeführt. Nach Induktion mit 0,75 mM IPTG wurden die Zellen für 16 h bei 30 °C kultiviert. Alternativ wurden Fab-Klone, die aus dem zweiten Maturationspool 2 erhalten worden waren, mit 0,1 mM IPTG induziert und anschließend bei 22 °C kultiviert. Periplasmatische Extrakte von Zellpellets wurden durch osmotischen Schock hergestellt und die Fab-Fragmente wurden durch strep-Tactin^{®}-Chromatographie (IBA, Göttingen, Deutschland) isoliert. Die apparenten Molekulargewichte wurden durch Größenausschlusschromatographie (SEC) mit Kalibrierungsstandards bestimmt. Die Konzentrationen wurden durch UV-Spektrophotometrie ermittelt.

### 1.6 Identifizierung von ED-B bindenden Fab-Fragmenten durch ELISA

96-Loch-Maxisorb ELISA-Platten wurden mit 100 µl ED-B-Lösung (10 µg/ml in Beschichtungspuffer (PBS pH 7,4, 1 mM CaCl₂, 1 mM MgCl₂) über Nacht bei 4 °C beschichtet. Es wurden rohe Lysate oder aufgereinigte Fab-Moleküle zugegeben, nicht bindende Fab-Moleküle wurden durch fünfmaliges Waschen mit Waschpuffer (PBS pH 7,4, 0,05 % Tween20) entfernt. Die Fab-Fragmente wurden durch Inkubation mit Anti-human-Fab-Antikörper-Peroxidase-Konjugaten (Dianova), gefolgt von Entwicklung mit einem löslichen Peroxidasesubstrat (Roche) und Messung bei 370 nm nachgewiesen. Die für ED-B spezifische Fab-Moleküle exprimierenden Klone wurden durch ein positives ELISA-Signal auf immobilisiertem ED-B gegenüber keinem oder nur einem geringen Signal bei 6FN identifiziert.

### 1.7 Bestimmung der Plasmastabilität unter physiologischen Temperaturbedingungen durch ELISA

Die Beschichtung mit ED-B (2,5 µg/ml) wurde im Wesentlichen wie oben beschrieben durchgeführt. Die Fab-Fragmente wurden für 4 h bei 37 °C und einer Konzentration von 50 µg/ml in humanem Plasma (Deutsches Rotes Kreuz; Charge 9985550) inkubiert. Nach Inkubation wurden die Fab-Moleküle für den ELISA-Test auf die Konzentrationen von 5,0, 2,5, 1,25, 0,62, 0,31, 0,156, 0,078 und 0,039 µg/ml in PBS mit 1 % Rinderserumalbumin verdünnt, um den linearen Bereich der Signalintensität zu bestimmen. Funktionelle Fab-Moleküle wurden mit dem anti-Flag M2-Antikörper (Sigma F3165) und einem sekundären anti-Maus-Antikörper-Alkalische Phosphatase-Konjugat, gefolgt durch Entwicklung mit Attophos (Roche) und durch Messung bein 535 nm bestimmt.

### 1.8 HDMVEc Zellkultur und Zelladhäsionstest

Humane dermale mikrovaskuläre Endothelialzellen (HDMVEc), isoliert aus juveniler Vorhaut, wurden in EGM-MV-Medium (Clonetics Inc.) unter Zusatz von 10 % fötalem Kälberserum, 2 mM Glutamin, 20 µg/ml Heparin und 3 ng/ml bFGF in Gefäßen, beschichtet mit 0,1 % Gelatine (Sigma), kultiviert. Für Adhäsionstests wurden Zellen verwendet, die nicht länger als Passage 8 kultiviert worden waren. ED-B (10 µg/ml) oder Plasmafibronektin (2,5 µg/ml) wurde in PBS pH 7,4, 0,9 mM CaCl₂, 0,5 mM MgCl₂ in 96-loch-ELISA-Platten über Nacht bei 4 °C immobilisiert und mit 1 % RSA in PBS pH 7,4, 0,9 mM CaCl₂, 0,5 mM MgCl₂ für 1 h bei 37 °C blockiert. Die Zellen wurden mit 0,15 µg/ml Calcein für 30 min bei 37 °C markiert und einmal in Adhäsionsmedium (MCDB 131, 2 mM Glutamin, 0,1 % RSA, 20 µg/ml Heparin) gewaschen. 1 x 10⁵ Zellen wurden mit der jeweils angegebenen Antikörperkonzentration, verdünnt in Adhäsionsmedium (Endvolumen 100 µl) für 20 min bei 37 °C inkubiert. Die Zellsuspension wurde zu den mit ED-B beschichteten Platten für 2 h bei 37 °C gegeben: Nach zweimaligem Waschen der Zellen in PBS pH 7,4, 0,9 mM CaCl₂, 0,5 mM MgCl₂ wurden adhärente Zellen in einem Plattenfluorimeter nachgewiesen (Anregung 485 nm; Emission 530 nm).

Zur Kontrolle wurde (A) die Zellbindung an Ligand-beschichteten Platten ohne Zugabe von spezifischen Antikörper und (B) die Zelladhäsion an Löcher ohne Ligandenbeschichtung bestimmt, wobei der bei der letztgenannten Kontrolle erhaltene Wert als Minimum der Zelladhäsion im Test definiert wurde.

### 1.9 Affinitätsmaturierung von von selektierten Fab-Molekülen durch schrittweisen Austausch von CDR-Kassetten

Zur Erhöhung der Affinität und biologischen Aktivität von ausgewählten Antikörperfragmenten wurden CDR-Regionen durch Kassettenmutagenese unter Verwendung von Trinukleotid-gerichteter Mutagenese optimiert.

Hierzu wurden Fab-Fragmente aus dem Expressionsvektor pMORPHx9 in den Phagemidvektor pMORPH-23 unter Verwendung der EcoRI/XbaI Restriktionsstellen kloniert. Zur Optimierung der Affinität der ausgewählten Fab-Fragmente wurden zwei aufeinanderfolgende Maturierungsschritte für jeden Maturierungspool durchgeführt. Im ersten Schritt wurde eine Antikörperfragment-Phagenbibliothek erzeugt, in der die L-CDR3-Region der Ausgangsklone durch ein Repertoire von 7 x 10⁸ (Pool 1) und 3,8 x 10⁸ (Pool 2) einzelnen Leichtketten-CDR-Sequenzen variiert wurde. In oder Affinität verbesserte Derivate aus dem ersten Maturierungsschritt wurden anschließend einer zweiten Maturierungsrunde ausgesetzt, indem die H-CDR-2-Region durch eine Bibliothek von diversifizierten Elementen ersetzt wurde. Auf diese Weise wurden zwei Phagenbibliotheken mit jeweils 1 x 10⁸ einzelnen Klonen erzeugt.

Die Affinitätsmaturierungsbibliotheken wurden durch Transformation in E.coli TOP10F erzeugt. Die Phagen wurden wie zuvor beschrieben hergestellt. Die Selektion auf Fab-Fragmente mit verbesserter Affinität wurde unter stringenten Bedingungen für drei Runden bei der ersten Maturierung und für zwei Runden beim zweiten Schritt durchgeführt.

### 1.10 Affinitätsbestimmung durch Oberflächenplasmonenresonanz (BIAcore^{®})

Zur Bestimmung der K_{D}-Werte wurden Monomerfraktionen (mindestens 80 % Monomergehalt, bestimmt durch analytische SEC; Superdex75, Amersham Pharmacia) von Fab-Fragmenten verwendet. F1-Chips (Biacore, Schweden) wurden mit ca. 200 RU ED-B (30µg/ml, 10 mM Acetatpuffer, pH 4,0) und Referenz-Flusszellen mit einer entsprechenden Menge von Humanserumalbumin (20 µg/ml, 10 mM Acetatpuffer, pH 4,5) unter Verwendung von Standard EDC-NHS-Aminkopplungschemie beschichtet. Die Antigendichte wurde für die Fab-Charakterisierung der zweiten Maturierung auf ca. 100 RU verringert. Die Regenerierung erfolgte mit 10 mM HCl. Alle kinetischen Messungen erfolgten in PBS-Puffer (136 mM NaCl, 2,7 mM KCI, 10 mM Na₂HPO₄, 1,76 mM KH₂PO₄ pH 7,4) mit einer Flussrate von 20 µl/min unter Verwendung eines Fab-Konzentrationsbereichs von 1,5-500 nM. Die Injektionzeit war jeweils 1 min. Alle Sensogramme wurden unter Verwendung der BIA-Evaluierungssoftware 3.1 ausgewertet.

Abkürzungen: EDC: 1-Ethyl-3-(3-dimethylaminopropyl)carbodiimid, NHS: N-Hydroxysuccinimid, RU: Resonanzeinheiten

### 2. Ergebnisse

### 2.1 Herstellung von Antikörpern gegen humanes ED-B

Es wurden zwei unterschiedliche Panning-Ansätze durchgeführt. Die Beschichtung mit ED-B für den ersten Ansatz (Panning A) wurde nach den aus dem biologischen Tests bekannten Bedingungen durchgeführt, um ED-B in einer Konformation zu präsentieren, welche die Adhäsion von HDMVEc-Zellen an das immobilisierte Antigen erlaubt. Beim zweiten Panning (B) wurden die Phagen zusätzlich mit einem Überschuss an 6FN blockiert, um eine Selektion von Bindemolekülen zu vermeiden, die mit der Fibronektin-Domäne 6 kreuzreagieren.

Insgesamt wurden 23 unterschiedliche HuCAL-Fab-Moleküle aus 1315 Primärtreffern gefunden, welche die Kriterien der Bindung an ED-B und nicht an 6FN erfüllen. Weiterhin wurden die Antikörper auf ihre Fähigkeit zur Bindung an ein rekombinant exprimiertes Fibronektin, umfassend die Domänen 7, 8 und 9 mit und ohne der inserierten ED-B-Domäne in ihrer natürlichen Anordnung getestet (siehe Figur 3). Alle getesteten Antikörper zeigten eine spezifische Erkennung für das 7-ED-B-8-9-Konstrukt, reagierten jedoch nicht mit dem entsprechenden Konstrukt ohne die ED-B-Domäne.

### 2.2 Funktionelle Charakterisierung der ED-B spezifischen Bindemoleküle

Die im ELISA als spezifisch identifizierten Antikörper wurden aufgereinigt und auf ihre Fähigkeit zur Blockierung der Adhäsion von HDMVEc-Zellen an mit ED-B beschichtete Platten getestet. Es wurde die Adhäsion von HDMVEc-Zellen in Gegenwart von ED-B spezifischen Fab-Fragmenten in zwei Konzentrationen im Vergleich zu einem unspezifischen HuCAL-Kontroll-Fab-Molekül und positiven Mauskontrollseren getestet.

Zwölf der 23 in Abschnitt 2.1 beschriebenen Fab-Moleküle zeigten eine Hemmung der Zelladhäsion an immobilisiertes ED-B. Eine repräsentative Auswahl der sechs wirksamsten Fab-Moleküle ist in Figur 4 gezeigt. Bei einer Konzentration von 25 µg/ml wurde die Zelladhäsion um 50 % bis nahezu 100 % gehemmt. Bei einer Konzentration von 0,4 µg/ml zeigten nur wenige Fab-Moleküle, z.B. MOR02610, MOR02616, MOR02715 und MOR02718 eine reproduzierbare Hemmung der Zelladhäsion.

Die funktionsblockierenden Fab-Antikörperfragmente wurden immunhistochemisch hinsichtlich ihrer Lokalisierung auf F9-Maus-Teratokarzimomzellen und humanen IRM30 Neuroblastom-Xenotransplantat-Cryoschnitten charakterisiert. Alle getesteten Antikörper zeigten eine vaskuläre Lokalisierung auf beiden Gewebeschnitten, aber keine Anfärbung von Gefäßen auf Normalgewebe (z.B. normale und sklerotische Leber und Haut). In Figur Figur 5B und 5D ist eine perivaskuläre abluminale Anfärbung von Gefäßen, proliferierendem Endothel und Tumorstroma durch MOR02616 gezeigt. Eine Ansammlung von ED-B in der Umgebung neovaskulärer Strukturen von exponentiell wachsenden Tumoren, aber nicht auf normalen Gefäßen, ist zu erkennen. Mit einem negativen Kontrollantikörper gegen Lysozym bei derselben Konzentration war keine Anfärbung sichtbar (Figuren 5A und 5C).

Die Eigenschaften von zwölf ausgewählten Bindemolekülen wurde durch BIAcore-Analyse bestimmt (siehe Tabelle 1). Die Affinitäten (Kₒₙ) lagen im Bereich von 15 bis 500 nM. Die Affinität des bivalenten L19-Derivats AP39 (eines biologisch inaktiven Vergleichsantikörpers gegen ED-B) wurde mit 2,4 nM bestimmt. Tabelle 1 zeigt auch die CDR-Sequenzen (nur L-CDR3 und H-CDR3) und die Framework-Kombinationen. Es wurden mindestens zwei unabhängige Messungen mit Fab-Molekülen aus unterschiedlichen Ansätzen zur Proteinexpression und -aufreinigung durchgeführt. Die Bindungsrate (Kₒₙ) und die Abdissoziationsrate (K_{off}) der Fab-Molekole ist in separaten Spalten angegeben.

**Tabelle 1**

| **Clone** | **VH** | **H-CDR3** | **VL** | **L-CDR3** | **kon** **[1/Ms]** | **koff** **[1/s]** | **KD** **[nM]** |
|---|---|---|---|---|---|---|---|
| AP 39 | VH3 | PFPY--FDY | K3 | QQTGRI--PP | 6,6E+05 | 1,5E-03 | 2,4 ±0,6 |
| MOR02610 | VH1B | SPVYYKYDY | L1 | QSYDKTSSTY | 1,3E+06 | 9,1E-02 | 75 ±19 |
| MOR02611 | VH1B | GLYYR-FAS | L2 | AAATG---GW | 1,5E+05 | 5,1E-02 | 332 ±23 |
| MOR02613 | VH3 | YVNG--FDI | L2 | QTYAKKDYSL | 7,8E+05 | 1,3E-01 | 164 ±14 |
| MOR02614 | VH3 | A-----YDV | L1 | AMFSP---EG | 2,8E+05 | 4,2E-02 | 160 ±6 |
| MOR02616 | VH3 | VIVL--FDY | K3 | LQKYSI--PF | 5,4E+05 | 2.8E-02 | 59±25 |
| MOR02618 | VH3 | NYWV--FAY | L3 | QSYDNFNDSV | 4,3E+05 | 2,0E-01 | 477 ± 177 |
| MOR02M9 | VH4 | F-----FDV | L1 | QSWDGAS-TG | 7,8E+05 | 1,2E-02 | 15,4±0,6 |
| MOR02622 | VH3 | GLVT--FDN | L2 | SSWTHSFTDY | 2,4E+05 | 5,1E-03 | 21,3±1.7 |
| MOR02715 | VH3 | NKVG--FDV | L2 | QAWDNQGMKY | 8,9E±05 | 4,7E-02 | 53,7 ±1,7. |
| MOR02718 | VH3 | GWF---FAH | k3 | FQYSSV--PL | 4,5E±05 | 3,9E-02 | 85±12,5 |
| MOR02721 | VH5 | YH----GAF | L1 | QAYTTG--SI | 4,3E+05 | 2,4E-02 | 56±11,6 |
| MOR02722 | VH3 | F-----IAS | K2 | QQYSNF--PF | 1,5E+05 | 1,4E-02 | 94 ± 2.3 |

### 2.3 Affinitätsmaturierungen von funktionsblockierenden ED-B-Antikörpern durch Optimierung der L-CDR3- und H-CDR2-Regionen

Die Affinitätsmaturierungen der Fab-Moleküle wurden in zwei Schritten durchgeführt. Zunächst wurde die L-CDR3-Sequenz diversifiziert und anschließend H-CDR2-Sequenz der im ersten Schritt erhaltenen, verbesserten Fab-Moleküle ausgetauscht.

Es wurden zwei Affinitätsmaturationsbibliotheken für jeden Schritt erzeugt. Die Optimierung wurde in zwei separaten Pools durchgeführt. Die L-CDR3-Bibliothek 1 mit einem Diversitätsgrad von 7 x 10⁸ Elementen enthielt die vier Ausgangs-Fab-Moleküle MOR02610, MOR02616, MOR02619 und MOR02622. Die L-CDR3-Bibiliothek 2 mit einem Diversitätsgrad von 3,8 x 10⁸ Elementen enthielt nur Derivate von MOR02715 und MOR02718. Die Fab-Moleküle wurden dabei jeweils entsprechend ihrer Affinität und biologischen Aktivität im Adhäsionstest in Gruppen zusammengefasst.

Beide Bibiliotheken wurden während der Selektitinsprozedur separat gehalte. Es wurden dabei zwei Panning-Strategien durchgeführt. Das Panning **I** erfolgte mit ED-B immobilisiert an Maxisorb-Platten für drei Runden, wie zuvor angegeben. Beim Panning II erfolgte eine Selektion an biotinyliertes ED-B in Lösung. Dabei wurde der Phage-Antigenkomplex durch Streptavidin-beschichtete Partikel in der ersten Runde und an Neutravidin-Platten in den zwei nachfolgenden Runden abgefangen. Im Rahmen der Selektionsprozedur wurden stringente Bedingungen verwendet.

Das Screening auf optimierte Fab-Moleküle erfolgte durch Bestimmung der K_{off}-Werte in BlAcore-System. Insgesamt wurden 11 Derivate der Bibiliothek 1 charakterisiert. Die 5 Derivate mit der höchsten Affinität sind in Tabelle 2 angegeben. Bei allen verbesserten Klonen handelte es sich dabei um Derivate von MOR02619 mit einer bis zu 7fachen Affinitätserhöhung. Beim Klon MOR03075 wurde eine Affinität von 2,4 nM gefunden.

Aus der Bibiliothek 2 wurden 2 Derivate von MOR02715 ausführlich analysiert. Für den Klon MOR03062 wurde eine 15fache Affinitätserhöhung gefunden, so dass eine Monomeraffinität von 2,4 nM erhalten wurde. Die Veränderungen in der Aminosäuresequenz der L-CDR3-Region ist in den Tabellen 2a und 2b gezeigt.

**Tabelle 2a**

| **Panning Pool 1** | **MOR0** | **Stammklon** | **LCDR3** | **kon** **[1/Ms]** | **koff** **[1/s]** | **KD** **[nm]** | **x fache Verbesserung gegenüber Stammklon** | | |
|---|---|---|---|---|---|---|---|---|---|
| | | | | | | | **kon** | **koff** | **KD** |
| | **AP 39-Biotin** | | QQTGRI -- PP | 6,6E + 05 | 1,49E - 003 | **2,4 ± 0,8** | | | |
| | **2619** | | QSWDGAS-TG | 7,8E + 05 | 1,2E -02 | **15,4 ± 0,6** | | | |
| fest | 3055 | 2619 | QAWTRAHRYP | 1,8E+08 | 5,1E , 03 | **3,0 ± 0,5** | 2,2 | 2,6 | **5,4** |
| gelöst | 3066 | 2619 | SSYD-TQVTR | 1,1E ± 06 | 4,7E - 03 | **4,2 ± 0,6** | 1,4 | 2,8 | **3,8** |
| gelöst | 3075 | 2619 | QSWDP-RSFT | 1,1E + 08 | 2,6E -03 | **2,4 ± 0,3** | 1,4 | 5.0 | **6,8** |
| gelöst | 3069 | 2619 | WTGM - - SYHF | 1,2E + 06 | 4,1E - 03 | **3.310,2** | 1,5 | 3,2 | **4,8** |
| gelöst | 3071 | 2619 | LAYIQS-KGH | 1,9E+06 | 5,7E - 03 | **3,1±0,8** | 2,3 | 2,3 | **5,1** |

**Tabelle 2b**

| **Panning Pool 2** | **MOR0** | **Stammklon** | **LCDR3** | **kon** **[1/Ms]** | **koff** **[1/s]** | **KD** **[nm]** | **× fache Verbesserung gegenüber Stammklon** | | |
|---|---|---|---|---|---|---|---|---|---|
| | | | | | | | **kon** | **koff** | **KD** |
| | **AP 39-Biotin** | | QQTGRI--PP | 6,75E + 05 | 1,49E -03 | **2,4 ± 0,8** | | | |
| | 2715 | | QAWDNQGMKY | 9,9E + 05 | 4,7E-02 | **53,7 ± 1,7** | | | |
| fest | 3064 | 2715 | SWDLLAPSV | 1,5E + 06 | 1,4E -02 | **10 ± 3,5** | 1,7 | 3,5 | **5,3** |
| fest | 3062 | 2715 | QSWDLSVHQV | 3,5E+ 0,6 | 1,1 E-02 | **3,4 ± 0,8** | 4,0 | 4,2 | **15,8** |

Die Spezifität für ED-B (bestimmt durch ELISA, Immunhistochemie und Zelladhäsionstest) aller Derivate aus der ersten Maturierungsrunde waren unverändert.

Die in Tabellen 2a und 2b gezeigten Fab-Moleküle wurden für eine anschließende H-CDR2-Maturierung in zwei Pools ausgewählt. In Pool 1 wurden fünf Fab-Moleküle mit einer ähnlichen Aktivität, aber unterschiedlichen L-CDR3-Regionen einer Diversifizierung in H-CDR2 unterzogen (Bibiliotheksgröße von 7 x 10⁷ Elementen). Für Pool 2 (Bibiliotheksgröße von 8,5 x 10⁷ Elementen) wurden zwei Fab-Moleküle ausgewählt. Die Bibliotheken wurden separat wie oben beschrieben selektioniert. Bindemoleküle mit erhöhter Affinität wurden durch zwei Panningrunden in Lösung unter stringenten Bedingungen angereichert.

Aus Pool 1 wurden drei Fab-Moleküle ausgewählt. Für die Derivate MOR03243 und MOR03245 wurden Affinitäten von etwa 0,7 nM gefunden. Für das Derivat MOR03246 wurde eine Affinität von 0,6 nM gefunden.

Die H-CDR2-Sequenzen der entsprechenden Klone und die Affinitätsdaten sind in Tabelle 3a gezeigt.

**Tabelle 3a**

| **MOR0** | **Parental Clone** | **Sequenz HCDR2** | **kon [1/Ms]** | **koff [1/s]** | **KD [nM]** |
|---|---|---|---|---|---|
| MOR03243 | 3055 | EIHRGGYTQYNPSLKS | 2,9E+06 | 1,8E-03 | 0,7 ± 0.2 |
| MOR03245 | 3069 | VIHKWGFTNYNPSLKS | 3,5E+06 | 1,6E-03 | 0,7 ± 0,3 |
| MOR03246 | 3075 | YIHKYGWTKYNPSLKS | 4,8E+06 | 3,0E-03 | 0,6 ± 0,1 |

Auch aus Pool 2 konnten Derivate mit verbesserten Eigenschaften selektioniert werden. Im Vergleich zu den Stammklonen waren die Affinitäten der Derivate um einen Faktor von 26 bis zu 250fach verbessert. Die Affinitäten der Klone MOR03255 und MOR03258 wurden mit 30 pM bzw. 40 pM bestimmt.

In Tabelle 3b sind H-CDR2-Aminosäuresequenzen der selektierten Klone aus Pool 2 sowie die entsprechenden Affinitätsdaten gezeigt.

**Tabelle 3b**

| **MOR0** | **Parental Clone** | **Sequenz HCDR2** | **kon [1/Ms]** | **koff [1/s]** | **KD [nM]** | **STDEV** |
|---|---|---|---|---|---|---|
| MOR03251 | 3062 | VISNMSYTIYYADSVKG | 3,3E+06 | 2,0E-04 | 0,07 | 0,04 |
| MOR03252 | 3062 | VISNYSWHIYYADSVKG | 3,1E+08 | 6,1E-05 | 0,03 | 0,03 |
| MOR03253 | 3062 Mut | VISNMGFEIYYADSVKG | 1,6E+08 | 2,0E-04 | 0,13 | 0,05 |
| MOR03255 | 3062 | VISNRSSYIYYADSVKG | 4,9E+06 | 8,6E-05 | 0,03 | 0,03 |
| MOR03257 | 3062 | VISNRGNYIYYADSVKG | 5,3E+06 | 3,0E-04 | 0,08 | 0,06 |
| MOR03258 | 3064 | VISNQSNYIYYADSVKG | 2,7E+06 | 8,8E-05 | 0,04 | 0,02 |

### 2.4 Charakterisierung von hochaffinen funktionsblockierenden ED-B-Antikörpern

Um zu testen, ob die durch Affinitätsmaturierung erhaltenen Antikörper immer noch für ED-B spezifisch sind, wurden Spezifitätstests im ELISA-Format (Figur 6), immunhistochemische Untersuchungen (Figur 7) und Adhäsionstests (Figur 8) durchgeführt. In allen drei Tests wurde gefunden, dass die Spezifität der affinitätsmaturierten Fab-Moleküle für ED-B unverändert blieb. Aus Figur 8 ist weiterhin ersichtlich, dass sowohl die Stammklone wie MOR 02715 als auch die durch Affinitätsmaturierung erzeugten Klone wie MOR 03062, MOR 03255, MOR 03064 und MOR 03259 eine signifikant höhere biologische Aktivität (Adhäsionshemmung) als der bekannte Antikörper L19 aufweisen.

Zusätzlich wurde die thermische Stabilität der Fab-Moleküle in Humanplasma getestet. Hierzu wurden die Fab-Moleküle mit humanem Plasma für 4 h bei 37°C inkubiert, ohne dass ein signifikanter Verlust an Immunreaktivität bestimmt durch ELISA (Figur 9) gefunden wurde.

Alle-selektierten Fab-Moleküle sind in der Lage, die Wechselwirkung eines Rezeptors auf humanen mikrovaskulären Endothelzellen an ED-B auf dosisabhängige Weise zu blockieren, eine Eigenschaft, der bekannte ED-B-Antikörper L19 nicht aufweist. Da die Adhäsion von HDMVEc an die extrazelluäre Matrix einer der frühen Schritte in der Neoangiogenese ist, stellt die Blockierung dieses Vorgangs durch die erfindungsgemäßen Bindemoleküle ein therapeutisches Mittel zur Hemmung der Bildung von neuen Gefäßen und zur Hemmung des Tumorwachstums dar.

### 2.5 In vivo Wirksamkeit

F9-Teratokarzinomazellen (10⁶/Maus in Matrigel mit/ohne 100µg Fab MOR 03255) wurden sc. in die Flanke von Nacktmäusen implantiert. Nach 3 Tagen wurden die Tiere mit jeweils 100 µg MOR03255/Maus i.v. für 7 aufeinanderfolgendeTage behandelt. Beide Behandlungsgruppen zeigen im Vergleich zur Lösungsmittelkontrolle eine.Wachstumshemmung des Tumors im Bereich von 42-64 %. Eine Zusammenfassung der Versuchsergebnisse ist in Figur 10 dargestellt.

### Literatur

(1)Brown LF, Guidi AJ, Schnitt SJ, Van De Water L, Iruela-Arispe ML, Yeo T-K, Tognazzi K, Dvorak HF (1999) Clin. Cancer Res. 5: 1041-1056
(2)Folkman J (1995) Nature (Med.) 1: 27-31
(3)Risau W und Lemmon V (1988) Develop. Biol. 125: 441-450
(4)Van Den Hoff A (1988) Adv. Cancer Res. 50: 159-196
(5)Folkman J und Shing Y (1992) J. Biol. Chem. 267: 10931-10934
(6)George EL, Baldwin HS, Hynes RO (1997) Blood 90: 3073-3081
(7)Bowersox JC und Sorgente N (1982) Cancer Res. 42: 2547-2551
(8)Madri JA, Pratt BM, Tucker AM (1988) J. Cell Biol. 106: 1375- 1384
(9)Nicosia RF, Bonnano E, Smith M (1993) J. Cell. Physiol. 154: 654-661
(10)Kornblihtt AR, Vibe-Pedersen K und Baralle FE, 1983. Isolation and characterization of cDNA clones for human and bovine fibronectins. Proc. Natl. Acad. Sci. USA, 80, 3218-22
(11)Leahy DJ, Hendrickson WA, Aukhil I und Erickson HP. 1992. Structure of fibronectin type III domain from tenascin phased by MAS analysis of the selenomethionyl protein. Science, 258, 987-91
(12)Hynes R.O., 1987, Cell 48, 549-550
(13)Plow E.F. et al. 2000, J. Biol. Chem., 275, 21785-21788
(14)Castellani P, Viale G, Dorcaratto A, Nicolo G, Kaczmarek J, Querze G, Zardi L (1994) Int. J. Cancer 59: 612-618
(15)Hashimoto-Uoshima M, Yan YZ, Schneider G, Aukhil I (1997) J. Cell Science 110: 227-2280
(16)Chen W and Culp LA (1996) Exp. Cell Res. 223: 9-19
(17)Chen W and Culp LA (1998) Clin. Exp. Metastasis 16: 30-42

## Patentansprüche

1. Antikörper oder Antikörperfragment,
der bzw. das spezifisch an die ED-B Domäne von Fibronektin bindet
**dadurch gekennzeichnet,**
**dass** er bzw. es die VH-Region kodiert von SEQ ID NO. 75 (MOR 03251) und die VL-Region kodiert von SEQ ID NO. 77 (MOR 03251) oder die H-CDR1-, H-CDR2- und H-CDR3- Region aus dieser VH Region und die L-CDR1-, L-CDR2- und L-CDR3- Region aus dieser VL Region aufweist, wobei der Antikörper oder das Antikörperfragment die ED-B-Domäne mit einem KD-Wert von 0,1 nM oder niedriger bindet und der KD-Wert durch Oberflächenplasmonenresonanz ermittelt wird, und die Interaktion zwischen der ED-B-Domäne und ihrem Rezeptor inhibiert.

2. Antikörper oder Antikörperfragment,
der bzw. das spezifisch an die ED-B Domäne von Fibronektin bindet,
**dadurch gekennzeichnet,**
**dass** er bzw. es die VH-Region kodiert von SEQ ID NO. 79 (MOR 03252) und die VL-Region kodiert von SEQ ID NO. 77 (MOR 03252) oder die H-CDR1-, H-CDR2- und H-CDR3- Region aus dieser VH Region und die L-CDR1-, L-CDR2- und L-CDR3- Region aus dieser VL Region aufweist, wobei der Antikörper oder das Antikörperfragment die ED-B-Domäne mit einem KD-Wert von 0,1 nM oder niedriger bindet und der KD-Wert durch Oberflächenplasmonenresonanz ermittelt wird, und die Interaktion zwischen der ED-B-Domäne und ihrem Rezeptor inhibiert.

3. Antikörper oder Antikörperfragment,
der bzw. das spezifisch an die ED-B Domäne von Fibronektin bindet"
**dadurch gekennzeichnet,**
**dass** er bzw. es die VH-Region kodiert von SEQ ID NO. 83 (MOR 03255) und die VL-Region kodiert von SEQ ID NO. 77 (MOR 03255) oder die H-CDR1-, H-CDR2- und H-CDR3- Region aus dieser VH Region und die L-CDR1-, L-CDR2- und L-CDR3- Region aus dieser VL Region aufweist, wobei der Antikörper oder das Antikörperfragment die ED-B-Domäne mit einem KD-Wert von 0,1 nM oder niedriger bindet und der KD-Wert durch Oberflächenplasmonenresonanz ermittelt wird, und die Interaktion zwischen der ED-B-Domäne und ihrem Rezeptor inhibiert.

4. Antikörper oder Antikörperfragment,
der bzw. das spezifisch an die ED-B Domäne von Fibronektin bindet,
**dadurch gekennzeichnet,**
**dass** er bzw. es die VH-Region kodiert von SEQ ID NO. 85 (MOR 03257) und die VL-Region kodiert von SEQ ID NO. 77 (MOR 03257) oder die H-CDR1-, H-CDR2- und H-CDR3- Region aus dieser VH Region und die L-CDR1-, L-CDR2- und L-CDR3- Region aus dieser VL Region aufweist, wobei der Antikörper oder das Antikörperfragment die ED-8-Domäne mit einem KO-Wert von 0,1 nM oder niedriger bindet und der KD-Wert durch Oberflächenplasmonenresonanz ermittelt wird, und die Interaktion zwischen der ED-B-Domäne und ihrem Rezeptor inhibiert.

5. Antikörper oder Antikörperfragment,
der bzw. das spezifisch an die ED-B Domäne von Fibronektin bindet,
**dadurch gekennzeichnet,**
**dass** er bzw. es die VH-Region kodiert von SEQ ID NO. 87 (MOR 03258) und die VL-Region kodiert von SEQ ID NO. 59 (MOR 03258) oder die H-COR1-, H-CDR2- und H-CDR3- Region aus dieser VH Region und die L-CDR1-, L-CDR2- und L-CDR3- Region aus dieser VL Region aufweist, wobei der Antikörper oder das Antikörperfragment die ED-B-Domäne mit einem KD-Wert von 0,1 nM oder niedriger bindet und der KD-Wert durch Oberflächenplasmonenresonanz ermittelt wird, und die Interaktion zwischen der ED-B-Domäne und ihrem Rezeptor inhibiert.

6. Antikörper oder Antikörperfragment nach einem der Ansprüche 1 bis 5 in Form eines Konjugats mit einem therapeutischen Wirkstoff.

7. Antikörper oder Antikörperfragment nach Anspruch 6,
**dadurch gekennzeichnet,**
**dass** der therapeutische Wirkstoff ausgewählt ist aus Radio- und Chemotherapeutika.

8. Antikörper oder Antikörperfragment nach einem der Ansprüche 1 bis 5 in Form eines Fusionsproteins.

9. Antikörper oder Antikörperfragment nach einem der Ansprüche 1 bis 5 in Form eines Konjugats mit einer diagnostisch nachweisbaren Markierungsgruppe.

10. Pharmazeutische Zusammensetzung,
**dadurch gekennzeichnet,**
**dass** sie als Wirkstoff einen Antikörper oder Antikörperfragment nach einem der Ansprüche 1 bis 8 sowie pharmakologisch übliche Träger, Hilfsstoffe oder/und Verdünnungsmittel enthält.

11. Diagnostische Zusammensetzung, welche als diagnostisches Reagenz einen Antikörper oder Antikörperfragment nach einem der Ansprüche 1 bis 5, oder 9 enthält.

12. Nukleinsäure, die für ein Antikörper oder Antikörperfragment nach einem der Ansprüche 1 bis 5 oder 8 kodiert.

13. Nukleinsäure nach Anspruch 12 in operativer Verknüpfung mit einer Expressionskontrollsequenz.

14. Vektor,
**dadurch gekennzeichnet,**
**dass** er eine Nukleinsäure nach einem der Ansprüche 12 bis 13 enthält.

15. Isolierte Zelle,
**dadurch gekennzeichnet,**
**dass** sie mit einer Nukleinsäure nach einem der Ansprüche 12 bis 13 oder einem Vektor nach Anspruch 14 transformiert ist.

16. Nicht-humaner Organismus,
**dadurch gekennzeichnet,**
**dass** er mit einer Nukleinsäure nach einem der Ansprüche 12 bis 13 oder einem Vektor nach Anspruch 14 transformiert ist.

17. Verfahren zur Herstellung eines Antikörpers oder Antikörperfragments nach einem der Ansprüche 1 bis 5,
**dadurch gekennzeichnet,**
**dass** man eine Zelle nach Anspruch 15 oder einen nicht-humanen Organismus nach Anspruch 16 unter Bedingungen kultiviert, bei denen eine Expression des Antikörpers oder Antikörperfragments erfolgt, und der exprimierte Antikörper oder Antikörperfragment gewonnen wird.

18. Verwendung eines Antikörpers oder Antikörperfragments nach einem der Ansprüche 1 bis 8 zur Herstellung eines Arzneimittels zur Prävention oder Behandlung von Erkrankungen die mit einer ED-B Expression einhergehen und die ausgewählt sind aus hyperproliferativen Erkrankungen, Erkrankungen, die mit einer Fehlfunktion von Myofibroblasten assoziiert sind, und entzündlichen Erkrankungen.

19. Verwendung eines Antikörpers oder Antikörperfragments nach einem der Ansprüche 1 bis 9 zur Herstellung eines Nachweisreagenzes zur Diagnose von Erkrankungen die mit einer ED-B Expression einhergehen und die ausgewählt sind aus hyperproliferativen Erkrankungen, Erkrankungen, die mit einer Fehlfunktion von Myofibroblasten assoziiert sind, und entzündlichen Erkrankungen, oder einer Prädisposition dafür.

## Claims

1. An antibody or antibody fragment,
specifically binding to the ED-B domain of fibronectin,
**characterized in that**
the same codes the VH region of SEQ ID NO. 75 (MOR 03251) and the VL region of SEQ ID NO. 77 (MOR 03251), or comprises the H-CDR1, H-CDR2, and H-CDR3 region from said VH region, and the L-CDR1, L-CDR2, and L-CDR3 region of said VL region, wherein the antibody or the antibody fragment binds the ED-B domain at a KD value of 0.1 nM or lower, and the KD value is determined by means of surface plasmon resonance, and inhibits the interaction between the ED/B domain and the receptor thereof.

2. The antibody or antibody fragment,
specifically binding to the ED-B domain of fibronectin,
**characterized in that**
the same codes the VH region of SEQ ID NO. 79 (MOR 03252) and the VL region of SEQ ID NO. 77 (MOR 03252), or comprises the H-CDR1, H-CDR2, and H-CDR3 region from said VH region, and the L-CDR1, L-CDR2, and L-CDR3 region of said VL region, wherein the antibody or the antibody fragment binds the ED-B domain at a KD value of 0.1 nM or lower, and the KD value is determined by means of surface plasmon resonance, and inhibits the interaction between the ED/B domain and the receptor thereof.

3. The antibody or antibody fragment,
specifically binding to the ED-B domain of fibronectin,
**characterized in that**
the same codes the VH region of SEQ ID NO. 83 (MOR 03255) and the VL region of SEQ ID NO. 77 (MOR 03255), or comprises the H-CDR1, H-CDR2, and H-CDR3 region from said VH region, and the L-CDR1, L-CDR2, and L-CDR3 region of said VL region, wherein the antibody or the antibody fragment binds the ED-B domain at a KD value of 0.1 nM or lower, and the KD value is determined by means of surface plasmon resonance, and inhibits the interaction between the ED/B domain and the receptor thereof.

4. The antibody or antibody fragment,
specifically binding to the ED-B domain of fibronectin,
**characterized in that**
the same codes the VH region of SEQ ID NO. 85 (MOR 03257) and the VL region of SEQ ID NO. 77 (MOR 03257), or comprises the H-CDR1, H-CDR2, and H-CDR3 region from said VH region, and the L-CDR1, L-CDR2, and L-CDR3 region of said VL region, wherein the antibody or the antibody fragment binds the ED-B domain at a KD value of 0.1 nM or lower, and the KD value is determined by means of surface plasmon resonance, and inhibits the interaction between the ED/B domain and the receptor thereof.

5. The antibody or antibody fragment,
specifically binding to the ED-B domain of fibronectin,
**characterized in that**
the same codes the VH region of SEQ ID NO. 87 (MOR 03258) and the VL region of SEQ ID NO. 59 (MOR 03258), or comprises the H-CDR1, H-CDR2, and H-CDR3 region from said VH region, and the L-CDR1, L-CDR2, and L-CDR3 region of said VL region, wherein the antibody or the antibody fragment binds the ED-B domain at a KD value of 0.1 nM or lower, and the KD value is determined by means of surface plasmon resonance, and inhibits the interaction between the ED/B domain and the receptor thereof.

6. The antibody or antibody fragment according to one of the claims 1 to 5 in the form of a conjugate having a therapeutic active agent.

7. The antibody or antibody fragment according to claim 6,
**characterized in that**
the active agent is selected from radio or chemotherapeutic agents.

8. The antibody or antibody fragment according to one of the claims 1 to 5 in the form of a fusion protein.

9. The antibody or antibody fragment according to one of the claims 1 to 5 in the form of a conjugate having a diagnostically verifiable marking group.

10. A pharmaceutical composition,
**characterized in that**
as the active agent the same comprises an antibody or antibody fragment according to one of the claims 1 to 8, and pharmacologically common carriers, additives, and/or thinning agents.

11. A diagnostic composition, which as a diagnostic reagent comprises an antibody or antibody fragment according to one of the claims 1 to 5, or 9.

12. A nucleic acid coding for an antibody or antibody fragment according to one of the claims 1 to 5, or 8.

13. The nucleic acid according to claim 12 being in operative ligation with an expression control sequence.

14. A vector,
**characterized in that**
the same comprises a nucleic acid according to one of the claims 12 to 13.

15. An isolated cell,
**characterized in that**
the same is transformed by a nucleic acid according to one of the claims 12 to 13, or by a vector according to claim 14.

16. A non-human organism,
**characterized in that**
the same is transformed by a nucleic acid according to one of the claims 12 to 13, or by a vector according to claim 14.

17. A method for the production of an antibody or antibody fragment according to one of the claims 1 to 5,
**characterized in that**
a cell according to claim 15, or a non-human organism according to claim 16 is cultivated under conditions wherein an expression of the antibody or of the antibody fragment occurs, and the expressed antibody or the antibody fragment is obtained.

18. A use of an antibody or antibody fragment according to one of the claims 1 to 8 for the production of a pharmaceutical for the prevention or treatment of diseases related to an ED-B expression, and which are selected from hyper-proliferative diseases, diseases associated with a dysfunction of myofibroblasts, and inflammatory diseases.

19. The use of an antibody or antibody fragment according to one of the claims 1 to 9 for the production of a verification reagent for the diagnosis of diseases related to an ED-B expression, and which are selected from hyper-proliferative diseases, diseases associated with a dysfunction of myofibroblasts, and inflammatory diseases, or a predisposition for the same.

## Revendications

1. Anticorps ou fragment d'anticorps, qui se lie spécifiquement au domaine ED-B de la fibronectine, **caractérisé par le fait qu'**il comprend la région VH codée par SEQ ID NO.75 (MOR 03251) et la région VL codée par SEQ ID NO.77 (MOR 03251) ou les régions H-CDR1, H-CDR2 et H-CDR3 de cette région VH et les régions L-CDR1, L-CDR2 et L-CDR3 de cette région VL, l'anticorps ou le fragment d'anticorps se liant au domaine ED-B avec une valeur de KD de 0,1 nM ou moins, la valeur de KD étant déterminée par résonance plasmonique de surface, et l'anticorps ou le fragment d'anticorps inhibant l'interaction entre le domaine ED-B et son récepteur.

2. Anticorps ou fragment d'anticorps, qui se lie spécifiquement au domaine ED-B de la fibronectine, **caractérisé par le fait qu'**il comprend la région VH codée par SEQ ID NO.79 (MOR 03252) et la région VL codée par SEQ ID NO.77 (MOR 03252) ou les régions H-CDR1, H-CDR2 et H-CDR3 de cette région VH et les régions L-CDR1, L-CDR2 et L-CDR3 de cette région VL, l'anticorps ou le fragment d'anticorps se liant au domaine ED-B avec une valeur de KD de 0,1 nM ou moins, la valeur de KD étant déterminée par résonance plasmonique de surface, et l'anticorps ou le fragment d'anticorps inhibant l'interaction entre le domaine ED-B et son récepteur.

3. Anticorps ou fragment d'anticorps, qui se lie spécifiquement au domaine ED-B de la fibronectine, **caractérisé par le fait qu'**il comprend la région VH codée par SEQ ID NO.83 (MOR 03255) et la région VL codée par SEQ ID NO.77 (MOR 03255) ou les régions H-CDR1, H-CDR2 et H-CDR3 de cette région VH et les régions L-CDR1, L-CDR2 et L-CDR3 de cette région VL, l'anticorps ou le fragment d'anticorps se liant au domaine ED-B avec une valeur de KD de 0,1 nM ou moins, la valeur de KD étant déterminée par résonance plasmonique de surface, et l'anticorps ou le fragment d'anticorps inhibant l'interaction entre le domaine ED-B et son récepteur.

4. Anticorps ou fragment d'anticorps, qui se lie spécifiquement au domaine ED-B de la fibronectine, **caractérisé par le fait qu'**il comprend la région VH codée par SEQ ID NO.85 (MOR 03257) et la région VL codée par SEQ ID NO.77 (MOR 03257) ou les régions H-CDR1, H-CDR2 et H-CDR3 de cette région VH et les régions L-CDR1, L-CDR2 et L-CDR3 de cette région VL, l'anticorps ou le fragment d'anticorps se liant au domaine ED-B avec une valeur de KD de 0,1 nM ou moins, la valeur de KD étant déterminée par résonance plasmonique de surface, et l'anticorps ou le fragment d'anticorps inhibant l'interaction entre le domaine ED-B et son récepteur.

5. Anticorps ou fragment d'anticorps, qui se lie spécifiquement au domaine ED-B de la fibronectine, **caractérisé par le fait qu'**il comprend la région VH codée par SEQ ID NO.87 (MOR 03258) et la région VL codée par SEQ ID NO.59 (MOR 03258) ou les régions H-CDR1, H-CDR2 et H-CDR3 de cette région VH et les régions L-CDR1, L-CDR2 et L-CDR3 de cette région VL, l'anticorps ou le fragment d'anticorps se liant au domaine ED-B avec une valeur de KD de 0,1 nM ou moins, la valeur de KD étant déterminée par résonance plasmonique de surface, et l'anticorps ou le fragment d'anticorps inhibant l'interaction entre le domaine ED-B et son récepteur.

6. Anticorps ou fragment d'anticorps selon l'une quelconque des revendications 1 à 5 sous la forme d'un conjugué avec une substance active thérapeutique.

7. Anticorps ou fragment d'anticorps selon la revendication 6, **caractérisé par le fait que** la substance active thérapeutique est choisie parmi une substance radiothérapique et chimiothérapique.

8. Anticorps ou fragment d'anticorps selon l'une quelconque des revendications 1 à 5 sous la forme d'une protéine de fusion.

9. Anticorps ou fragment d'anticorps selon l'une quelconque des revendications 1 à 5 sous la forme d'un conjugué avec un groupe de marquage identifiable en diagnostic.

10. Composition pharmaceutique, **caractérisée par le fait qu'**elle contient comme substance active un anticorps ou un fragment d'anticorps selon l'une quelconque des revendications 1 à 8 ainsi qu'un support, une substance auxiliaire et/ou un diluant pharmacologiquement usuel.

11. Composition de diagnostic, qui contient comme réactif de diagnostic un anticorps ou un fragment d'anticorps selon l'une quelconque des revendications 1 à 5, ou 9.

12. Acide nucléique, qui code pour un anticorps ou un fragment d'anticorps selon l'une quelconque des revendications 1 à 5 ou 8.

13. Acide nucléique selon la revendication 12 en combinaison opérationnelle avec une séquence de contrôle d'expression.

14. Vecteur, **caractérisé par le fait qu'**il comprend un acide nucléique selon l'une quelconque des revendications 12 à 13.

15. Cellule isolée, **caractérisée par le fait qu'**elle est transformée avec un acide nucléique selon l'une quelconque des revendications 12 à 13 ou un vecteur selon la revendication 14.

16. Organisme non humain, **caractérisé par le fait qu'**il est transformé avec un acide nucléique selon l'une quelconque des revendications 12 à 13 ou un vecteur selon la revendication 14.

17. Procédé de production d'un anticorps ou d'un fragment d'anticorps selon l'une quelconque des revendications 1 à 5, **caractérisé par le fait qu'**on cultive une cellule selon la revendication 15 ou un organisme non humain selon la revendication 16 dans des conditions appropriées pour une expression de l'anticorps ou du fragment d'anticorps, résultant en un anticorps ou un fragment d'anticorps exprimé.

18. Utilisation d'un anticorps ou d'un fragment d'anticorps selon l'une quelconque des revendications 1 à 8 pour la production d'un médicament pour la prévention ou le traitement de maladies qui sont associées à une expression de ED-B et qui sont choisies parmi des maladies hyperprolifératives, des maladies associées à un dysfonctionnement des myofibroblastes et des maladies inflammatoires.

19. Utilisation d'un anticorps ou d'un fragment d'anticorps selon l'une quelconque des revendications 1 à 9 pour la production d'un réactif de détection pour le diagnostic de maladies qui sont associées à une expression de ED-B et qui sont choisies parmi des maladies hyperprolifératives, des maladies associées à un dysfonctionnement des myofibroblastes, et des maladies inflammatoires, ou à une prédisposition pour celles-ci.


### SEQUENZPROTOKOLL

<110> Schering Aktiengesellschaft
<120> Identifizierung und charakterisierung von funktionsblockierenden anti-ED-B-Fibronektin Antikörpern
<130> 33042P DE (WWHC)
<140>
   <141>
<160> 90
<170> PatentIn ver. 2.1
<210> 1
   <211> 354
   <212> DNA
   <213> human
<220>
   <221> CDS
   <222> (1)..(354)
   <223> Antikörper-VH-Region; MOR02610
<400> 1
   cag gtg caa ttg gtt cag agc ggc gcg gaa gtg aaa aaa ccg ggc gcg     48
   Gln Val Gln Leu Val Gln Ser Gly Ala Glu Val Lys Lys Pro Gly Ala
   1               5                   10                  15
   agc gtg aaa gtg agc tgc aaa gcc tcc gga tat acc ttt act ggt ttt     96
   Ser Val Lys Val Ser Cys Lys Ala Ser Gly Tyr Thr Phe Thr Gly Phe
               20                  25                  30
   tat att aat tgg gtc cgc caa gcc cct ggg cag ggt ctc gag tgg atg     144
   Tyr Ile Asn Trp Val Arg Gln Ala Pro Gly Gln Gly Leu Glu Trp Met
           35                  40                  45
   ggc tgg atc aat ccg tat tct ggc aat acg cgt tac gcg cag aag ttt     192
   Gly Trp Ile Asn Pro Tyr Ser Gly Asn Thr Arg Tyr Ala Gln Lys Phe
       50                  55                  60
   cag ggc cgg gtg acc atg acc cgt gat acc agc att agc acc gcg tat     240
   Gln Gly Arg Val Thr Met Thr Arg Asp Thr Ser Ile Ser Thr Ala Tyr
   65                  70                  75                  80
   atg gaa ctg agc agc ctg cgt agc gaa gat acg gcc gtg tat tat tgc     288
   Met Glu Leu Ser Ser Leu Arg Ser Glu Asp Thr Ala Val Tyr Tyr Cys
                   85                  90                  95
   gcg cgt tct cct gtt tat tat aag tat gat tat tgg ggc caa ggc acc     336
   Ala Arg Ser Pro Val Tyr Tyr Lys Tyr Asp Tyr Trp Gly Gln Gly Thr
               100                 105                 110
   ctg gtg acg gtt agc tca                                             354
   Leu Val Thr Val Ser Ser
           115
<210> 2
   <211> 118
   <212> PRT
   <213> human
<400> 2
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Phe
               20                  25                  30
   Tyr Ile Asn Trp Val Arg Gln Ala Pro Gly Gln Gly Leu Glu Trp Met
           35                  40                  45
   Gly Trp Ile Asn Pro Tyr Ser Gly Asn Thr Arg Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Pro Val Tyr Tyr Lys Tyr Asp Tyr Trp Gly Gln Gly Thr
               100                 105                 110
   Leu Val Thr Val Ser Ser
           115
<210> 3
   <211> 336
   <212> DNA
   <213> human
<220>
   <221> CDS
   <222> (1) .. (336)
   <223> Antikörper-VL-Region; MOR02610
<400> 3
   gat atc gtg ctg acc cag ccg cct tca gtg agt ggc gca cca ggt cag     48
   Asp Ile Val Leu Thr Gln Pro Pro Ser Val Ser Gly Ala Pro Gly Gln
   1               5                   10                  15
   cgt gtg acc atc tcg tgt agc ggc agc agc ggc aac att ggt att aat     96
   Arg Val Thr Ile Ser Cys Ser Gly Ser Ser Gly Asn Ile Gly Ile Asn
               20                  25                  30
   ttt gtg aat tgg tac cag cag ttg ccc ggg acg gcg ccg aaa ctt ctg     144
   Phe Val Asn Trp Tyr Gln Gln Leu Pro Gly Thr Ala Pro Lys Leu Leu
           35                  40                  45
   att tat aag aat aat aag cgt ccc tca ggc gtg ccg gat cgt ttt agc     192
   Ile Tyr Lys Asn Asn Lys Arg Pro Ser Gly Val Pro Asp Arg Phe Ser
       50                  55                  60
   ggc tcc aaa agc ggc acc agc gcg agc ctt gcg att acg ggc ctg caa     240
   Gly Ser Lys Ser Gly Thr Ser Ala Ser Leu Ala Ile Thr Gly Leu Gln
   65                  70                  75                  80
   agc gaa gac gaa gcg gat tat tat tgc cag tct tat gat aag act tct     288
   Ser Glu Asp Glu Ala Asp Tyr Tyr Cys Gln Ser Tyr Asp Lys Thr Ser
                   85                  90                  95
   tct act tat gtg ttt ggc ggc ggc acg aag tta acc gtt ctt ggc cag     336
   Ser Thr Tyr Val Phe Gly Gly Gly Thr Lys Leu Thr Val Leu Gly Gln
               100                 105                 110
<210> 4
   <211> 112
   <212> PRT
   <213> human
<400> 4
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Gly Asn Ile Gly Ile Asn
               20                  25                  30
   Phe Val Asn Trp Tyr Gln Gln Leu Pro Gly Thr Ala Pro Lys Leu Leu
           35                  40                  45
   Ile Tyr Lys Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Tyr Asp Lys Thr Ser
                   85                  90                  95
   Ser Thr Tyr Val Phe Gly Gly Gly Thr Lys Leu Thr Val Leu Gly Gln
               100                 105                 110
<210> 5
   <211> 351
   <212> DNA
   <213> human
<220>
   <221> CDS
   <222> (1)..(351)
   <223> Antikörper-VH-Region; MOR02611
<400> 5
   cag gtg caa ttg gtt cag agc ggc gcg gaa gtg aaa aaa ccg ggc gcg      48
   Gln Val Gln Leu Val Gln Ser Gly Ala Glu Val Lys Lys Pro Gly Ala
   1               5                   10                  15
   agc gtg aaa gtg agc tgc aaa gcc tcc gga tat acc ttt tct ggt tct      96
   Ser Val Lys Val Ser Cys Lys Ala Ser Gly Tyr Thr Phe Ser Gly Ser
               20                  25                  30
   tat atg cat tgg gtc cgc caa gcc cct ggg cag ggt ctc gag tgg atg      144
   Tyr Met His Trp Val Arg Gln Ala Pro Gly Gln Gly Leu Glu Trp Met
           35                  40                  45
   ggc att atc aat ccg gct tct ggc aag acg ctt tac gcg cag aag ttt      192
   Gly Ile Ile Asn Pro Ala Ser Gly Lys Thr Leu Tyr Ala Gln Lys Phe
       50                  55                  60
   cag ggc cgg gtg acc atg acc cgt gat acc agc att agc acc gcg tat      240
   Gln Gly Arg Val Thr Met Thr Arg Asp Thr Ser Ile Ser Thr Ala Tyr
   65                  70                  75                  80
   atg gaa ctg agc agc ctg cgt agc gaa gat acg gcc gtg tat tat tgc      288
   Met Glu Leu Ser Ser Leu Arg Ser Glu Asp Thr Ala Val Tyr Tyr Cys
                   85                  90                  95
   gcg cgt ggt ctt tat tat cgt ttt gct tct tgg ggc caa ggc acc ctg      336
   Ala Arg Gly Leu Tyr Tyr Arg Phe Ala Ser Trp Gly Gln Gly Thr Leu
               100                 105                 110
   gtg acg gtt agc tca                                                  351
   Val Thr Val Ser Ser
           115
<210> 6
   <211> 117
   <212> PRT
   <213> human
<400> 6
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Ser Gly Ser
               20                  25                  30
   Tyr Met His Trp Val Arg Gln Ala Pro Gly Gln Gly Leu Glu Trp Met
           35                  40                  45
   Gly Ile Ile Asn Pro Ala Ser Gly Lys Thr Leu Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Leu Tyr Tyr Arg Phe Ala Ser Trp Gly Gln Gly Thr Leu
               100                 105                 110
   Val Thr Val Ser Ser
           115
<210> 7
   <211> 327
   <212> DNA
   <213> human
<220>
   <221> CDS
   <222> (1)..(327)
   <223> Antikörper-VL-Region; MOR02611
<400> 7
   gat atc gca ctg acc cag cca gct tca gtg agc ggc tca cca ggt cag      48
   Asp Ile Ala Leu Thr Gln Pro Ala Ser Val Ser Gly Ser Pro Gly Gln
    1               5                  10                  15
   agc att acc atc tcg tgt acg ggt act agc agc gat att ggt ggt tat      96
   Ser Ile Thr Ile Ser Cys Thr Gly Thr Ser Ser Asp Ile Gly Gly Tyr
               20                  25                  30
   cat tct gtg tct tgg tac cag cag cat ccc ggg aag gcg ccg aaa ctt     144
   His Ser Val Ser Trp Tyr Gln Gln His Pro Gly Lys Ala Pro Lys Leu
           35                  40                  45
   atg att tat cgt tct aat cgt ccc tca ggc gtg agc aac cgt ttt agc     192
   Met Ile Tyr Arg Ser Asn Arg Pro Ser Gly Val Ser Asn Arg Phe Ser
       50                  55                  60
   gga tcc aaa agc ggc aac acc gcg agc ctg acc att agc ggc ctg caa     240
   Gly Ser Lys Ser Gly Asn Thr Ala Ser Leu Thr Ile Ser Gly Leu Gln
   65                  70                  75                  80
   gcg gaa gac gaa gcg gat tat tat tgc gct gct gct act ggt ggt tgg     288
   Ala Glu Asp Glu Ala Asp Tyr Tyr Cys Ala Ala Ala Thr Gly Gly Trp
                   85                  90                  95
   gtg ttt ggc ggc ggc acg aat tta acc gtt ctt ggc cag                 327
   Val Phe Gly Gly Gly Thr Asn Leu Thr Val Leu Gly Gln
               100                 105
<210> 8
   <211> 109
   <212> PRT
   <213> human
<400> 8
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Ile Gly Gly Tyr
               20                  25                  30
   His Ser Val Ser Trp Tyr Gln Gln His Pro Gly Lys Ala Pro Lys Leu
           35                  40                  45
   Met Ile Tyr Arg Ser Asn Arg Pro Ser Gly Val Ser Asn Arg Phe Ser
       50                  55                  60
   Gly Ser Lys Ser Gly Asn Thr Ala Ser Leu Thr Ile Ser Gly Leu Gln
   65                  70                  75                  80
   Ala Glu Asp Glu Ala Asp Tyr Tyr Cys Ala Ala Ala Thr Gly Gly Trp
                   85                  90                  95
   Val Phe Gly Gly Gly Thr Asn Leu Thr Val Leu Gly Gln
               100                 105
<210> 9
   <211> 348
   <212> DNA
   <213> human
<220>
   <221> CDS
   <222> (1)..(348)
   <223> Antikörper-VH-Region; MOR02613
<400> 9
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt tct aat tat      96
   Ser Leu Arg Leu Ser Cys Ala Ala Ser Gly Phe Thr Phe Ser Asn Tyr
               20                  25                  30
   act atg act tgg gtg cgc caa gcc cct ggg aag ggt ctc gag tgg gtg      144
   Thr Met Thr Trp Val Arg Gln Ala Pro Gly Lys Gly Leu Glu Trp Val
           35                  40                  45
   agc ttt atc att ggt tct ggt agc aat acc tct tat gcg gat agc gtg      192
   Ser Phe Ile Ile Gly Ser Gly Ser Asn Thr Ser Tyr Ala Asp Ser Val
       50                  55                  60
   aaa ggc cgt ttt acc att tca cgt gat aat tcg aaa aac acc ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc      288
   Leu Gln Met Asn Ser Leu Arg Ala Glu Asp Thr Ala Val Tyr Tyr Cys
               85                  90                  95
   gcg cgt tat gtt aat ggt ttt gat att tgg ggc caa ggc acc ctg gtg    336
   Ala Arg Tyr Val Asn Gly Phe Asp Ile Trp Gly Gln Gly Thr Leu Val
               100                 105                 110
   acg gtt agc tca                                                    348
   Thr Val Ser Ser
           115
<210> 10
   <211> 116
   <212> PRT
   <213> human
<400> 10
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Thr Met Thr Trp Val Arg Gln Ala Pro Gly Lys Gly Leu Glu Trp Val
           35                  40                  45
   Ser Phe Ile Ile Gly Ser Gly Ser Asn Thr Ser Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Val Asn Gly Phe Asp Ile Trp Gly Gln Gly Thr Leu Val
               100                 105                 110
   Thr Val Ser Ser
           115
<210> 11
   <211> 339
   <212> DNA
   <213> human
<220>
   <221> CDS
   <222> (1)..(339)
   <223> Antikörper-VL-Region; MOR02613
<400> 11
   gat atc gca ctg acc cag cca gct tca gtg agc ggc tca cca ggt cag      48
   Asp Ile Ala Leu Thr Gln Pro Ala Ser Val Ser Gly Ser Pro Gly Gln
    1               5                  10                  15
   agc att acc atc tcg tgt acg ggt act agc agc gat gtt ggt gtt tat      96
   Ser Ile Thr Ile Ser Cys Thr Gly Thr Ser Ser Asp Val Gly Val Tyr
               20                  25                  30
   tat tat gtg tct tgg tac cag cag cat ccc ggg aag gcg ccg aaa ctt     144
   Tyr Tyr Val Ser Trp Tyr Gln Gln His Pro Gly Lys Ala Pro Lys Leu
           35                  40                  45
   atg att tat tat gat tct aag cgt ccc tca ggc gtg agc aac cgt ttt     192
   Met Ile Tyr Tyr Asp Ser Lys Arg Pro Ser Gly Val Ser Asn Arg Phe
       50                  55                  60
   agc gga tcc aaa agc ggc aac acc gcg agc ctg acc att agc ggc ctg     240
   Ser Gly Ser Lys Ser Gly Asn Thr Ala Ser Leu Thr Ile Ser Gly Leu
   65                  70                  75                  80
   caa gcg gaa gac gaa gcg gat tat tat tgc cag act tat gct aag aag     288
   Gln Ala Glu Asp Glu Ala Asp Tyr Tyr Cys Gln Thr Tyr Ala Lys Lys
                   85                  90                  95
   gat tat tct ctt gtg ttt ggc ggc ggc acg aag tta acc gtt ctt ggc     336
   Asp Tyr Ser Leu Val Phe Gly Gly Gly Thr Lys Leu Thr Val Leu Gly
               100                 105                 110
   cag                                                                 339
   Gln
<210> 12
   <211> 113
   <212> PRT
   <213> human
<400> 12
   Asp Ile Ala Leu Thr Gln Pro Ala Ser Val Ser Gly Ser Pro Gly Gln
    1               5                  10                  15
   Ser Ile Thr Ile Ser Cys Thr Gly Thr Ser Ser Asp Val Gly Val Tyr
               20                  25                  30
   Tyr Tyr Val Ser Trp Tyr Gln Gln His Pro Gly Lys Ala Pro Lys Leu
           35                  40                  45
   Met Ile Tyr Tyr Asp Ser Lys Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Thr Tyr Ala Lys Lys
                   85                  90                  95
   Asp Tyr Ser Leu Val Phe Gly Gly Gly Thr Lys Leu Thr Val Leu Gly
               100                 105                 110
   Gln
<210> 13
   <211> 339
   <212> DNA
   <213> human
<220>
   <221> CDS
   <222> (1) .. (339)
   <223> Antikörper-VH-Region; MOR02614
<400> 13
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
    1               5                  10                  15
   agc ctg cgt ctg agc tgc gcg gcc tcc gga ttt acc ttt aat aat aat      96
   Ser Leu Arg Leu Ser Cys Ala Ala Ser Gly Phe Thr Phe Asn Asn Asn
               20                  25                  30
   tgg atg cat tgg gtg cgc caa gcc cct ggg aag ggt ctc gag tgg gtg     144
   Trp Met His Trp Val Arg Gln Ala Pro Gly Lys Gly Leu Glu Trp Val
           35                  40                  45
   agc ttt atc tct ggt tct ggt agc cat acc tat tat gcg gat agc gtg     192
   Ser Phe Ile Ser Gly Ser Gly Ser His Thr Tyr Tyr Ala Asp Ser Val
       50                  55                  60
   aaa ggc cgt ttt acc att tca cgt gat aat tcg aaa aac acc ctg tat     240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc     288
   Leu Gln Met Asn Ser Leu Arg Ala Glu Asp Thr Ala Val Tyr Tyr Cys
                   85                  90                  95
   gcg cgt gct tat gat gtt tgg ggc caa ggc acc ctg gtg acg gtt agc     336
   Ala Arg Ala Tyr Asp Val Trp Gly Gln Gly Thr Leu Val Thr Val Ser
               100                 105                 110
   tca                                                                 339
   Ser
<210> 14
   <211> 113
   <212> PRT
   <213> human
<400> 14
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Asn Asn
               20                  25                  30
   Trp Met His Trp Val Arg Gln Ala Pro Gly Lys Gly Leu Glu Trp Val
           35                  40                  45
   Ser Phe Ile Ser Gly Ser Gly Ser His Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ala Tyr Asp Val Trp Gly Gln Gly Thr Leu Val Thr Val Ser
               100                 105                 110
   Ser
<210> 15
   <211> 327
   <212> DNA
   <213> human
<220>
   <221> CDS
   <222> (1)..(327)
   <223> Antikörper-VL-Region; MOR02614
<400> 15
   gat atc gtg ctg acc cag ccg cct tca gtg agt ggc gca cca ggt cag     48
   Asp Ile Val Leu Thr Gln Pro Pro Ser Val Ser Gly Ala Pro Gly Gln
   1               5                   10                  15
   cgt gtg acc atc tcg tgt agc ggc agc agc agc aac att ggt ttt aat     96
   Arg Val Thr Ile Ser Cys Ser Gly Ser Ser Ser Asn Ile Gly Phe Asn
               20                  25                  30
   tat gtg aat tgg tac cag cag ttg ccc ggg acg gcg ccg aaa ctt ctg     144
   Tyr Val Asn Trp Tyr Gln Gln Leu Pro Gly Thr Ala Pro Lys Leu Leu
           35                  40                  45
   att tat ggt aat tct aag cgt ccc tca ggc gtg ccg gat cgt ttt agc     192
   Ile Tyr Gly Asn Ser Lys Arg Pro Ser Gly Val Pro Asp Arg Phe Ser
       50                  55                  60
   gga tcc aaa agc ggc acc agc gcg agc ctt gcg att acg ggc ctg caa     240
   Gly Ser Lys Ser Gly Thr Ser Ala Ser Leu Ala Ile Thr Gly Leu Gln
   65                  70                  75                  80
   agc gaa gac gaa gcg gat tat tat tgc gct atg ttt tct cct gag ggt     288
   Ser Glu Asp Glu Ala Asp Tyr Tyr Cys Ala Met Phe Ser Pro Glu Gly
                   85                  90                  95
   gtg ttt ggc ggc ggc acg aag tta acc gtt ctt ggc cag                 327
   Val Phe Gly Gly Gly Thr Lys Leu Thr Val Leu Gly Gln
               100                 105
<210> 16
   <211> 109
   <212> PRT
   <213> human
<400> 16
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Phe Asn
               20                  25                  30
   Tyr Val Asn Trp Tyr Gln Gln Leu Pro Gly Thr Ala Pro Lys Leu Leu
           35                  40                  45
   Ile Tyr Gly Asn Ser Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Ala Met Phe Ser Pro Glu Gly
                   85                  90                  95
   Val Phe Gly Gly Gly Thr Lys Leu Thr Val Leu Gly Gln
               100                 105
<210> 17
   <211> 348
   <212> DNA
   <213> human
<220>
   <221> CDS
   <222> (1)..(348)
   <223> Antikörper-VH-Region; MOR02616
<400> 17
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt cgt aat aat      96
   Ser Leu Arg Leu Ser Cys Ala Ala Ser Gly Phe Thr Phe Arg Asn Asn
               20                  25                  30
   gct atg cat tgg gtg cgc caa gcc cct ggg aag ggt ctc gag tgg gtg      144
   Ala Met His Trp Val Arg Gln Ala Pro Gly Lys Gly Leu Glu Trp Val
           35                  40                  45
   agc gct atc tct tct tct ggt agc ggt acc tat tat gcg gat agc gtg      192
   Ser Ala Ile Ser Ser Ser Gly Ser Gly Thr Tyr Tyr Ala Asp Ser Val
       50                  55                  60
   aaa ggc cgt ttt acc att tca cgt gat aat tcg aaa aac acc ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc      288
   Leu Gln Met Asn Ser Leu Arg Ala Glu Asp Thr Ala Val Tyr Tyr Cys
                   85                  90                  95
   gcg cgt gtt att gtt ctt ttt gat tat tgg ggc caa ggc acc ctg gtg      336
   Ala Arg Val Ile Val Leu Phe Asp Tyr Trp Gly Gln Gly Thr Leu Val
               100                 105                 110
   acg gtt agc tca                                                      348
   Thr Val Ser Ser
           115
<210> 18
   <211> 116
   <212> PRT
   <213> human
<400> 18
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Asn Asn
               20                  25                  30
   Ala Met His Trp Val Arg Gln Ala Pro Gly Lys Gly Leu Glu Trp Val
           35                  40                  45
   Ser Ala Ile Ser Ser Ser Gly Ser Gly Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Ile Val Leu Phe Asp Tyr Trp Gly Gln Gly Thr Leu Val
               100                 105                 110
   Thr Val Ser Ser
           115
<210> 19
   <211> 327
   <212> DNA
   <213> human
<220>
   <221> CDS
   <222> (1)..(327)
   <223> Antikörper-VL-Region; MOR02616
<400> 19
   gat atc gtg ctg acc cag agc ccg gcg acc ctg agc ctg tct ccg ggc      48
   Asp Ile Val Leu Thr Gln Ser Pro Ala Thr Leu Ser Leu Ser Pro Gly
   1               5                   10                  15
   gaa cgt gcg acc ctg agc tgc aga gcg agc cag aat gtt cgt tct aat      96
   Glu Arg Ala Thr Leu Ser Cys Arg Ala Ser Gln Asn Val Arg Ser Asn
               20                  25                  30
   ctg gct tgg tac cag cag aaa cca ggt caa gca ccg cgt cta tta att      144
   Leu Ala Trp Tyr Gln Gln Lys Pro Gly Gln Ala Pro Arg Leu Leu Ile
           35                  40                  45
   tat ggt gct tct aat cgt gca act ggg gtc ccg gcg cgt ttt agc ggc      192
   Tyr Gly Ala Ser Asn Arg Ala Thr Gly Val Pro Ala Arg Phe Ser Gly
       50                  55                  60
   tct gga tcc ggc acg gat ttt acc ctg acc att agc agc ctg gaa cct      240
   Ser Gly Ser Gly Thr Asp Phe Thr Leu Thr Ile Ser Ser Leu Glu Pro
   65                  70                  75                  80
   gaa gac ttt gcg gtt tat tat tgc ctt cag aag tac tct att cct ttt      288
   Glu Asp Phe Ala Val Tyr Tyr Cys Leu Gln Lys Tyr Ser Ile Pro Phe
                   85                  90                  95
   acc ttt ggc cag ggt acg aaa gtt gaa att aaa cgt acg                  327
   Thr Phe Gly Gln Gly Thr Lys Val Glu Ile Lys Arg Thr
               100                 105
<210> 20
   <211> 109
   <212> PRT
   <213> human
<400> 20
   Asp Ile Val Leu Thr Gln Ser Pro Ala Thr Leu Ser Leu Ser Pro Gly
   1 5 10 15
   Glu Arg Ala Thr Leu Ser Cys Arg Ala Ser Gln Asn Val Arg Ser Asn
   20 25 30
   Leu Ala Trp Tyr Gln Gln Lys Pro Gly Gln Ala Pro Arg Leu Leu Ile
   35 40 45
   Tyr Gly Ala Ser Asn Arg Ala Thr Gly Val Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Leu Gln Lys Tyr Ser Ile Pro Phe
   85 90 95
   Thr Phe Gly Gln Gly Thr Lys Val Glu Ile Lys Arg Thr
   100 105
<210> 20
   <211> 109
   <212> PRT
   <213> human
<400> 20
<211> 348
   <212> DNA
   <213> human
<220>
   <221> CDS
   <222> (1)..(348)
   <223> Antikörper-VH-Region; MOR02618
<400> 21
<210> 22
   <211> 116
   <212> PRT
   <213> human
<400> 22
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Asn Asn Tyr
               20                  25                  30
   Gly Met Thr Trp Val Arg Gln Ala Pro Gly Lys Gly Leu Glu Trp Val
           35                  40                  45
   Ser Ser Ile Tyr Gly Tyr Gly Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Tyr Trp Val Phe Ala Tyr Trp Gly Gln Gly Thr Leu Val
   100 105 110
   Thr Val Ser Ser
   115
<210> 23
   <211> 330
   <212> DNA
   <213> human
<220>
   <221> CDS
   <222> (1)..(330)
   <223> Antikörper-VL-Region; MOR02618
<400> 23
   gat atc gaa ctg acc cag ccg cct tca gtg agc gtt gca cca ggt cag      48
   Asp Ile Glu Leu Thr Gln Pro Pro Ser Val Ser Val Ala Pro Gly Gln
   1               5                   10                  15
   acc gcg cgt atc tcg tgt agc ggc gat aat att cct ggt aag tct gtt      96
   Thr Ala Arg Ile Ser Cys Ser Gly Asp Asn Ile Pro Gly Lys Ser Val
               20                  25                  30
   cat tgg tac cag cag aaa ccc ggg cag gcg cca gtt ctt gtg att tat      144
   His Trp Tyr Gln Gln Lys Pro Gly Gln Ala Pro Val Leu Val Ile Tyr
           35                  40                  45
   ggt aag atg aat cgt ccc tca ggc atc ccg gaa cgc ttt agc gga tcc      192
   Gly Lys Met Asn Arg Pro Ser Gly Ile Pro Glu Arg Phe Ser Gly Ser
       50                  55                  60
   aac agc ggc aac acc gcg acc ctg acc att agc ggc act cag gcg gaa      240
   Asn Ser Gly Asn Thr Ala Thr Leu Thr Ile Ser Gly Thr Gln Ala Glu
   65                  70                  75                  80
   gac gaa gcg gat tat tat tgc cag tct tat gat aat ttt aat gat tct      288
   Asp Glu Ala Asp Tyr Tyr Cys Gln Ser Tyr Asp Asn Phe Asn Asp Ser
                   85                  90                  95
   gtt gtg ttt ggc ggc ggc acg aag tta acc gtt ctt ggc cag              330
   Val Val Phe Gly Gly Gly Thr Lys Leu Thr Val Leu Gly Gln
               100                 105                 110
<210> 24
   <211> 110
   <212> PRT
   <213> human
<400> 24
   Asp Ile Glu Leu Thr Gln Pro Pro Ser Val Ser Val Ala Pro Gly Gln
   1 5 10 15
   Thr Ala Arg Ile Ser Cys Ser Gly Asp Asn Ile Pro Gly Lys Ser Val
   20 25 30
   His Trp Tyr Gln Gln Lys Pro Gly Gln Ala Pro Val Leu Val Ile Tyr
   35 40 45
   Gly Lys Met Asn Arg Pro Ser Gly Ile Pro Glu Arg Phe Ser Gly Ser
   50 55 60
   Asn Ser Gly Asn Thr Ala Thr Leu Thr Ile Ser Gly Thr Gln Ala Glu
   65 70 75 80
   Asp Glu Ala Asp Tyr Tyr Cys Gln Ser Tyr Asp Asn Phe Asn Asp Ser
   85 90 95
   Val Val Phe Gly Gly Gly Thr Lys Leu Thr Val Leu Gly Gln
   100 105 110
<210> 25
   <211> 333
   <212> DNA
   <213> human
<220>
   <221> CDS
   <222> (1)..(333)
   <223> Antikörper-VH-Region; MOR02619
<400> 25
   cag gtg caa ttg caa gaa agt ggt ccg ggc ctg gtg aaa ccg ggc gaa 48
   Gln Val Gln Leu Gln Glu Ser Gly Pro Gly Leu Val Lys Pro Gly Glu
   1 5 10 15
   acc ctg agc ctg acc tgc acc gtt tcc gga ggc agc att tct tct tat 96
   Thr Leu Ser Leu Thr Cys Thr Val Ser Gly Gly Ser Ile Ser Ser Tyr
   20 25 30
   tat tgg tct tgg att cgc cag gcc cct ggg aag ggt ctc gag tgg att 144
   Tyr Trp Ser Trp Ile Arg Gln Ala Pro Gly Lys Gly Leu Glu Trp Ile
   35 40 45
   ggc atc ggt cat tgg ggc tct acc cag tat aat ccg agc ctg aaa ggc 192
   Gly Ile Gly His Trp Gly Ser Thr Gln Tyr Asn Pro Ser Leu Lys Gly
   50 55 60
   cgg gtg acc att agc gtt gat act tcg aaa aac cag ttt agc ctg aaa 240
   Arg Val Thr Ile Ser Val Asp Thr Ser Lys Asn Gln Phe Ser Leu Lys
   65 70 75 80
   ctg agc agc gtg acg gcg gaa gat acg gcc gtg tat tat tgc gcg cgt 288
   Leu Ser Ser Val Thr Ala Glu Asp Thr Ala Val Tyr Tyr Cys Ala Arg
   85 90 95
   ttt ttt gat gtt tgg ggc caa ggc acc ctg gtg acg gtt agc tca 333
   Phe Phe Asp Val Trp Gly Gln Gly Thr Leu Val Thr Val Ser Ser
   100 105 110
<210> 26
   <211> 111
   <212> PRT
   <213> human
<400> 26
   Gln Val Gln Leu Gln Glu Ser Gly Pro Gly Leu Val Lys Pro Gly Glu
   1 5 10 15
   Thr Leu Ser Leu Thr Cys Thr Val Ser Gly Gly Ser Ile Ser Ser Tyr
   20 25 30
   Tyr Trp Ser Trp Ile Arg Gln Ala Pro Gly Lys Gly Leu Glu Trp Ile
   35 40 45
   Gly Ile Gly His Trp Gly Ser Thr Gln Tyr Asn Pro Ser Leu Lys Gly
   50 55 60
   Arg Val Thr Ile Ser Val Asp Thr Ser Lys Asn Gln Phe Ser Leu Lys
   65 70 75 80
   Leu Ser Ser Val Thr Ala Glu Asp Thr Ala Val Tyr Tyr Cys Ala Arg
   85 90 95
   Phe Phe Asp Val Trp Gly Gln Gly Thr Leu Val Thr Val Ser Ser
   100 105 110
<210> 27
   <211> 333
   <212> DNA
   <213> human
<220>
   <221> CDS
   <222> (1)..(333)
   <223> Antikörper-VL-Region; MOR02619
<400> 27
   gat atc gtg ctg acc cag ccg cct tca gtg agt ggc gca cca ggt cag      48
   Asp Ile Val Leu Thr Gln Pro Pro Ser Val Ser Gly Ala Pro Gly Gln
    1               5                  10                  15
   cgt gtg acc atc tcg tgt agc ggc agc agc agc aac att ggt tct aat      96
   Arg Val Thr Ile Ser Cys Ser Gly Ser Ser Ser Asn Ile Gly Ser Asn
               20                  25                  30
   act gtg cgt tgg tac cag cag ttg ccc ggg acg gcg ccg aaa ctt ctg      144
   Thr Val Arg Trp Tyr Gln Gln Leu Pro Gly Thr Ala Pro Lys Leu Leu
           35                  40                  45
   att tat tct aat aat aag cgt ccc tca ggc gtg ccg gat cgt ttt agc      192
   Ile Tyr Ser Asn Asn Lys Arg Pro Ser Gly Val Pro Asp Arg Phe Ser
       50                  55                  60
   gga tcc aaa agc ggc acc agc gcg agc ctt gcg att acg ggc ctg caa      240
   Gly Ser Lys Ser Gly Thr Ser Ala Ser Leu Ala Ile Thr Gly Leu Gln
   65                  70                  75                  80
   agc gaa gac gaa gcg gat tat tat tgc cag tct tgg gat ggt gct tct      288
   Ser Glu Asp Glu Ala Asp Tyr Tyr Cys Gln Ser Trp Asp Gly Ala Ser
                   85                  90                  95
   act ggt gtg ttt ggc ggc ggc acg aag tta acc gtt ctt ggc cag          333
   Thr Gly Val Phe Gly Gly Gly Thr Lys Leu Thr Val Leu Gly Gln
               100                 105                 110
<210> 28
   <211> 111
   <212> PRT
   <213> human
<400> 28
   Asp Ile Val Leu Thr Gln Pro Pro Ser Val Ser Gly Ala Pro Gly Gln
    1               5                  10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Trp Asp Gly Ala Ser
                   85                  90                  95
   Thr Gly Val Phe Gly Gly Gly Thr Lys Leu Thr Val Leu Gly Gln
               100                 105                 110
<210> 29
   <211> 348
   <212> DNA
   <213> human
<220>
   <221> CDS
   <222> (1)..(348)
   <223> Antikörper-VH-Region; MOR02622
<400> 29
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt tct aat tat      96
   Ser Leu Arg Leu Ser Cys Ala Ala Ser Gly Phe Thr Phe Ser Asn Tyr
               20                  25                  30
   gct atg act tgg gtg cgc caa gcc cct ggg aag ggt ctc gag tgg gtg     144
   Ala Met Thr Trp Val Arg Gln Ala Pro Gly Lys Gly Leu Glu Trp Val
           35                  40                  45
   agc ggt atc tct tac aag tct agc tct acc tat tat gcg gat agc gtg     192
   Ser Gly Ile Ser Tyr Lys Ser Ser Ser Thr Tyr Tyr Ala Asp Ser Val
       50                  55                  60
   aaa ggc cgt ttt acc att tca cgt gat aat tcg aaa aac acc ctg tat     240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc     288
   Leu Gln Met Asn Ser Leu Arg Ala Glu Asp Thr Ala Val Tyr Tyr Cys
                   85                  90                  95
   gcg cgt ggt ctt gtt act ttt gat aat tgg ggc caa ggc acc ctg gtg     336
   Ala Arg Gly Leu Val Thr Phe Asp Asn Trp Gly Gln Gly Thr Leu Val
               100                 105                 110
   acg gtt agc tca                                                     348
   Thr Val Ser Ser
           115
<210> 30
   <211> 116
   <212> PRT
   <213> human
<400> 30
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Ala Met Thr Trp Val Arg Gln Ala Pro Gly Lys Gly Leu Glu Trp Val
           35                  40                  45
   Ser Gly Ile Ser Tyr Lys Ser Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Leu Val Thr Phe Asp Asn Trp Gly Gln Gly Thr Leu Val
               100                 105                 110
   Thr Val Ser Ser
           115
<210> 31
   <211> 339
   <212> DNA
   <213> human
<220>
   <221> CDS
   <222> (1)..(339)
   <223> Antikörper-VL-Region; MOR02622
<400> 31
   gat atc gca ctg acc cag cca gct tca gtg agc ggc tca cca ggt cag      48
   Asp Ile Ala Leu Thr Gln Pro Ala Ser Val Ser Gly Ser Pro Gly Gln
   1               5                   10                  15
   agc att acc atc tcg tgt acg ggt act agc agc gat ggt ggt act tat      96
   Ser Ile Thr Ile Ser Cys Thr Gly Thr Ser Ser Asp Gly Gly Thr Tyr
               20                  25                  30
   aat ttt gtg tct tgg tac cag cag cat ccc ggg aag gcg ccg aaa ctt      144
   Asn Phe Val Ser Trp Tyr Gln Gln His Pro Gly Lys Ala Pro Lys Leu
           35                  40                  45
   atg att tat cgt gtt tct aat cgt ccc tca ggc gtg agc aac cgt ttt      192
   Met Ile Tyr Arg Val Ser Asn Arg Pro Ser Gly Val Ser Asn Arg Phe
       50                  55                  60
   agc gga tcc aaa agc ggc aac acc gcg agc ctg acc att agc ggc ctg      240
   Ser Gly Ser Lys Ser Gly Asn Thr Ala Ser Leu Thr Ile Ser Gly Leu
   65                  70                  75                  80
   caa gcg gaa gac gaa gcg gat tat tat tgc tct tct tgg act cat tct      288
   Gln Ala Glu Asp Glu Ala Asp Tyr Tyr Cys Ser Ser Trp Thr His Ser
                   85                  90                  95
   ttt act gat tat gtg ttt ggc ggc ggc acg aag tta acc gtt ctt ggc      336
   Phe Thr Asp Tyr Val Phe Gly Gly Gly Thr Lys Leu Thr Val Leu Gly
               100                 105                 110
   cag                                                                  339
   Gln
<210> 32
   <211> 113
   <212> PRT
   <213> human
<400> 32
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Gly Gly Thr Tyr
               20                  25                  30
   Asn Phe Val Ser Trp Tyr Gln Gln His Pro Gly Lys Ala Pro Lys Leu
           35                  40                  45
   Met Ile Tyr Arg Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Trp Thr His Ser
                   85                  90                  95
   Phe Thr Asp Tyr Val Phe Gly Gly Gly Thr Lys Leu Thr Val Leu Gly
               100                 105                 110
   Gln
<210> 33
   <211> 348
   <212> DNA
   <213> human
<220>
   <221> CDS
   <222> (1)..(348)
   <223> Antikörper-VH-Region; MOR02715
<400> 33
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt tct tct tat      96
   Ser Leu Arg Leu Ser Cys Ala Ala Ser Gly Phe Thr Phe Ser Ser Tyr
               20                  25                  30
   ggt atg cat tgg gtg cgc caa gcc cct ggg aag ggt ctc gag tgg gtg     144
   Gly Met His Trp Val Arg Gln Ala Pro Gly Lys Gly Leu Glu Trp Val
           35                  40                  45
   agc gtt atc tct tct tct ggt agc tat atc tat tat gcg gat agc gtg     192
   Ser Val Ile Ser Ser Ser Gly Ser Tyr Ile Tyr Tyr Ala Asp Ser Val
       50                  55                  60
   aaa ggc cgt ttt acc att tca cgt gat aat tcg aaa aac acc ctg tat     240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc     288
   Leu Gln Met Asn Ser Leu Arg Ala Glu Asp Thr Ala Val Tyr Tyr Cys
                   85                  90                  95
   gcg cgt aat aag gtt ggt ttt gat gtt tgg ggc caa ggc acc ctg gtg     336
   Ala Arg Asn Lys Val Gly Phe Asp Val Trp Gly Gln Gly Thr Leu Val
               100                 105                 110
   acg gtt agc tca                                                     348
   Thr Val Ser Ser
           115
<210> 34
   <211> 116
   <212> PRT
   <213> human
<400> 34
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ser Val Ile Ser Ser Ser Gly Ser Tyr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Lys Val Gly Phe Asp Val Trp Gly Gln Gly Thr Leu Val
               100                 105                 110
   Thr Val Ser Ser
           115
<210> 35
   <211> 339
   <212> DNA
   <213> human
<220>
   <221> CDS
   <222> (1)..(339)
   <223> Antikörper-VL-Region; MOR02715
<400> 35
   gat atc gca ctg acc cag cca gct tca gtg agc ggc tca cca ggt cag      48
   Asp Ile Ala Leu Thr Gln Pro Ala Ser Val Ser Gly Ser Pro Gly Gln
   1               5                   10                  15
   agc att acc atc tcg tgt acg ggt act agc agc gat ggt ggt ggt tat      96
   Ser Ile Thr Ile Ser Cys Thr Gly Thr Ser Ser Asp Gly Gly Gly Tyr
               20                  25                  30
   aat act gtg tct tgg tac cag cag cat ccc ggg aag gcg ccg aaa ctt      144
   Asn Thr Val Ser Trp Tyr Gln Gln His Pro Gly Lys Ala Pro Lys Leu
           35                  40                  45
   atg att tat tat gtt cat aag cgt ccc tca ggc gtg agc aac cgt ttt      192
   Met Ile Tyr Tyr Val His Lys Arg Pro Ser Gly Val Ser Asn Arg Phe
       50                  55                  60
   agc gga tcc aaa agc ggc aac acc gcg agc ctg acc att agc ggc ctg      240
   Ser Gly Ser Lys Ser Gly Asn Thr Ala Ser Leu Thr Ile Ser Gly Leu
   65                  70                  75                  80
   caa gcg gaa gac gaa gcg gat tat tat tgc cag gct tgg gat aat cag      288
   Gln Ala Glu Asp Glu Ala Asp Tyr Tyr Cys Gln Ala Trp Asp Asn Gln
                   85                  90                  95
   ggt atg aag tat gtg ttt ggc ggc ggc acg aag tta acc gtt ctt ggc      336
   Gly Met Lys Tyr Val Phe Gly Gly Gly Thr Lys Leu Thr Val Leu Gly
               100                 105                 110
   cag                                                                  339
   Gln
<210> 36
   <211> 113
   <212> PRT
   <213> human
<400> 36
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Gly Gly Gly Tyr
               20                  25                  30
   Asn Thr Val Ser Trp Tyr Gln Gln His Pro Gly Lys Ala Pro Lys Leu
           35                  40                  45
   Met Ile Tyr Tyr Val His Lys Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ala Trp Asp Asn Gln
                   85                  90                  95
   Gly Met Lys Tyr Val Phe Gly Gly Gly Thr Lys Leu Thr Val Leu Gly
               100                 105                 110
   Gln
<210> 37
   <211> 345
   <212> DNA
   <213> human
<220>
   <221> CDS
   <222> (1)..(345)
   <223> Antikörper-VH-Region; MOR02718
<400> 37
   cag gtg caa ttg gtg gaa agc ggc ggc ggc ctg gtg caa ccg ggc ggc 48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt aat tct aat 96
   Ser Leu Arg Leu Ser Cys Ala Ala Ser Gly Phe Thr Phe Asn Ser Asn
               20                  25                  30
   gct atg cat tgg gtg cgc caa gcc cct ggg aag ggt ctc gag tgg gtg 144
   Ala Met His Trp Val Arg Gln Ala Pro Gly Lys Gly Leu Glu Trp Val
           35                  40                  45
   agc ttt atc tct ggt tct ggt agc tct acc tat tat gcg gat agc gtg 192
   Ser Phe Ile Ser Gly Ser Gly Ser Ser Thr Tyr Tyr Ala Asp Ser Val
       50                  55                  60
   aaa ggc cgt ttt acc att tca cgt gat aat tcg aaa aac acc ctg tat 240
   Lys Gly Arg Phe Thr Ile Ser Arg Asp Asn Ser Lys Asn Thr Leu Tyr
    65                  70                  75                  80
   ctg caa atg aac agc ctg cgt gcg gaa gat acg gcc gtg tat tat tgc     288
   Leu Gln Met Asn Ser Leu Arg Ala Glu Asp Thr Ala Val Tyr Tyr Cys
                    85                  90                  95
   gcg cgt ggt tgg ttt ttt gct cat tgg ggc caa ggc acc ctg gtg acg     336
   Ala Arg Gly Trp Phe Phe Ala His Trp Gly Gln Gly Thr Leu Val Thr
               100                 105                 110
   gtt agc tca                                                         345
   Val Ser Ser
           115
<210> 38
   <211> 115
   <212> PRT
   <213> human
<400> 38
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Ser Asn
               20                  25                  30
   Ala Met His Trp Val Arg Gln Ala Pro Gly Lys Gly Leu Glu Trp Val
           35                  40                  45
   Ser Phe Ile Ser Gly Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Trp Phe Phe Ala His Trp Gly Gln Gly Thr Leu Val Thr
               100                 105                 110
   Val Ser Ser
           115
<210> 39
   <211> 327
   <212> DNA
   <213> human
<220>
   <221> CDS
   <222> (1)..(327)
   <223> Antikörper-VL-Region; MOR02718
<400> 39
   gat atc gtg ctg acc cag agc ccg gcg acc ctg agc ctg tct ccg ggc      48
   Asp Ile Val Leu Thr Gln Ser Pro Ala Thr Leu Ser Leu Ser Pro Gly
   1               5                   10                  15
   gaa cgt gcg acc ctg agc tgc aga gcg agc cag tct ggt cgt ggt aat      96
   Glu Arg Ala Thr Leu Ser Cys Arg Ala Ser Gln Ser Gly Arg Gly Asn
               20                  25                  30
   ctg gct tgg tac cag cag aaa cca ggt caa gca ccg cgt cta tta att      144
   Leu Ala Trp Tyr Gln Gln Lys Pro Gly Gln Ala Pro Arg Leu Leu Ile
           35                  40                  45
   tat gat gct tct aat cgt gca act ggg gtc ccg gcg cgt ttt agc ggc      192
   Tyr Asp Ala Ser Asn Arg Ala Thr Gly Val Pro Ala Arg Phe Ser Gly
       50                  55                  60
   tct gga tcc ggc acg gat ttt acc ctg acc att agc agc ctg gaa cct      240
   Ser Gly Ser Gly Thr Asp Phe Thr Leu Thr Ile Ser Ser Leu Glu Pro
   65                  70                  75                  80
   gaa gac ttt gcg gtt tat tat tgc ttt cag tat tct tct gtt cct ctt      288
   Glu Asp Phe Ala Val Tyr Tyr Cys Phe Gln Tyr Ser Ser Val Pro Leu
                   85                  90                  95
   acc ttt ggc cag ggt acg aaa gtt gaa att aaa cgt acg                  327
   Thr Phe Gly Gln Gly Thr Lys Val Glu Ile Lys Arg Thr
               100                 105
<210> 40
   <211> 109
   <212> PRT
   <213> human
<400> 40
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Gly Arg Gly Asn
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Val Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Phe Gln Tyr Ser Ser Val Pro Leu
                   85                  90                  95
   Thr Phe Gly Gln Gly Thr Lys Val Glu Ile Lys Arg Thr
               100                 105
<210> 41
   <211> 342
   <212> DNA
   <213> human
<220>
   <221> CDS
   <222> (1)..(342)
   <223> Antikörper-VH-Region; MOR02721
<400> 41
   cag gtg caa ttg gtt cag agc ggc gcg gaa gtg aaa aaa ccg ggc gaa      48
   Gln Val Gln Leu Val Gln Ser Gly Ala Glu Val Lys Lys Pro Gly Glu
   1               5                   10                  15
   agc ctg aaa att agc tgc aaa ggt tcc gga tat tcc ttt act tct tat      96
   Ser Leu Lys Ile Ser Cys Lys Gly Ser Gly Tyr Ser Phe Thr Ser Tyr
               20                  25                  30
   tat att aat tgg gtg cgc cag atg cct ggg aag ggt ctc gag tgg atg     144
   Tyr Ile Asn Trp Val Arg Gln Met Pro Gly Lys Gly Leu Glu Trp Met
           35                  40                  45
   ggc att atc tat ccg tct act agc cgt acc att tat tct ccg agc ttt     192
   Gly Ile Ile Tyr Pro Ser Thr Ser Arg Thr Ile Tyr Ser Pro Ser Phe
       50                  55                  60
   cag ggc cag gtg acc att agc gcg gat aaa agc att agc acc gcg tat     240
   Gln Gly Gln Val Thr Ile Ser Ala Asp Lys Ser Ile Ser Thr Ala Tyr
   65                  70                  75                  80
   ctt caa tgg agc agc ctg aaa gcg agc gat acg gcc atg tat tat tgc     288
   Leu Gln Trp Ser Ser Leu Lys Ala Ser Asp Thr Ala Met Tyr Tyr Cys
                   85                  90                  95
   gcg cgt tat cat ggt gct ttt tgg ggc caa ggc acc ctg gtg acg gtt     336
   Ala Arg Tyr His Gly Ala Phe Trp Gly Gln Gly Thr Leu Val Thr Val
               100                 105                 110
   agc tca                                                             342
   Ser Ser
<210> 42
   <211> 114
   <212> PRT
   <213> human
<400> 42
   Gln Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ser Tyr
               20                  25                  30
   Tyr Ile Asn Trp Val Arg Gln Met Pro Gly Lys Gly Leu Glu Trp Met
           35                  40                  45
   Gly Ile Ile Tyr Pro Ser Thr Ser Arg Thr Ile Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Tyr His Gly Ala Phe Trp Gly Gln Gly Thr Leu Val Thr Val
               100                 105                 110
   Ser Ser
<210> 43
   <211> 342
   <212> DNA
   <213> human
<220>
   <221> CDS
   <222> (1)..(342)
   <223> Antikörper-VL-Region; MOR02721
<400> 43
   cag gtg caa ttg gtt cag agc ggc gcg gaa gtg aaa aaa ccg ggc gaa 48
   Gln Val Gln Leu Val Gln Ser Gly Ala Glu Val Lys Lys Pro Gly Glu
   1 5 10 15
   agc ctg aaa att agc tgc aaa ggt tcc gga tat tcc ttt act tct tat 96
   Ser Leu Lys Ile Ser Cys Lys Gly Ser Gly Tyr Ser Phe Thr Ser Tyr
   20 25 30
   tat att aat tgg gtg cgc cag atg cct ggg aag ggt ctc gag tgg atg 144
   Tyr Ile Asn Trp Val Arg Gln Met Pro Gly Lys Gly Leu Glu Trp Met
   35 40 45
   ggc att atc tat ccg tct act agc cgt acc att tat tct ccg agc ttt 192
   Gly Ile Ile Tyr Pro Ser Thr Ser Arg Thr Ile Tyr Ser Pro Ser Phe
   50 55 60
   cag ggc cag gtg acc att agc gcg gat aaa agc att agc acc gcg tat 240
   Gln Gly Gln Val Thr Ile Ser Ala Asp Lys Ser Ile Ser Thr Ala Tyr
   65 70 75 80
   ctt caa tgg agc agc ctg aaa gcg agc gat acg gcc atg tat tat tgc 288
   Leu Gln Trp Ser Ser Leu Lys Ala Ser Asp Thr Ala Met Tyr Tyr Cys
   85 90 95
   gcg cgt tat cat ggt gct ttt tgg ggc caa ggc acc ctg gtg acg gtt 336
   Ala Arg Tyr His Gly Ala Phe Trp Gly Gln Gly Thr Leu Val Thr Val
   100 105 110
   agc tca 342
   Ser Ser
<210> 44
   <211> 114
   <212> PRT
   <213> human
<400> 44
   Gln Val Gln Leu Val Gln Ser Gly Ala Glu Val Lys Lys Pro Gly Glu
   1 5 10 15
   Ser Leu Lys Ile Ser Cys Lys Gly Ser Gly Tyr Ser Phe Thr Ser Tyr
   20 25 30
   Tyr Ile Asn Trp Val Arg Gln Met Pro Gly Lys Gly Leu Glu Trp Met
   35 40 45
   Gly Ile Ile Tyr Pro Ser Thr Ser Arg Thr Ile Tyr Ser Pro Ser Phe
   50 55 60
   Gln Gly Gln Val Thr Ile Ser Ala Asp Lys Ser Ile Ser Thr Ala Tyr
   65 70 75 80
   Leu Gln Trp Ser Ser Leu Lys Ala Ser Asp Thr Ala Met Tyr Tyr Cys
   85 90 95
   Ala Arg Tyr His Gly Ala Phe Trp Gly Gln Gly Thr Leu Val Thr Val
   100 105 110
   Ser Ser
<210> 45
   <211> 339
   <212> DNA
   <213> human
<220>
   <221> CDS
   <222> (1)..(339)
   <223> Antikörper-VH-Region; MOR02722
<400> 45
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt tct tct aat      96
   Ser Leu Arg Leu Ser Cys Ala Ala Ser Gly Phe Thr Phe Ser Ser Asn
               20                  25                  30
   gct att cat tgg gtg cgc caa gcc cct ggg aag ggt ctc gag tgg gtg     144
   Ala Ile His Trp Val Arg Gln Ala Pro Gly Lys Gly Leu Glu Trp Val
           35                  40                  45
   agc tct atc tct ggt tct ggt agc aat acc tat tat gcg gat agc gtg     192
   Ser Ser Ile Ser Gly Ser Gly Ser Asn Thr Tyr Tyr Ala Asp Ser Val
       50                  55                  60
   aaa ggc cgt ttt acc att tca cgt gat aat tcg aaa aac acc ctg tat     240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc     288
   Leu Gln Met Asn Ser Leu Arg Ala Glu Asp Thr Ala Val Tyr Tyr Cys
                   85                  90                  95
   gcg cgt ttt att gct tct tgg ggc caa ggc acc ctg gtg acg gtt agc     336
   Ala Arg Phe Ile Ala Ser Trp Gly Gln Gly Thr Leu Val Thr Val Ser
               100                 105                 110
   tca                                                                 339
   Ser
<210> 46
   <211> 113
   <212> PRT
   <213> human
<400> 46
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Asn
               20                  25                  30
   Ala Ile His Trp Val Arg Gln Ala Pro Gly Lys Gly Leu Glu Trp Val
           35                  40                  45
   Ser Ser Ile Ser Gly Ser Gly Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Phe Ile Ala Ser Trp Gly Gln Gly Thr Leu Val Thr Val Ser
               100                 105                 110
   Ser
<210> 47
   <211> 342
   <212> DNA
   <213> human
<220>
   <221> CDS
   <222> (1)..(342)
   <223> Antikörper-VL-Region; MOR02722
<400> 47
   gat atc gtg atg acc cag agc cca ctg agc ctg cca gtg act ccg ggc      48
   Asp Ile Val Met Thr Gln Ser Pro Leu Ser Leu Pro Val Thr Pro Gly
    1               5                  10                  15
   gag cct gcg agc att agc tgc aga agc agc caa agc ctg gtt cat tct      96
   Glu Pro Ala Ser Ile Ser Cys Arg Ser Ser Gln Ser Leu Val His Ser
               20                  25                  30
   aat ggc tat act gat ctg aat tgg tac ctt caa aaa cca ggt caa agc     144
   Asn Gly Tyr Thr Asp Leu Asn Trp Tyr Leu Gln Lys Pro Gly Gln Ser
           35                  40                  45
   ccg cag cta tta att tat ctt ggt tct tat cgt gcc agt ggg gtc ccg     192
   Pro Gln Leu Leu Ile Tyr Leu Gly Ser Tyr Arg Ala Ser Gly Val Pro
       50                  55                  60
   gat cgt ttt agc ggc tct gga tcc ggc acc gat ttt acc ctg aaa att     240
   Asp Arg Phe Ser Gly Ser Gly Ser Gly Thr Asp Phe Thr Leu Lys Ile
   65                  70                  75                  80
   agc cgt gtg gaa gct gaa gac gtg ggc gtg tat tat tgc cag cag tat     288
   Ser Arg Val Glu Ala Glu Asp Val Gly Val Tyr Tyr Cys Gln Gln Tyr
                   85                  90                  95
   tct aat ttt cct ttt acc ttt ggc cag ggt acg aaa gtt gaa att aaa     336
   Ser Asn Phe Pro Phe Thr Phe Gly Gln Gly Thr Lys Val Glu Ile Lys
               100                 105                 110
   cgt acg                                                             342
   Arg Thr
<210> 48
   <211> 114
   <212> PRT
   <213> human
<400> 48
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Val His Ser
               20                  25                  30
   Asn Gly Tyr Thr Asp Leu Asn Trp Tyr Leu Gln Lys Pro Gly Gln Ser
           35                  40                  45
   Pro Gln Leu Leu Ile Tyr Leu Gly Ser Tyr Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Gln Gln Tyr
                   85                  90                  95
   Ser Asn Phe Pro Phe Thr Phe Gly Gln Gly Thr Lys Val Glu Ile Lys
               100                 105                 110
   Arg Thr
<210> 49
   <211> 336
   <212> DNA
   <213> human
<220>
   <221> CDS
   <222> (1)..(336)
   <223> Antikörper-VL-Region; MOR03055
<400> 49
   gat atc gtg ctg acc cag ccg cct tca gtg agt ggc gca cca ggt cag      48
   Asp Ile Val Leu Thr Gln Pro Pro Ser Val Ser Gly Ala Pro Gly Gln
   1               5                   10                  15
   cgt gtg acc atc tcg tgt agc ggc agc agc agc aac att ggt tct aat      96
   Arg Val Thr Ile Ser Cys Ser Gly Ser Ser Ser Asn Ile Gly Ser Asn
               20                  25                  30
   act gtg cgt tgg tac cag cag ttg ccc ggg acg gcg ccg aaa ctt ctg      144
   Thr Val Arg Trp Tyr Gln Gln Leu Pro Gly Thr Ala Pro Lys Leu Leu
           35                  40                  45
   att tat tct aat aat aag cgt ccc tca ggc gtg ccg gat cgt ttt agc      192
   Ile Tyr Ser Asn Asn Lys Arg Pro Ser Gly Val Pro Asp Arg Phe Ser
       50                  55                  60
   gga tcc aaa agc ggc acc agc gcg agc ctt gcg att acg ggc ctg caa      240
   Gly Ser Lys Ser Gly Thr Ser Ala Ser Leu Ala Ile Thr Gly Leu Gln
   65                  70                  75                  80
   agc gaa gac gaa gcg gat tat tat tgc cag gct tgg act cgt gct cat      288
   Ser Glu Asp Glu Ala Asp Tyr Tyr Cys Gln Ala Trp Thr Arg Ala His
                   85                  90                  95
   cgt tat cct gtg ttt ggc ggc ggc acg aag tta acc gtt ctt ggc cag      336
   Arg Tyr Pro Val Phe Gly Gly Gly Thr Lys Leu Thr Val Leu Gly Gln
               100                 105                 110
<210> 50
   <211> 112
   <212> PRT
   <213> human
<400> 50
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ala Trp Thr Arg Ala His
                   85                  90                  95
   Arg Tyr Pro Val Phe Gly Gly Gly Thr Lys Leu Thr Val Leu Gly Gln
               100                 105                 110
<210> 51
   <211> 333
   <212> DNA
   <213> human
<220>
   <221> CDS
   <222> (1)..(333)
   <223> Antikörper-VL-Region; MOR03066
<400> 51
   gat atc gtg ctg acc cag ccg cct tca gtg agt ggc gca cca ggt cag      48
   Asp Ile Val Leu Thr Gln Pro Pro Ser Val Ser Gly Ala Pro Gly Gln
   1               5                   10                  15
   cgt gtg acc atc tcg tgt agc ggc agc agc agc aac att ggt tct aat      96
   Arg Val Thr Ile Ser Cys Ser Gly Ser Ser Ser Asn Ile Gly Ser Asn
               20                  25                  30
   act gtg cgt tgg tac cag cag ttg ccc ggg acg gcg ccg aaa ctt ctg      144
   Thr Val Arg Trp Tyr Gln Gln Leu Pro Gly Thr Ala Pro Lys Leu Leu
           35                  40                  45
   att tat tct aat aat aag cgt ccc tca ggc gtg ccg gat cgt ttt agc      192
   Ile Tyr Ser Asn Asn Lys Arg Pro Ser Gly Val Pro Asp Arg Phe Ser
       50                  55                  60
   gga tcc aaa agc ggc acc agc gcg agc ctt gcg att acg ggc ctg caa      240
   Gly Ser Lys Ser Gly Thr Ser Ala Ser Leu Ala Ile Thr Gly Leu Gln
   65                  70                  75                  80
   agc gaa gac gaa gcg gat tat tat tgc tct tct tat gat act cag gtt      288
   Ser Glu Asp Glu Ala Asp Tyr Tyr Cys Ser Ser Tyr Asp Thr Gln Val
                   85                  90                  95
   act cgt gtg ttt ggc ggc ggc acg aag tta acc gtt ctt ggc cag          333
   Thr Arg Val Phe Gly Gly Gly Thr Lys Leu Thr Val Leu Gly Gln
               100                 105                 110
<210> 52
   <211> 111
   <212> PRT
   <213> human
<400> 52
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Ser Ser Tyr Asp Thr Gln Val
                   85                  90                  95
   Thr Arg Val Phe Gly Gly Gly Thr Lys Leu Thr Val Leu Gly Gln
               100                 105                 110
<210> 53
   <211> 333
   <212> DNA
   <213> human
<220>
   <221> CDS
   <222> (1)..(333)
   <223> Antikörper-VL-Region; MOR03075
<400> 53
   gat atc gtg ctg acc cag ccg cct tca gtg agt ggc gca cca ggt cag      48
   Asp Ile Val Leu Thr Gln Pro Pro Ser Val Ser Gly Ala Pro Gly Gln
   1               5                   10                  15
   cgt gtg acc atc tcg tgt agc ggc agc agc agc aac att ggt tct aat      96
   Arg Val Thr Ile Ser Cys Ser Gly Ser Ser Ser Asn Ile Gly Ser Asn
               20                  25                  30
   act gtg cgt tgg tac cag cag ttg ccc ggg acg gcg ccg aaa ctt ctg      144
   Thr Val Arg Trp Tyr Gln Gln Leu Pro Gly Thr Ala Pro Lys Leu Leu
           35                  40                  45
   att tat tct aat aat aag cgt ccc tca ggc gtg ccg gat cgt ttt agc      192
   Ile Tyr Ser Asn Asn Lys Arg Pro Ser Gly Val Pro Asp Arg Phe Ser
       50                  55                  60
   gga tcc aaa agc ggc acc agc gcg agc ctt gcg att acg ggc ctg caa      240
   Gly Ser Lys Ser Gly Thr Ser Ala Ser Leu Ala Ile Thr Gly Leu Gln
   65                  70                  75                  80
   agc gaa gac gaa gcg gat tat tat tgc cag tct tgg gat cct cgt tct      288
   Ser Glu Asp Glu Ala Asp Tyr Tyr Cys Gln Ser Trp Asp Pro Arg Ser
                   85                  90                  95
   ttt act gtg ttt ggc ggc ggc acg aag tta acc gtt ctt ggc cag          333
   Phe Thr Val Phe Gly Gly Gly Thr Lys Leu Thr Val Leu Gly Gln
               100                 105                 110
<210> 54
   <211> 111
   <212> PRT
   <213> human
<400> 54
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Trp Asp Pro Arg Ser
                   85                  90                  95
   Phe Thr Val Phe Gly Gly Gly Thr Lys Leu Thr Val Leu Gly Gln
               100                 105                 110
<210> 55
   <211> 330
   <212> DNA
   <213> human
<220>
   <221> CDS
   <222> (1)..(330)
   <223> Antikörper-VL-Region; MOR03069
<400> 55
   gat atc gtg ctg acc cag ccg cct tca gtg agt ggc gca cca ggt cag      48
   Asp Ile Val Leu Thr Gln Pro Pro Ser Val Ser Gly Ala Pro Gly Gln
   1               5                   10                  15
   cgt gtg acc atc tcg tgt agc ggc agc agc agc aac att ggt tct aat      96
   Arg Val Thr Ile Ser Cys Ser Gly Ser Ser Ser Asn Ile Gly Ser Asn
               20                  25                  30
   act gtg cgt tgg tac cag cag ttg ccc ggg acg gcg ccg aaa ctt ctg      144
   Thr Val Arg Trp Tyr Gln Gln Leu Pro Gly Thr Ala Pro Lys Leu Leu
           35                  40                  45
   att tat tct aat aat aag cgt ccc tca ggc gtg ccg gat cgt ttt agc      192
   Ile Tyr Ser Asn Asn Lys Arg Pro Ser Gly Val Pro Asp Arg Phe Ser
       50                  55                  60
   gga tcc aaa agc ggc acc agc gcg agc ctt gcg att acg ggc ctg caa      240
   Gly Ser Lys Ser Gly Thr Ser Ala Ser Leu Ala Ile Thr Gly Leu Gln
   65                  70                  75                  80
   agc gaa gac gaa gcg gat tat tat tgc tgg act ggt atg tct tat cat      288
   Ser Glu Asp Glu Ala Asp Tyr Tyr Cys Trp Thr Gly Met Ser Tyr His
                   85                  90                  95
   ttt gtg ttt ggc ggc ggc acg aag tta acc gtt ctt ggc cag              330
   Phe Val Phe Gly Gly Gly Thr Lys Leu Thr Val Leu Gly Gln
               100                 105                 110
<210> 56
   <211> 110
   <212> PRT
   <213> human
<400> 56
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
   50 55 60
   Gly Ser Lys Ser Gly Thr Ser Ala Ser Leu Ala Ile Thr Gly Leu Gln
   65 70 75 80
   Ser Glu Asp Glu Ala Asp Tyr Tyr Cys Trp Thr Gly Met Ser Tyr His
   85 90 95
   Phe Val Phe Gly Gly Gly Thr Lys Leu Thr Val Leu Gly Gln
   100 105 110
<210> 57
   <211> 333
   <212> DNA
   <213> human
<220>
   <221> CDS
   <222> (1)..(333)
   <223> Antikörper-VL-Region; MOR03071
<400> 57
   gat atc gtg ctg acc cag ccg cct tca gtg agt ggc gca cca ggt cag 48
   Asp Ile Val Leu Thr Gln Pro Pro Ser Val Ser Gly Ala Pro Gly Gln
   1 5 10 15
   cgt gtg acc atc tcg tgt agc ggc agc agc agc aac att ggt tct aat 96
   Arg Val Thr Ile Ser Cys Ser Gly Ser Ser Ser Asn Ile Gly Ser Asn
   20 25 30
   act gtg cgt tgg tac cag cag ttg ccc ggg acg gcg ccg aaa ctt ctg 144
   Thr Val Arg Trp Tyr Gln Gln Leu Pro Gly Thr Ala Pro Lys Leu Leu
   35 40 45
   att tat tct aat aat aag cgt ccc tca ggc gtg ccg gat cgt ttt agc 192
   Ile Tyr Ser Asn Asn Lys Arg Pro Ser Gly Val Pro Asp Arg Phe Ser
   50 55 60
   gga tcc aaa agc ggc acc agc gcg agc ctt gcg att acg ggc ctg caa 240
   Gly Ser Lys Ser Gly Thr Ser Ala Ser Leu Ala Ile Thr Gly Leu Gln
   65 70 75 80
   agc gaa gac gaa gcg gat tat tat tgc ctt gct tat att cag tct aag 288
   Ser Glu Asp Glu Ala Asp Tyr Tyr Cys Leu Ala Tyr Ile Gln Ser Lys
   85 90 95
   ggt cat gtg ttt ggc ggc ggc acg aag tta acc gtt ctt ggc cag 333
   Gly His Val Phe Gly Gly Gly Thr Lys Leu Thr Val Leu Gly Gln
   100 105 110
<210> 58
   <211> 111
   <212> PRT
   <213> human
<400> 58
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Leu Ala Tyr Ile Gln Ser Lys
                   85                  90                  95
   Gly His Val Phe Gly Gly Gly Thr Lys Leu Thr Val Leu Gly Gln
               100                 105                 110
<210> 59
   <211> 339
   <212> DNA
   <213> human
<220>
   <221> CDS
   <222> (1)..(339)
   <223> Antikörper-VL-Region; MOR03064
<400> 59
   gat atc gca ctg acc cag cca gct tca gtg agc ggc tca cca ggt cag      48
   Asp Ile Ala Leu Thr Gln Pro Ala Ser Val Ser Gly Ser Pro Gly Gln
   1               5                   10                  15
   agc att acc atc tcg tgt acg ggt act agc agc gat ggt ggt ggt tat      96
   Ser Ile Thr Ile Ser Cys Thr Gly Thr Ser Ser Asp Gly Gly Gly Tyr
               20                  25                  30
   aat act gtg tct tgg tac cag cag cat ccc ggg aag gcg ccg aaa ctt      144
   Asn Thr Val Ser Trp Tyr Gln Gln His Pro Gly Lys Ala Pro Lys Leu
           35                  40                  45
   atg att tat tat gtt cat aag cgt ccc tca ggc gtg agc aac cgt ttt      192
   Met Ile Tyr Tyr Val His Lys Arg Pro Ser Gly Val Ser Asn Arg Phe
       50                  55                  60
   agc gga tcc aaa agc ggc aac acc gcg agc ctg acc att agc ggc ctg      240
   Ser Gly Ser Lys Ser Gly Asn Thr Ala Ser Leu Thr Ile Ser Gly Leu
   65                  70                  75                  80
   caa gcg gaa gac gaa gcg gat tat tat tgc cag tct tgg gat ctt ctt      288
   Gln Ala Glu Asp Glu Ala Asp Tyr Tyr Cys Gln Ser Trp Asp Leu Leu
                   85                  90                  95
   gct cct tct gtt gtg ttt ggc ggc ggc acg aag tta acc gtt ctt ggc      336
   Ala Pro Ser Val Val Phe Gly Gly Gly Thr Lys Leu Thr Val Leu Gly
               100                 105                 110
   cag                                                                  339
   Gln
<210> 60
   <211> 113
   <212> PRT
   <213> human
<400> 60
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Gly Gly Gly Tyr
               20                  25                  30
   Asn Thr Val Ser Trp Tyr Gln Gln His Pro Gly Lys Ala Pro Lys Leu
           35                  40                  45
   Met Ile Tyr Tyr Val His Lys Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Trp Asp Leu Leu
                   85                  90                  95
   Ala Pro Ser Val Val Phe Gly Gly Gly Thr Lys Leu Thr Val Leu Gly
               100                 105                 110
   Gln
<210> 61
   <211> 339
   <212> DNA
   <213> human
<220>
   <221> CDS
   <222> (1)..(339)
   <223> Antikörper-VL-Region; MOR03062
<400> 61
   gat atc gca ctg acc cag cca gct tca gtg agc ggc tca cca ggt cag      48
   Asp Ile Ala Leu Thr Gln Pro Ala Ser Val Ser Gly Ser Pro Gly Gln
   1               5                   10                  15
   agc att acc atc tcg tgt acg ggt act agc agc gat ggt ggt ggt tat      96
   Ser Ile Thr Ile Ser Cys Thr Gly Thr Ser Ser Asp Gly Gly Gly Tyr
               20                  25                  30
   aat act gtg tct tgg tac cag cag cat ccc ggg aag gcg ccg aaa ctt      144
   Asn Thr Val Ser Trp Tyr Gln Gln His Pro Gly Lys Ala Pro Lys Leu
           35                  40                  45
   atg att tat tat gtt cat aag cgt ccc tca ggc gtg agc aac cgt ttt      192
   Met Ile Tyr Tyr Val His Lys Arg Pro Ser Gly Val Ser Asn Arg Phe
       50                  55                  60
   agc gga tcc aaa agc ggc aac acc gcg agc ctg acc att agc ggc ctg      240
   Ser Gly Ser Lys Ser Gly Asn Thr Ala Ser Leu Thr Ile Ser Gly Leu
   65                  70                  75                  80
   caa gcg gaa gac gaa gcg gat tat tat tgc cag tct tgg gat ctt tct      288
   Gln Ala Glu Asp Glu Ala Asp Tyr Tyr Cys Gln Ser Trp Asp Leu Ser
                   85                  90                  95
   gtt cat cag gtt gtg ttt ggc ggc ggc acg aag tta acc gtt ctt ggc      336
   Val His Gln Val Val Phe Gly Gly Gly Thr Lys Leu Thr Val Leu Gly
               100                 105                 110
   cag                                                                  339
   Gln
<210> 62
   <211> 113
   <212> PRT
   <213> human
<400> 62
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Gly Gly Gly Tyr
               20                  25                  30
   Asn Thr Val Ser Trp Tyr Gln Gln His Pro Gly Lys Ala Pro Lys Leu
           35                  40                  45
   Met Ile Tyr Tyr Val His Lys Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Trp Asp Leu Ser
                   85                  90                  95
   Val His Gln Val Val Phe Gly Gly Gly Thr Lys Leu Thr Val Leu Gly
               100                 105                 110
   Gln
<210> 63
   <211> 336
   <212> DNA
   <213> human
<220>
   <221> CDS
   <222> (1)..(336)
   <223> Derivat von MOR03055: Antikörper-VH-Region; MOR03243
<400> 63
   cag gtg caa ttg caa gaa agt ggt ccg ggc ctg gtg aaa ccg ggc gaa      48
   Gln Val Gln Leu Gln Glu Ser Gly Pro Gly Leu Val Lys Pro Gly Glu
   1               5                   10                  15
   acc ctg agc ctg acc tgc acc gtt tcc gga ggc agc att tct tct tat      96
   Thr Leu Ser Leu Thr Cys Thr Val Ser Gly Gly Ser Ile Ser Ser Tyr
               20                  25                  30
   tat tgg tct tgg att cgc cag gcc cct ggg aag ggt ctc gag tgg att     144
   Tyr Trp Ser Trp Ile Arg Gln Ala Pro Gly Lys Gly Leu Glu Trp Ile
           35                  40                  45
   ggc gag att cat cgt ggt ggt tat act cag tat aat cct tct ctt aag     192
   Gly Glu Ile His Arg Gly Gly Tyr Thr Gln Tyr Asn Pro Ser Leu Lys
       50                  55                  60
   tct cgg gtc acc att agc gtt gat act tcg aaa aac cag ttt agc ctg     240
   Ser Arg Val Thr Ile Ser Val Asp Thr Ser Lys Asn Gln Phe Ser Leu
   65                  70                  75                  80
   aaa ctg agc agc gtg acg gcg gcg gat acg gcc gtg tat tat tgc gcg     288
   Lys Leu Ser Ser Val Thr Ala Ala Asp Thr Ala Val Tyr Tyr Cys Ala
                   85                  90                  95
   cgt ttt ttt gat gtt tgg ggc caa ggc acc ctg gtg acg gtt agc tca     336
   Arg Phe Phe Asp Val Trp Gly Gln Gly Thr Leu Val Thr Val Ser Ser
               100                 105                 110
<210> 64
   <211> 112
   <212> PRT
   <213> human
<400> 64
   Gln Val Gln Leu Gln Glu Ser Gly Pro Gly Leu Val Lys Pro Gly Glu
   1               5                   10                  15
   Thr Leu Ser Leu Thr Cys Thr Val Ser Gly Gly Ser Ile Ser Ser Tyr
               20                  25                  30
   Tyr Trp Ser Trp Ile Arg Gln Ala Pro Gly Lys Gly Leu Glu Trp Ile
           35                  40                  45
   Gly Glu Ile His Arg Gly Gly Tyr Thr Gln Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Phe Phe Asp Val Trp Gly Gln Gly Thr Leu Val Thr Val Ser Ser
               100                 105                 110
<210> 65
   <211> 336
   <212> DNA
   <213> human
<220>
   <221> CDS
   <222> (1)..(336)
   <223> Derivat von MOR03055: Antikörper-VL-Region; MOR03243
<400> 65
   gat atc gtg ctg acc cag ccg cct tca gtg agt ggc gca cca ggt cag     48
   Asp Ile Val Leu Thr Gln Pro Pro Ser Val Ser Gly Ala Pro Gly Gln
   1               5                   10                  15
   cgt gtg acc atc tcg tgt agc ggc agc agc agc aac att ggt tct aat     96
   Arg Val Thr Ile Ser Cys Ser Gly Ser Ser Ser Asn Ile Gly Ser Asn
               20                  25                  30
   act gtg cgt tgg tac cag cag ttg ccc ggg acg gcg ccg aaa ctt ctg    144
   Thr Val Arg Trp Tyr Gln Gln Leu Pro Gly Thr Ala Pro Lys Leu Leu
           35                  40                  45
   att tat tct aat aat aag cgt ccc tca ggc gtg ccg gat cgt ttt agc    192
   Ile Tyr Ser Asn Asn Lys Arg Pro Ser Gly Val Pro Asp Arg Phe Ser
       50                  55                  60
   gga tcc aaa agc ggc acc agc gcg agc ctt gcg att acg ggc ctg caa    240
   Gly Ser Lys Ser Gly Thr Ser Ala Ser Leu Ala Ile Thr Gly Leu Gln
   65                  70                  75                  80
   agc gaa gac gaa gcg gat tat tat tgc cag gct tgg act cgt gct cat    288
   Ser Glu Asp Glu Ala Asp Tyr Tyr Cys Gln Ala Trp Thr Arg Ala His
                   85                  90                  95
   cgt tat cct gtg ttt ggc ggc ggc acg aag tta acc gtt ctt ggc cag    336
   Arg Tyr Pro Val Phe Gly Gly Gly Thr Lys Leu Thr Val Leu Gly Gln
               100                 105                 110
<210> 66
   <211> 112
   <212> PRT
   <213> human
<400> 66
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ala Trp Thr Arg Ala His
                   85                  90                  95
   Arg Tyr Pro Val Phe Gly Gly Gly Thr Lys Leu Thr Val Leu Gly Gln
               100                 105                 110
<210> 67
   <211> 336
   <212> DNA
   <213> human
<220>
   <221> CDS
   <222> (1)..(336)
   <223> Derivat von MOR03069; Antikörper-vH-Region; MOR03245
<400> 67
   cag gtg caa ttg caa gaa agt ggt ccg ggc ctg gtg aaa ccg ggc gaa      48
   Gln Val Gln Leu Gln Glu Ser Gly Pro Gly Leu Val Lys Pro Gly Glu
   1               5                   10                  15
   acc ctg agc ctg acc tgc acc gtt tcc gga ggc agc att tct tct tat      96
   Thr Leu Ser Leu Thr Cys Thr Val Ser Gly Gly Ser Ile Ser Ser Tyr
               20                  25                  30
   tat tgg tct tgg att cgc cag gcc cct ggg aag ggt ctc gag tgg att      144
   Tyr Trp Ser Trp Ile Arg Gln Ala Pro Gly Lys Gly Leu Glu Trp Ile
           35                  40                  45
   ggc gtt att cat aag tgg ggt ttt act aat tat aat cct tct ctt aag      192
   Gly Val Ile His Lys Trp Gly Phe Thr Asn Tyr Asn Pro Ser Leu Lys
       50                  55                  60
   tct cgg gtc acc att agc gtt gat act tcg aaa aac cag ttt agc ctg      240
   Ser Arg Val Thr Ile Ser Val Asp Thr Ser Lys Asn Gln Phe Ser Leu
   65                  70                  75                  80
   aaa ctg agc agc gtg acg gcg gcg gat acg gcc gtg tat tat tgc gcg      288
   Lys Leu Ser Ser Val Thr Ala Ala Asp Thr Ala Val Tyr Tyr Cys Ala
                   85                  90                  95
   cgt ttt ttt gat gtt tgg ggc caa ggc acc ctg gtg acg gtt agc tca      336
   Arg Phe Phe Asp Val Trp Gly Gln Gly Thr Leu Val Thr Val Ser Ser
               100                 105                 110
<210> 68
   <211> 112
   <212> PRT
   <213> human
<400> 68
   Gln Val Gln Leu Gln Glu Ser Gly Pro Gly Leu Val Lys Pro Gly Glu
   1               5                   10                  15
   Thr Leu Ser Leu Thr Cys Thr Val Ser Gly Gly Ser Ile Ser Ser Tyr
               20                  25                  30
   Tyr Trp Ser Trp Ile Arg Gln Ala Pro Gly Lys Gly Leu Glu Trp Ile
           35                  40                  45
   Gly Val Ile His Lys Trp Gly Phe Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Phe Phe Asp Val Trp Gly Gln Gly Thr Leu Val Thr Val Ser Ser
               100                 105                 110
<210> 69
   <211> 330
   <212> DNA
   <213> human
<220>
   <221> CDS
   <222> (1)..(330)
   <223> Derivat von MOR03069; Antikörper-VL-Region; MOR03245
<400> 69
   gat atc gtg ctg acc cag ccg cct tca gtg agt ggc gca cca ggt cag      48
   Asp Ile Val Leu Thr Gln Pro Pro Ser Val Ser Gly Ala Pro Gly Gln
   1               5                   10                  15
   cgt gtg acc atc tcg tgt agc ggc agc agc agc aac att ggt tct aat      96
   Arg Val Thr Ile Ser Cys Ser Gly Ser Ser Ser Asn Ile Gly Ser Asn
               20                  25                  30
   act gtg cgt tgg tac cag cag ttg ccc ggg acg gcg ccg aaa ctt ctg     144
   Thr Val Arg Trp Tyr Gln Gln Leu Pro Gly Thr Ala Pro Lys Leu Leu
           35                  40                  45
   att tat tct aat aat aag cgt ccc tca ggc gtg ccg gat cgt ttt agc     192
   Ile Tyr Ser Asn Asn Lys Arg Pro Ser Gly Val Pro Asp Arg Phe Ser
       50                  55                  60
   gga tcc aaa agc ggc acc agc gcg agc ctt gcg att acg ggc ctg caa     240
   Gly Ser Lys Ser Gly Thr Ser Ala Ser Leu Ala Ile Thr Gly Leu Gln
   65                  70                  75                  80
   agc gaa gac gaa gcg gat tat tat tgc tgg act ggt atg tct tat cat     288
   Ser Glu Asp Glu Ala Asp Tyr Tyr Cys Trp Thr Gly Met Ser Tyr His
                   85                  90                  95
   ttt gtg ttt ggc ggc ggc acg aag tta acc gtt ctt ggc cag             330
   Phe Val Phe Gly Gly Gly Thr Lys Leu Thr Val Leu Gly Gln
               100                 105                 110
<210> 70
   <211> 110
   <212> PRT
   <213> human
<400> 70
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Trp Thr Gly Met Ser Tyr His
                   85                  90                  95
   Phe Val Phe Gly Gly Gly Thr Lys Leu Thr Val Leu Gly Gln
               100                 105                 110
<210> 71
   <211> 354
   <212> DNA
   <213> human
<220>
   <221> CDS
   <222> (1) .. (354)
   <223> Derivat von MOR03075; Antikörper-VH-Region; MOR03246
<400> 71
   cag gtg caa ttg gtt cag agc ggc gcg gaa gtg aaa aaa ccg ggc gcg      48
   Gln Val Gln Leu Val Gln Ser Gly Ala Glu Val Lys Lys Pro Gly Ala
   1               5                   10                  15
   agc gtg aaa gtg agc tgc aaa gcc tcc gga tat acc ttt act ggt ttt      96
   Ser Val Lys Val Ser Cys Lys Ala Ser Gly Tyr Thr Phe Thr Gly Phe
               20                  25                  30
   tat att aat tgg gtc cgc caa gcc cct ggg cag ggt ctc gag tgg atg      144
   Tyr Ile Asn Trp Val Arg Gln Ala Pro Gly Gln Gly Leu Glu Trp Met
           35                  40                  45
   ggc tgg atc aat ccg tat tct ggc aat acg cgt tac gcg cag aag ttt      192
   Gly Trp Ile Asn Pro Tyr Ser Gly Asn Thr Arg Tyr Ala Gln Lys Phe
       50                  55                  60
   cag ggc cgg gtg acc atg acc cgt gat acc agc att agc acc gcg tat      240
   Gln Gly Arg Val Thr Met Thr Arg Asp Thr Ser Ile Ser Thr Ala Tyr
   65                  70                  75                  80
   atg gaa ctg agc agc ctg cgt agc gaa gat acg gcc gtg tat tat tgc    288
   Met Glu Leu Ser Ser Leu Arg Ser Glu Asp Thr Ala Val Tyr Tyr Cys
                   85                  90                  95
   gcg cgt tct cct gtt tat tat aag tat gat tat tgg ggc caa ggc acc    336
   Ala Arg Ser Pro Val Tyr Tyr Lys Tyr Asp Tyr Trp Gly Gln Gly Thr
               100                 105                 110
   ctg gtg acg gtt agc tca                                            354
   Leu Val Thr Val Ser Ser
           115
<210> 72
   <211> 118
   <212> PRT
   <213> human
<400> 72
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Phe
               20                  25                  30
   Tyr Ile Asn Trp Val Arg Gln Ala Pro Gly Gln Gly Leu Glu Trp Met
           35                  40                  45
   Gly Trp Ile Asn Pro Tyr Ser Gly Asn Thr Arg Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Pro Val Tyr Tyr Lys Tyr Asp Tyr Trp Gly Gln Gly Thr
               100                 105                 110
   Leu Val Thr Val Ser Ser
           115
<210> 73
   <211> 333
   <212> DNA
   <213> human
<220>
   <221> CDS
   <222> (1)..(333)
   <223> Derivat von MOR03075; Antikörper-VL-Region; MOR03246
<400> 73
   gat atc gtg ctg acc cag ccg cct tca gtg agt ggc gca cca ggt cag      48
   Asp Ile Val Leu Thr Gln Pro Pro Ser Val Ser Gly Ala Pro Gly Gln
    1               5                  10                  15
   cgt gtg acc atc tcg tgt agc ggc agc agc agc aac att ggt tct aat      96
   Arg Val Thr Ile Ser Cys Ser Gly Ser Ser Ser Asn Ile Gly Ser Asn
               20                  25                  30
   act gtg cgt tgg tac cag cag ttg ccc ggg acg gcg ccg aaa ctt ctg      144
   Thr Val Arg Trp Tyr Gln Gln Leu Pro Gly Thr Ala Pro Lys Leu Leu
           35                  40                  45
   att tat tct aat aat aag cgt ccc tca ggc gtg ccg gat cgt ttt agc      192
   Ile Tyr Ser Asn Asn Lys Arg Pro Ser Gly Val Pro Asp Arg Phe Ser
       50                  55                  60
   gga tcc aaa agc ggc acc agc gcg agc ctt gcg att acg ggc ctg caa      240
   Gly Ser Lys Ser Gly Thr Ser Ala Ser Leu Ala Ile Thr Gly Leu Gln
   65                  70                  75                  80
   agc gaa gac gaa gcg gat tat tat tgc cag tct tgg gat cct cgt tct      288
   Ser Glu Asp Glu Ala Asp Tyr Tyr Cys Gln Ser Trp Asp Pro Arg Ser
                   85                  90                  95
   ttt act gtg ttt ggc ggc ggc acg aag tta acc gtt ctt ggc cag          333
   Phe Thr Val Phe Gly Gly Gly Thr Lys Leu Thr Val Leu Gly Gln
               100                 105                 110
<210> 74
   <211> 111
   <212> PRT
   <213> human
<400> 74
   Asp Ile Val Leu Thr Gln Pro Pro Ser Val Ser Gly Ala Pro Gly Gln
    1               5                  10                  15
   Arg Val Thr Ile Ser Cys Ser Gly Ser Ser Ser Asn Ile Gly Ser Asn
               20                  25                  30
   Thr Val Arg Trp Tyr Gln Gln Leu Pro Gly Thr Ala Pro Lys Leu Leu
           35                  40                  45
   Ile Tyr Ser Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Trp Asp Pro Arg Ser
                   85                  90                  95
   Phe Thr Val Phe Gly Gly Gly Thr Lys Leu Thr Val Leu Gly Gln
               100                 105                 110
<210> 75
   <211> 348
   <212> DNA
   <213> human
<220>
   <221> CDS
   <222> (1)..(348)
   <223> Derivat von MOR03062; Antikörper-VH-Region; MOR03251
<400> 75
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt tct tct tat      96
   Ser Leu Arg Leu Ser Cys Ala Ala Ser Gly Phe Thr Phe Ser Ser Tyr
               20                  25                  30
   ggt atg cat tgg gtg cgc caa gcc cct ggg aag ggt ctc gag tgg gtg     144
   Gly Met His Trp Val Arg Gln Ala Pro Gly Lys Gly Leu Glu Trp Val
           35                  40                  45
   agc gtt att tct aat atg tct tat act att tat tat gct gat tct gtt     192
   Ser Val Ile Ser Asn Met Ser Tyr Thr Ile Tyr Tyr Ala Asp Ser Val
       50                  55                  60
   aag ggt cgt ttt acc att tca cgt gat aat tcg aaa aac acc ctg tat     240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc     288
   Leu Gln Met Asn Ser Leu Arg Ala Glu Asp Thr Ala Val Tyr Tyr Cys
                   85                  90                  95
   gcg cgt aat aag gtt ggt ttt gat gtt tgg ggc caa ggc acc ctg gtg     336
   Ala Arg Asn Lys Val Gly Phe Asp Val Trp Gly Gln Gly Thr Leu Val
               100                 105                 110
   acg gtt agc tca                                                     348
   Thr Val Ser Ser
           115
<210> 76
   <211> 116
   <212> PRT
   <213> human
<400> 76
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ser Val Ile Ser Asn Met Ser Tyr Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Lys Val Gly Phe Asp Val Trp Gly Gln Gly Thr Leu Val
               100                 105                 110
   Thr Val Ser Ser
           115
<210> 77
   <211> 339
   <212> DNA
   <213> human
<220>
   <221> CDS
   <222> (1)..(339)
   <223> Derivat von MOR03062; Antikörper-VL-Region; MOR03251
<400> 77
   gat atc gca ctg acc cag cca gct tca gtg agc ggc tca cca ggt cag     48
   Asp Ile Ala Leu Thr Gln Pro Ala Ser Val Ser Gly Ser Pro Gly Gln
    1               5                  10                  15
   agc att acc atc tcg tgt acg ggt act agc agc gat ggt ggt ggt tat     96
   Ser Ile Thr Ile Ser Cys Thr Gly Thr Ser Ser Asp Gly Gly Gly Tyr
               20                  25                  30
   aat act gtg tct tgg tac cag cag cat ccc ggg aag gcg ccg aaa ctt    144
   Asn Thr Val Ser Trp Tyr Gln Gln His Pro Gly Lys Ala Pro Lys Leu
           35                  40                  45
   atg att tat tat gtt cat aag cgt ccc tca ggc gtg agc aac cgt ttt    192
   Met Ile Tyr Tyr Val His Lys Arg Pro Ser Gly Val Ser Asn Arg Phe
       50                  55                  60
   agc gga tcc aaa agc ggc aac acc gcg agc ctg acc att agc ggc ctg    240
   Ser Gly Ser Lys Ser Gly Asn Thr Ala Ser Leu Thr Ile Ser Gly Leu
   65                  70                  75                  80
   caa gcg gaa gac gaa gcg gat tat tat tgc cag tct tgg gat ctt tct    288
   Gln Ala Glu Asp Glu Ala Asp Tyr Tyr Cys Gln Ser Trp Asp Leu Ser
                   85                  90                  95
   gtt cat cag gtt gtg ttt ggc ggc ggc acg aag tta acc gtt ctt ggc    336
   Val His Gln Val Val Phe Gly Gly Gly Thr Lys Leu Thr Val Leu Gly
               100                 105                 110
   cag                                                                339
   Gln
<210> 78
   <211> 113
   <212> PRT
   <213> human
<400> 78
   Asp Ile Ala Leu Thr Gln Pro Ala Ser Val Ser Gly Ser Pro Gly Gln
    1               5                  10                  15
   Ser Ile Thr Ile Ser Cys Thr Gly Thr Ser Ser Asp Gly Gly Gly Tyr
               20                  25                  30
   Asn Thr Val Ser Trp Tyr Gln Gln His Pro Gly Lys Ala Pro Lys Leu
           35                  40                  45
   Met Ile Tyr Tyr Val His Lys Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Trp Asp Leu Ser
                   85                  90                  95
   Val His Gln Val Val Phe Gly Gly Gly Thr Lys Leu Thr Val Leu Gly
               100                 105                 110
   Gln
<210> 79
   <211> 348
   <212> DNA
   <213> human
<220>
   <221> CDS
   <222> (1)..(348)
   <223> Antikörper-VH-Region; MOR03252
<400> 79
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt tct tct tat      96
   Ser Leu Arg Leu Ser Cys Ala Ala Ser Gly Phe Thr Phe Ser Ser Tyr
               20                  25                  30
   ggt atg cat tgg gtg cgc caa gcc cct ggg aag ggt ctc gag tgg gtg      144
   Gly Met His Trp Val Arg Gln Ala Pro Gly Lys Gly Leu Glu Trp Val
           35                  40                  45
   agc gtt att tct aat tat tct tgg cat att tat tat gct gat tct gtt      192
   Ser Val Ile Ser Asn Tyr Ser Trp His Ile Tyr Tyr Ala Asp Ser Val
       50                  55                  60
   aag ggt cgt ttt acc att tca cgt gat aat tcg aaa aac acc ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc      288
   Leu Gln Met Asn Ser Leu Arg Ala Glu Asp Thr Ala Val Tyr Tyr Cys
                   85                  90                  95
   gcg cgt aat aag gtt ggt ttt gat gtt tgg ggc caa ggc acc ctg gtg      336
   Ala Arg Asn Lys Val Gly Phe Asp Val Trp Gly Gln Gly Thr Leu Val
               100                 105                 110
   acg gtt agc tca                                                      348
   Thr Val Ser Ser
           115
<210> 80
   <211> 116
   <212> PRT
   <213> human
<400> 80
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ser Val Ile Ser Asn Tyr Ser Trp His Ile Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asn Lys Val Gly Phe Asp Val Trp Gly Gln Gly Thr Leu Val
   100 105 110
   Thr Val Ser Ser
   115
<210> 81
   <211> 348
   <212> DNA
   <213> human
<220>
   <221> CDS
   <222> (1) .. (348)
   <223> Antikörper-VH-Region; MOR03253
<400> 81
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt tct tct tat      96
   Ser Leu Arg Leu Ser Cys Ala Ala Ser Gly Phe Thr Phe Ser Ser Tyr
               20                  25                  30
   ggt atg cat tgg gtg cgc caa gcc cct ggg aag ggt ctc gag tgg gtg      144
   Gly Met His Trp Val Arg Gln Ala Pro Gly Lys Gly Leu Glu Trp Val
           35                  40                  45
   agc gtt att tct aat atg ggt ttt gag att tat tat gct gat tct gtt      192
   Ser Val Ile Ser Asn Met Gly Phe Glu Ile Tyr Tyr Ala Asp Ser Val
       50                  55                  60
   aag ggt cgt ttt acc att tca cgt gat aat tcg aaa aac acc ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc      288
   Leu Gln Met Asn Ser Leu Arg Ala Glu Asp Thr Ala Val Tyr Tyr Cys
                   85                  90                  95
   gcg cgt aat aag gtt ggt ttt gat gtt tgg ggc caa ggc acc ctg gtg      336
   Ala Arg Asn Lys Val Gly Phe Asp Val Trp Gly Gln Gly Thr Leu Val
               100                 105                 110
   acg gtt agc tca                                                      348
   Thr Val Ser Ser
           115
<210> 82
   <211> 116
   <212> PRT
   <213> human
<400> 82
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ser Val Ile Ser Asn Met Gly Phe Glu Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Lys Val Gly Phe Asp Val Trp Gly Gln Gly Thr Leu Val
               100                 105                 110
   Thr Val Ser Ser
           115
<210> 83
   <211> 348
   <212> DNA
   <213> human
<220>
   <221> CDS
   <222> (1)..(348)
   <223> Antikörper-VH-Region; MOR03255
<400> 83
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
   1               5                   10                  15
   agc ctg cgt ctg agc tgc gcg gcc tcc gga ttt acc ttt tct tct tat      96
   Ser Leu Arg Leu Ser Cys Ala Ala Ser Gly Phe Thr Phe Ser Ser Tyr
               20                  25                  30
   ggt atg cat tgg gtg cgc caa gcc cct ggg aag ggt ctc gag tgg gtg      144
   Gly Met His Trp Val Arg Gln Ala Pro Gly Lys Gly Leu Glu Trp Val
           35                  40                  45
   agc gtt att tct aat cgt tct tct tat att tat tat gct gat tct gtt      192
   Ser Val Ile Ser Asn Arg Ser Ser Tyr Ile Tyr Tyr Ala Asp Ser Val
       50                  55                  60
   aag ggt cgt ttt acc att tca cgt gat aat tcg aaa aac acc ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc      288
   Leu Gln Met Asn Ser Leu Arg Ala Glu Asp Thr Ala Val Tyr Tyr Cys
                   85                  90                  95
   gcg cgt aat aag gtt ggt ttt gat gtt tgg ggc caa ggc acc ctg gtg      336
   Ala Arg Asn Lys Val Gly Phe Asp Val Trp Gly Gln Gly Thr Leu Val
               100                 105                 110
   acg gtt agc tca                                                      348
   Thr Val Ser Ser
           115
<210> 84
   <211> 116
   <212> PRT
   <213> human
<400> 84
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
   35 40 45
   Ser Val Ile Ser Asn Arg Ser Ser Tyr Ile Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asn Lys Val Gly Phe Asp Val Trp Gly Gln Gly Thr Leu Val
   100 105 110
   Thr Val Ser Ser
   115
<210> 85
   <211> 348
   <212> DNA
   <213> human
<220>
   <221> CDS
   <222> (1)..(348)
   <223> Antikörper-VH-Region; MOR03257
<400> 85
   cag gtg caa ttg gtg gaa agc ggc ggc ggc ctg gtg caa ccg ggc ggc 48
   Gln Val Gln Leu Val Glu Ser Gly Gly Gly Leu Val Gln Pro Gly Gly
   1 5 10 15
   agc ctg cgt ctg agc tgc gcg gcc tcc gga ttt acc ttt tct tct tat 96
   Ser Leu Arg Leu Ser Cys Ala Ala Ser Gly Phe Thr Phe Ser Ser Tyr
   20 25 30
   ggt atg cat tgg gtg cgc caa gcc cct ggg aag ggt ctc gag tgg gtg 144
   Gly Met His Trp Val Arg Gln Ala Pro Gly Lys Gly Leu Glu Trp Val
   35 40 45
   agc gtt att tct aat cgt ggt aat tat att tat tat gct gat tct gtt 192
   Ser Val Ile Ser Asn Arg Gly Asn Tyr Ile Tyr Tyr Ala Asp Ser Val
   50 55 60
   aag ggt cgt ttt acc att tca cgt gat aat tcg aaa aac acc ctg tat 240
   Lys Gly Arg Phe Thr Ile Ser Arg Asp Asn Ser Lys Asn Thr Leu Tyr
   65 70 75 80
   ctg caa atg aac agc ctg cgt gcg gaa gat acg gcc gtg tat tat tgc 288
   Leu Gln Met Asn Ser Leu Arg Ala Glu Asp Thr Ala Val Tyr Tyr Cys
   85 90 95
   gcg cgt aat aag gtt ggt ttt gat gtt tgg ggc caa ggc acc ctg gtg 336
   Ala Arg Asn Lys Val Gly Phe Asp Val Trp Gly Gln Gly Thr Leu Val
   100 105 110
   acg gtt agc tca 348
   Thr Val Ser Ser
   115
<210> 86
   <211> 116
   <212> PRT
   <213> human
<210> 86
   <211> 116
   <212> PRT
   <213> human
<400> 86
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ser Val Ile Ser Asn Arg Gly Asn Tyr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Lys Val Gly Phe Asp Val Trp Gly Gln Gly Thr Leu Val
               100                 105                 110
   Thr Val Ser Ser
           115
<210> 87
   <211> 348
   <212> DNA
   <213> human
<220>
   <221> CDS
   <222> (1)..(348)
   <223> Derivat von MOR03075; Antikörper-VH-Region; MOR03258
<400> 87
   cag gtg caa ttg gtg gaa agc ggc ggc ggc ctg gtg caa ccg ggc ggc      48
   Gln Val Gln Leu Val Glu Ser Gly Gly Gly Leu Val Gln Pro Gly Gly
    1               5                  10                  15
   agc ctg cgt ctg agc tgc gcg gcc tcc gga ttt acc ttt tct tct tat      96
   Ser Leu Arg Leu Ser Cys Ala Ala Ser Gly Phe Thr Phe Ser Ser Tyr
               20                  25                  30
   ggt atg cat tgg gtg cgc caa gcc cct ggg aag ggt ctc gag tgg gtg     144
   Gly Met His Trp Val Arg Gln Ala Pro Gly Lys Gly Leu Glu Trp Val
           35                  40                  45
   agc gtt att tct aat cag tct aat tat att tat tat gct gat tct gtt     192
   Ser Val Ile Ser Asn Gln Ser Asn Tyr Ile Tyr Tyr Ala Asp Ser Val
       50                  55                  60
   aag ggt cgt ttt acc att tca cgt gat aat tcg aaa aac acc ctg tat     240
   Lys Gly Arg Phe Thr Ile Ser Arg Asp Asn Ser Lys Asn Thr Leu Tyr
   65                  70                  75                  80
   ctg caa atg aac agc ctg cgt gcg gaa gat acg gcc gtg tat tat tgc     288
   Leu Gln Met Asn Ser Leu Arg Ala Glu Asp Thr Ala Val Tyr Tyr Cys
                   85                  90                  95
   gcg cgt aat aag gtt ggt ttt gat gtt tgg ggc caa ggc acc ctg gtg     336
   Ala Arg Asn Lys Val Gly Phe Asp Val Trp Gly Gln Gly Thr Leu Val
               100                 105                 110
   acg gtt agc tca                                                     348
   Thr Val Ser Ser
           115
<210> 88
   <211> 116
   <212> PRT
   <213> human
<400> 88
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ser Val Ile Ser Asn Gln Ser Asn Tyr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asn Lys Val Gly Phe Asp Val Trp Gly Gln Gly Thr Leu Val
               100                 105                 110
   Thr Val Ser Ser
           115
<210> 89
   <211> 354
   <212> DNA
   <213> human
<220>
   <221> CDS
   <222> (1)..(354)
   <223> Derivat von MOR03075; Antikörper-VL-Region; MOR03258
<400> 89
   cag gtg caa ttg gtt cag agc ggc gcg gaa gtg aaa aaa ccg ggc gcg      48
   Gln Val Gln Leu Val Gln Ser Gly Ala Glu Val Lys Lys Pro Gly Ala
   1               5                   10                  15
   agc gtg aaa gtg agc tgc aaa gcc tcc gga tat acc ttt act ggt ttt      96
   Ser Val Lys Val Ser Cys Lys Ala Ser Gly Tyr Thr Phe Thr Gly Phe
               20                  25                  30
   tat att aat tgg gtc cgc caa gcc cct ggg cag ggt ctc gag tgg atg      144
   Tyr Ile Asn Trp Val Arg Gln Ala Pro Gly Gln Gly Leu Glu Trp Met
           35                  40                  45
   ggc tgg atc aat ccg tat tct ggc aat acg cgt tac gcg cag aag ttt      192
   Gly Trp Ile Asn Pro Tyr Ser Gly Asn Thr Arg Tyr Ala Gln Lys Phe
       50                  55                  60
   cag ggc cgg gtg acc atg acc cgt gat acc agc att agc acc gcg tat      240
   Gln Gly Arg Val Thr Met Thr Arg Asp Thr Ser Ile Ser Thr Ala Tyr
   65                  70                  75                  80
   atg gaa ctg agc agc ctg cgt agc gaa gat acg gcc gtg tat tat tgc      288
   Met Glu Leu Ser Ser Leu Arg Ser Glu Asp Thr Ala Val Tyr Tyr Cys
                   85                  90                  95
   gcg cgt tct cct gtt tat tat aag tat gat tat tgg ggc caa ggc acc      336
   Ala Arg Ser Pro Val Tyr Tyr Lys Tyr Asp Tyr Trp Gly Gln Gly Thr
               100                 105                 110
   ctg gtg acg gtt agc tca                                              354
   Leu Val Thr Val Ser Ser
           115
<210> 90
   <211> 118
   <212> PRT
   <213> human
<400> 90
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Phe
   20 25 30
   Tyr Ile Asn Trp Val Arg Gln Ala Pro Gly Gln Gly Leu Glu Trp Met
   35 40 45
   Gly Trp Ile Asn Pro Tyr Ser Gly Asn Thr Arg Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Ser Leu Arg Ser Glu Asp Thr Ala Val Tyr Tyr Cys
   85 90 95
   Ala Arg Ser Pro Val Tyr Tyr Lys Tyr Asp Tyr Trp Gly Gln Gly Thr
   100 105 110
   Leu Val Thr Val Ser Ser
   115